# EUROPÄISCHE PATENTANMELDUNG

(11) **EP 4 265 602 A2**
(43) Veröffentlichungstag der Anmeldung: **25.10.2023**
(21) Anmeldenummer: 23189508.7
(22) Anmeldetag: 24.06.2015
(51) Int. Cl.: C07D 307/16

(54) **LEOLIGIN-DERIVATE ALS GLATTMUSKELZELLEN-PROLIFERATIONSHEMMER**

(30) Priorität: 24.06.2014 AT 5002014; 01.09.2014 AT 6722014
(62) Teilanmeldung aus: 15742173.6
(71) Anmelder: TECHNISCHE UNIVERSITÄT WIEN, 1040 Wien (AT); Universität Innsbruck, 6020 Innsbruck (AT)
(72) Erfinder: Mihovilovic, Marko, 2380 Perchtoldsdorf (AT); Lindner, Thomas, 1040 Wien (AT); Dirsch, Verena M., 1180 Wien (AT); Atanasov, Atanas, 1210 Wien (BG); Bernhard, David, 6300 Wörgl (AT); Stuppner, Hermann, 6091 Götzens (AT); Schwaiger, Stefan, 6020 Innsbruck (AT)
(74) Vertreter: Ellmeyer, Wolfgang

(57) **Zusammenfassung**

Die Erfindung betrifft ein Verfahren zur Herstellung von Verbindungen der Formel (II): 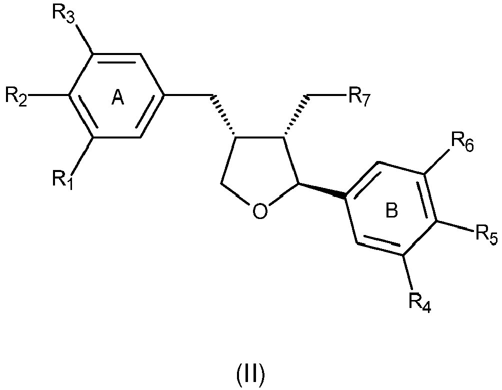 worin entweder
R₁ bis R₆ ausgewählt sind aus -H, -F, -CH₃, -CF₃, -CF₂CH₃, -OCH₃, -COCH₃, -C₄H₉, -COOC₂H₅ und -C₆H₅ oder zwei vicinale Reste aus R₁ bis R₆ so beschaffen und miteinander verbunden sind, dass sie zusammen mit den beiden Kohlenstoffatomen, an die sie gebunden sind, einen gesättigten oder ungesättigten carbocyclischen Ring bilden, und R₇ ausgewählt ist aus OH, Propargyloxy, Cyclopropylcarbonyloxy, Cyclobutylcarbonyloxy, Cyclopentylcarbonyloxy, Cyclopentenylcarbonyloxy, Cyclohexylcarbonyloxy, Cyclohexenylcarbonyloxy, Adamantylethanoyloxy, 3-Phenylpropenoyloxy (Cinnamyloyloxy), 2-Methylbenzoyloxy und Naphthoyloxy, wobei im PhenylRing A gegebenenfalls das ortho-Kohlenstoffatom durch ein Stickstoffatom ersetzt ist, oder
R₁ bis R₆ ausgewählt sind aus -H, -OH, Halogen, Alkyl und Alkoxy und R₇ ausgewählt ist aus -OR₉, -N(R₉')R₉, -SR₉, -C(O)R₉, -OC(O)R₉, -C(O)OR₉, -N(R₉')C(O)R₉, -C(O)N(R₉')R₉ und -S(O)R₉, worin R₉ aus Alkyl und Alkenyl und R₉' aus -H sowie Alkyl und Alkenyl ausgewählt sind,
mittels Durchführung der folgenden Reaktionsschritte a) bis h):
a) Umsetzung eines mit den entsprechenden Resten R₄ bis R₆ substituierten Benzaldehyds der Formel (1) mit Vinylmagnesiumbromid zum entsprechenden racemischen α-Vinylbenzylalkohol *rac*-(2);
b) kinetische Racematspaltung von *rac*-(2) mittels enzymatischer Katalyse zum Erhalt des (*S*)-Enantiomers (*S*)-(2) unter gleichzeitiger Bildung von (*R*)-R₈-(2) als abzutrennendes Nebenprodukt, worin R₈ für einen Acylrest steht;
c) anschließende Veretherung und Oxidation von (*S*)-(2) in beliebiger Reihenfolge, d.h. entweder: c1) die Veretherung von (*S*)-(2) mit Propargylbromid zum Propargylether (3) und darauf folgende Oxidation von (3) zum Propargylether-Epoxid (5), oder umgekehrt: c2) Oxidation von (*S*)-(2) zum Epoxid (4) und darauf folgende Veretherung von (4) mit Propargylbromid zum Propargylether-Epoxid (5);
d) Zyklisierung von (5) mithilfe eines Zyklisierungsmittels zum Tetrahydrofuranylmethanol (6);
e) Schützen der Alkoholfunktionalität von (6) mit der tert-Butyldimethylsilyl- (TBDMS-) zum geschützten Methanol (7);
f) Einführung eines mit den entsprechenden Resten R₁ bis R₃ substituierten Phenyl- oder 2-Pyridylrests (7b) durch Reaktion mit der exozyklischen Methylengruppe von (7) zum geschützten Produkt (8);
g) Abspaltung der Schutzgruppe von (8) zum freien Alkohol (9);
h) wenn R₇ ≠ OH ist, die gleichzeitige oder anschließende Veretherung oder Veresterung der freien OH-Gruppe, um Verbindung (10) mit dem entsprechenden Rest R₇ zu erhalten.

## Beschreibung

Die vorliegende Erfindung betrifft ein neues Syntheseverfahren zur Herstellung von Leoligin-Derivaten.

### STAND DER TECHNIK

Hyperplasie, d.h. die Vergrößerung eines Gewebes oder Organs aufgrund erhöhter Zellteilung, ist ein weit verbreitetes Phänomen und die Ursache zahlreicher Krankheitszustände. Die Intimahyperplasie, eine Verdickung der *Tunica intima,* der innersten, dem Blutstrom zugenwandten Schicht von Blutgefäßen, die häufig nach gefäßchirurgischen Eingriffen oder perkutanenen Katheterinterventionen auftritt, ist eine bedeutenden Ursache für Gefäßverengungen (Stenosen) mit häufig besonders gravierenden Folgen, z.B. koronaren Herzkrankheiten.

Tatsächlich ist die Ursache von Intimahyperplasie nicht die vermehrte Zellteilung von Zellen der *Tunica intima,* sondern vielmehr eine Infiltration, gefolgt von Proliferation glatter Muskelzellen ("smooth muscle cells", SMC), deren Ursprung in der *Tunica media* liegt. Die Intimahyperplasie bildet, durch den Gewebsumbau bedingt, zusammen mit Lipideinlagerungen die Basis für die Bildung atherosklerotischer Plaques. Deren Ruptur führt zur Thrombenbildung. Das Ablösen eines Thrombus und einer daraus resultierenden Embolie, z.B. der Koronargefäße, ist die häufigste Ursache für Herzinfarkt und Schlaganfall.

Eine gängige Behandlungsmethode von Intimahyperplasie, aber auch von Arterio- und Atherosklerose, die gleichfalls zu Stenosen führen können, und von Kombinationen davon, wie z.B. "vein graft disease", einem gemeinsamen Auftreten von Intimahyperplasie und Arteriosklerose, umfasst die Aufweitung des Gefäßes an der betroffenen Stelle mittels Katheter und der anschließenden Platzierung eines Stents, um einen raschen Wiederverschluss zu verhindern. Trotz der Platzierung eines Stents besteht das Risiko einer Restenose, d.h. einer erneuten Verengung des Gefäßes. Die Mechanismen ähneln hierbei den ursprünglichen Vorgängen, können jedoch auch durch den Stent selbst ausgelöst bzw. verändert werden.

Zur Vorbeugung der Restenose wurde vorgeschlagen, den Stent vor der Implantation mit einem Arzneimittel zu beschichten, das die Zellproliferation, insbesondere jene von SMC, hemmt. Zu diesem Zweck wurde unter anderem auch Lariciresinol, ein Lignan, das z.B. in Sesamsamen und Kohlpflanzen vorkommt und dessen (+)-Isomer nachstehend dargestellt ist, sowie ein Methoxyderivat davon offenbart (siehe beispielsweise DE 10 2004 046 244).

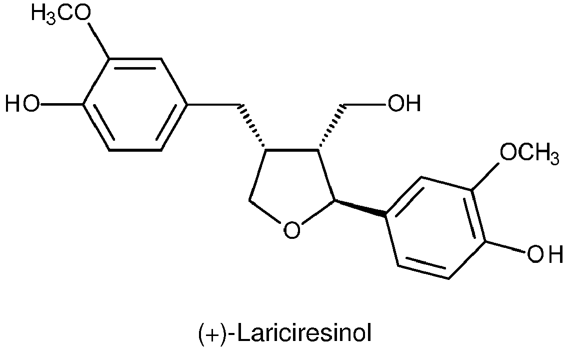

In WO 2010/007169 A1 werden mehrere Derivate von (+)-Lariciresinol, nämlich (+)-Leoligin, d.h. (*Z*)-2-Methyl-2-butensäure-((2*S*,3*R*,4*R*)-4-(3,4-dimethoxybenzyl)-2-(3,4-dimethoxyphenyl)tetrahydrofuran-3-yl)methylester: 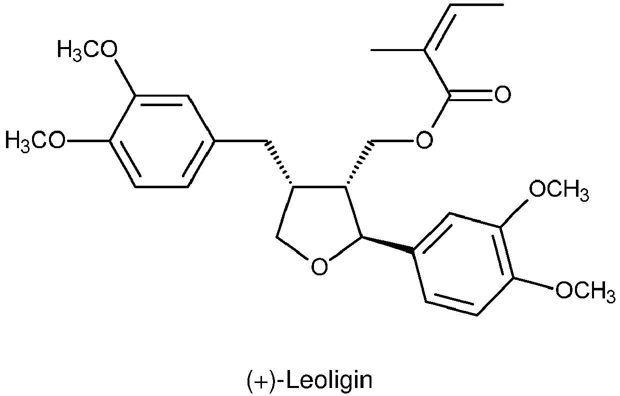 sowie dessen an den beiden Phenylringen 5-Methoxy- bzw. 5,5'-Dimethoxy-substituierte Derivate als wirksame Hemmer der Proliferation glatter Muskelzellen geoffenbart, wobei die beiden Methoxy-Derivate die Aktivität von Leoligin sogar noch übertrafen. Alle drei Verbindungen, die aus den Wurzeln des Edelweiß (*Leontopodium alpinum* Cass.) extrahiert worden waren, zeigten jedoch eine deutlich höhere (anhand der IC₅₀-Werte ausgedrückte) Hemmaktivität als Lariciresinol.

Antiproliferative Aktivität wird in WO 2010/007169 A1 für Verbindungen der nachstehenden Formel (I) beansprucht, vorzugsweise solche mit der in Formel (Ia) angegebenen Stereochemie, die jener von (+)-Leoligin und (+)-Lariciresinol entspricht:

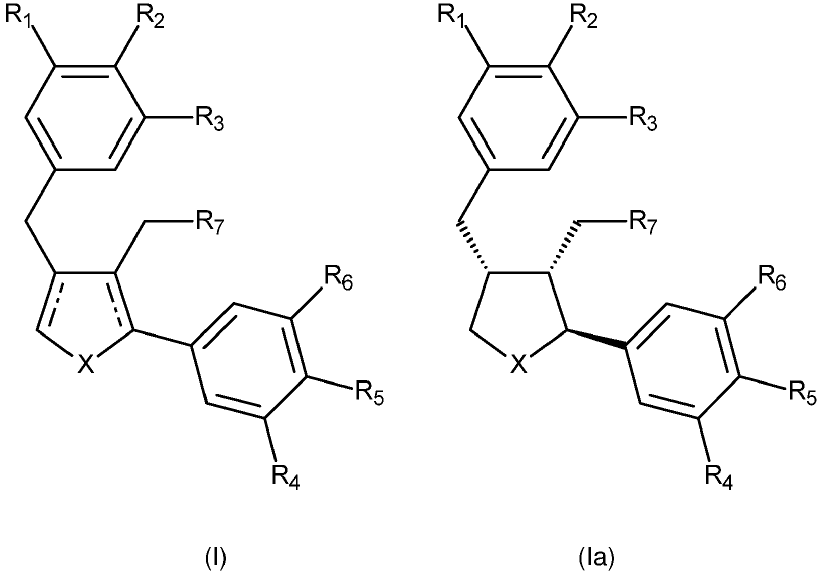

Die Reste R₁ bis R₆ sind dabei jeweils unabhängig aus H, OH, Halogen, Alkyl und Alkoxy ausgewählt, während R₇ aus folgenden Resten ausgewählt ist: -OR₉, -N(R₉')R₉, -SR₉, -C(O)R₉, -OC(O)R₉, -C(O)OR₉, -N(R₉')C(O)R₉, -C(O)N(R₉')R₉ und -S(O)R₉, worin R₉ aus Alkyl und Alkenyl und R₉' aus Wasserstoff, Alkyl und Alkenyl ausgewählt sind, die ihrerseits mit OH, Halogen oder Alkoxy weiter substituiert sein können, und X für O, S, C(R₁₀)R₁₀ oder NR₁₀ stehen kann, worin R₁₀ jeweils für H, Alkyl oder Alkenyl steht.

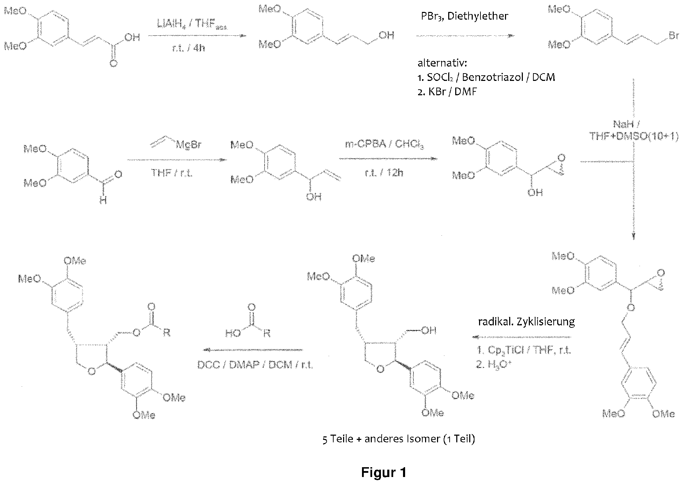

Zur Synthese von Leoligin sowie Derivaten davon mit einer anderen Carbonsäure als Angelikasäure im Rest R₇ ist in Fig. 8 der WO 2010/007169 A1 ein Reaktionsschema angegeben, das auf der bereits davor in Roy et al., J. Org. Chem. 2002(67), 3242-3248, offenbarten Synthese von Furolignanen beruht. Diese Synthese besteht allgemein darin, die beiden jeweils mit zwei Methoxygruppen und einem jeweiligen Linkerrest substituierten Phenylgruppierungen voneinander getrennt herzustellen, die beiden Linkerreste über eine Veretherungsreaktion miteinander zu einer einzigen Linkergruppierung zu verbinden und diese anschließend zum entsprechenden Tetrahydrofuran-Diastereomer ringzuschließen, um Dimethyllariciresinol zu erhalten, das abschließend mit Angelikasäure zu Leoligin - oder mit einer alternativen Carbonsäure zu Derivaten mit unterschiedlichen Resten R₇ - verestert werden soll. Das Schema ist hierin in Fig. 1 dargestellt.

Dabei wird der linke der beiden Dimethoxyphenyl-Reste in Formel (II) ausgehend von im Handel erhältlicher Dimethoxyzimtsäure (Dimethylkaffeesäure), Reduktion zum Dimethoxycinnamylalkohol und Überführung in das Dimethoxycinnamylbromid bereitgestellt (erste Zeile in Fig. 1), und der rechte Rest ausgehend von im Handel erhältlichem Dimethoxybenzaldehyd (Veratrumaldehyd), Umsetzung desselben mit Vinylmagnesiumbromid zum racemischen α-Vinylbenzylalkohol und Oxidation mit m-Chlorperbenzoesäure zum Epoxid (α-Oxiranylbenzylalkohol) (siehe die zweite Zeile in Fig. 1).

Anschließend erfolgt die Verknüpfung der beiden durch Veretherung zum vierfach methoxysubstituierten Cinnamyl-α-oxiranylbenzylether, der dann mit Titanocendichlorid (Dichlorobis(cyclopentadienyl)titan(IV), Cp₂TiCl₂) radikalisch zyklisiert wird, wobei laut Roy et al. und WO 2010/007169 A1 Dimethyllariciresinol als 5:1-Gemisch von Isomeren erhalten wird. Aus diesem wird das gewünschte Isomer mittels präparativer Dünnschichtchromatographie abgetrennt und mit der den jeweiligen Rest R₇ ergebenden Carbonsäure einer Steglich-Veresterung unter Verwendung von N,N'-Dicyclohexylcarbodiimid, DCC, und 4-Dimethylaminopyridin, DMAP, unterzogen, um das gewünschte Leoligin-Derivat zu ergeben.

Die Nachteile dieses Synthesewegs sind die folgenden:
- Wenn Leoligin-Derivate hergestellt werden sollen, die sich in den Substituenten R₁ bis R₆ unterscheiden, muss für jede einzelne Kombination von R₁ bis R₆ zumindest ein neues Zwischenprodukt hergestellt werden, das entweder aus dem entsprechend substituierten Benzaldehyd oder der entsprechend substituierten Zimtsäure resultiert.
- Dabei ist zudem die Auswahl der Substituenten auf solche eingeschränkt, die der anfänglichen Reduktion mit LiAlH₄ standhalten, wodurch bestimmte andere Verbindungen als in WO 2010/007169 A1 gelehrt *a priori* ausscheiden.
- Weiters werden gemäß dem in Roy et al. offenbarten Syntheseweg keine optisch reinen Isomere, sondern Racemate erhalten, wie dies bereits aus dem Titel von Roy et al. hervorgeht: Es wird eine "Short and Stereoselective Total Synthesis of Furano Lignans", darunter auch von (±)-Lariciresinol und dessen Dimethylether, offenbart. Das heißt, dass in dem erwähnten Verhältnis von 5:1 tatsächlich 2 Paare von Enantiomeren und somit insgesamt 4 Isomere erhalten werden. Nach Abtrennung des im 1 :5-Unterschuss vorliegenden, unerwünschten racemischen Isomers bleibt demnach weiterhin ein Racemat zurück, das - vor oder nach der abschließenden Veresterung zu Leoligin oder einem Derivat davon - gespalten werden muss, um das gewünschte (+)-Enantiomer zu isolieren. Dadurch verringert sich natürlich die Ausbeute um 50 %, was sich auf den Reagenzienverbrauch besonders nachteilig auswirkt, da bis dahin das unerwünschte Enantiomer in der sechs- bzw. siebenstufigen Synthese mitgeschleppt wurde.

Vor diesem Hintergrund war es ein Ziel der Erfinder, neue Leoligin-Derivate zu synthetisieren, die gegenüber Leoligin verbesserte proliferationshemmende Aktivität zeigen, sowie ein Herstellungsverfahren zu entwickeln, durch das diese neuen Derivate auf einfachere und wirtschaftlichere Weise herstellbar sind als nach dem Stand der Technik.

### OFFENBARUNG DER ERFINDUNG

Durch die vorliegende Offenbarung werden einerseits Verbindungen der nachstehenden Formel (II) zur Verwendung als Mittel zur selektiven Hemmung der Proliferation von Glattmuskelzellen (SMC) gegenüber Endothelzellen (EC) bereitgestellt. Die von den Erfindern synthetisierten Verbindungen der Formel (II) entsprechen dabei zum Teil der Definition von Formel (Ia) aus WO 2010/007169 A1 (wenn X für O steht), zum Teil aber handelt es sich um völlig neue Verbindungen, die sowohl als Arzneimittel zur Hemmung von SMC in vivo als auch zur Hemmung derselben ex vivo wie etwa in In-vitro-Tests und diversen (anderen) Assayformaten eingesetzt werden können. Wesentlich ist freilich in allen Fällen die überraschende Entdeckung der Erfinder, dass manche Verbindungen der Formel (II) die Proliferation von SMC in deutlich stärkerem Ausmaß hemmen als jene von EC, worauf später noch näher eingegangen wird.

Konkret betrifft die Offenbarung daher einerseits völlig neue Verbindungen der nachstehenden Formel (II) zur Verwendung als die Proliferation von Glattmuskelzellen (SMC) hemmendes Arzneimittelzur Behandlung von oder Prophylaxe gegen Hyperplasie, Stenose oder Restenose, wobei durch die Verbindung die Proliferation von Glattmuskelzellen (SMC) selektiv in höherem Ausmaß als jene von Endothelzellen (EC) gehemmt wird: 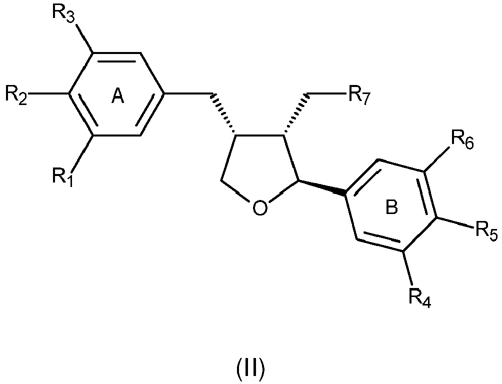 worin
R₁ bis R₆ ausgewählt sind aus -H, -F, -CH₃, -CF₃, -CF₂CH₃, -OCH₃, -COCH₃, -C₄H₉, -COOC₂H₅ und -C₆H₅ oder zwei vicinale Reste aus R₁ bis R₆ so beschaffen und miteinander verbunden sind, dass sie zusammen mit den beiden Kohlenstoffatomen, an die sie gebunden sind, einen gesättigten oder ungesättigten carbocyclischen Ring bilden, R₇ ausgewählt ist aus OH, Propargyloxy, Cyclopropylcarbonyloxy, Cyclobutylcarbonyloxy, Cyclopentylcarbonyloxy, Cyclopentenylcarbonyloxy, Cyclohexylcarbonyloxy, Cyclohexenylcarbonyloxy, Adamantylethanoyloxy, 3-Phenylpropenoyloxy (Cinnamyloyloxy), 2-Methylbenzoyloxy und Naphthoyloxy, wobei in Ring A und/oder Ring B gegebenenfalls ein oder mehrere Ring-Kohlenstoffatome durch Heteroatome ersetzt sind, und
wobei die Verbindungen der Formel (II) aus den folgenden, durch entsprechende Kombination der Reste R₁ bis R₇ erhaltenen Verbindungen ausgewählt sind:
   ((*2S*,*3R*,*4R*)-4-(4-(Trifluormethyl)benzyl)-2-(3,4,5-trimethoxyphenyl)tetrahydrofuran-3-yl)methanol (2760),
   (*Z*)-2-Methyl-2-butensäure-((*2S*,*3R*,*4R*)-2-(4-fluorphenyl)-4-(4-(trifluormethyl)benzyl)-tetrahydrofuran-3-yl)methylester (3016),
   ((*2S,3R,4R*)-2-(3,4-Dimethoxyphenyl)-4-(4-methylbenzyl)tetrahydrofuran-3-yl)-methanol (2754),
   ((*2S*,*3R*,*4R*)-4-Benzyl-2-(3,4,5-trimethoxyphenyl)tetrahydrofuran-3-yl)methanol (2756),
   (*Z*)-2-Methyl-2-butensäure-((*2S*,*3R*,*4R*)-2-phenyl-4-(4-(trifluormethyl)benzyl)-tetrahydrofuran-3-yl)methylester (3015),
   (*Z*)-2-Methyl-2-butensäure-((*2S*,*3R*,*4R*)-2-(3,4-dimethoxyphenyl)-4-(pyridin-2-yl-methyl)tetrahydrofuran-3-yl)methylester (2823),
   ((*2S,3R,4R*)-4-(Biphenyl-4-ylmethyl)-2-(3,4,5-trimethoxyphenyl)tetrahydrofuran-3-yl)-methanol (2767),
   ((*2S,3R,4R*)-4-(4-(1,1-Difluorethyl)benzyl)-2-(3,4-dimethoxyphenyl)tetrahydrofuran-3-yl)methanol (3013),
   Cyclobutancarbonsäure-((*2S*,*3R*,*4R*)-4-(3,4-dimethoxybenzyl)-2-(4-fluorphenyl)-tetrahydrofuran-3-yl)methylester (2863),
   Cyclopentancarbonsäure-((*2S,3R,4R*)-4-(3,4-dimethoxybenzyl)-2-(3,4,5-trimethoxyphenyl)tetrahydrofuran-3-yl)methylester (2635),
   Cyclopropancarbonsäure-((*2S,3R,4R*)-4-(3,4-dimethoxybenzyl)-2-(3,4,5-trimethoxyphenyl)tetrahydrofuran-3-yl)methylester (2637),
   ((*2S,3R,4R*)-2-(3,4-Dimethoxyphenyl)-4-(naphthalin-1-ylmethyl)tetrahydrofuran-3-yl)-methanol (2821),
   ((*2S,3R,4R*)-4-(4-Butylbenzyl)-2-(3,4-dimethoxyphenyl)tetrahydrofuran-3-yl)methanol (3008),
   2-(Adamantan-1-yl)essigsäure-((*2S,3R,4R*)-4-(3,4-dimethoxybenzyl)-2-(3,4-dimethoxyphenyl)tetrahydrofuran-3-yl)methylester (2745),
   (*Z*)-2-Methyl-2-butensäure-((*2S*,*3R*,*4R*)-4-(4-acetylbenzyl)-2-(3,4,5-trimethoxyphenyl)tetrahydrofuran-3-yl)methylester (2771),
   4-(((*3R*,*4R*,*5S*)-4-((((*Z*)-2-Methyl-2-butenoyl)oxy)methyl)-5-(3,4,5-trimethoxyphenyl)-tetrahydrofuran-3-yl)methyl)benzoesäureethylester (2774),
   Cyclopenten-1-carbonsäure-((*2S,3R,4R*)-4-(3,4-dimethoxybenzyl)-2-(3,4,5-trimethoxyphenyl)tetrahydrofuran-3-yl)methylester (2636),
   Zimtsäure-((*2S,3R,4R*)-4-(3,4-dimethoxybenzyl)-2-(3,4-dimethoxyphenyl)tetrahydrofuran-3-yl)methylester (2739),
   Cyclopentancarbonsäure-((*2S*,*3R*,*4R*)-4-(4-(*tert-*butyl)benzyl)-2-(3,4-dimethoxyphenyl)tetrahydrofuran-3-yl)methylester (2867),
   Cyclohexancarbonsäure-((2*S*,3*R*,4*R*)-4-(3,4-dimethoxybenzyl)-2-phenyltetrahydrofuran-3-yl)methylester (2746),
   (2*S*,3*R*,4*R*)-4-(3,4-Dimethoxybenzyl)-2-(3,4-dimethoxyphenyl)-3-(propargyloxymethyl)tetrahydrofuran (2541),
   ((*2S,3R,4R*)-2-(3,4-Dimethoxyphenyl)-4-(4-(trifluormethyl)benzyl)tetrahydrofuran-3-yl)methanol (3005),
   ((2*S*,3*R*,4*R*)-4-(4-(tert-Butyl)benzyl)-2-(3,4-dimethoxyphenyl)tetrahydrofuran-3-yl)-methanol (2749),
   Cyclopentancarbonsäure-((2*S*,3*R*,4*R*)-4-(3,4-dimethoxybenzyl)-2-phenyltetrahydrofuran-3-yl)methylester (2742),
   (*Z*)-2-Methyl-2-butensäure-((*2S*,*3R*,*4R*)-4-(4-(1,1-difluorethyl)benzyl)-2-(3,4-dimethoxyphenyl)tetrahydrofuran-3-yl)methylester (3029),
   Cyclopentancarbonsäure-((*2S,3R,4R*)-4-(3,4-dimethoxybenzyl)-2-(4-fluorphenyl)-tetrahydrofuran-3-yl)methylester (2781),
   4-(((*3R*,*4R*,*5S*)-5-(3,4-Dimethoxyphenyl)-4-((((*Z*)-2-methyl-2-butenoyl)oxy)methyl)-tetrahydrofuran-3-yl)methyl)benzoesäureethylester (2792),
   Benzoesäure-((*2S,3R,4R*)-4-(3,4-dimethoxybenzyl)-2-(3,4,5-trimethoxyphenyl)-tetrahydrofuran-3-yl)methylester (2638) und
   Cyclohexancarbonsäure-((*2S,3R,4R*)-4-(3,4-dimethoxybenzyl)-2-(3,4,5-trimethoxyphenyl)tetrahydrofuran-3-yl)methylester (2633);
   sowie andererseits Verbindungen der Formel (II), die zwar von den Erfindern ebenfalls erstmalig hergestellt und getestet wurden, aber unter die Definition von Formel (Ia) aus WO 2010/007169 A1 (wenn X für O steht) fallen, d.h. Verbindungen der nachstehenden Formel (II) zur Verwendung als die Proliferation von Glattmuskelzellen (SMC) hemmendes Arzneimittel zur Behandlung von oder Prophylaxe gegen Hyperplasie, Stenose oder Restenose: 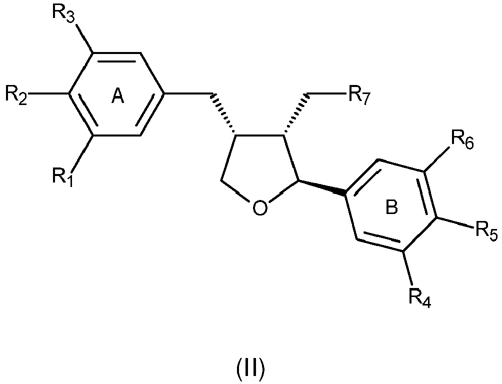
   worin R₁ bis R₆ ausgewählt sind aus -H, -OH, Halogen, Alkyl und Alkoxy und R₇ ausgewählt ist aus -OR₉, -N(R₉')R₉, -SR₉, -C(O)R₉, -OC(O)R₉, -C(O)OR₉, -N(R₉')C(O)R₉, -C(O)N(R₉')R₉ und -S(O)R₉, worin R₉ aus Alkyl und Alkenyl und R₉' aus -H sowie Alkyl und Alkenyl ausgewählt sind,
   die dadurch gekennzeichnet sind, dass:
      a) in Formel (II)
         R₁ bis R₆ ausgewählt sind aus -H, -F, -CH₃, -OCH₃ und -C₄H₉;
         R₇ ausgewählt ist aus Allyloxy, 2,2-Dimethylpropanoyloxy (Pivaloyloxy), Butanoyloxy, 3-Methylbutanoyloxy, 2-Butenoyloxy, 2-Methyl-2-butenoyloxy, 3-Methyl-2-butenoyloxy, Isopentanoyloxy, 2-Ethylbutanoyloxy und 3,3-Dimethylbutanoyloxy;
         wobei die Verbindungen der Formel (II) aus den folgenden, durch entsprechende Kombination der Reste R₁ bis R₇ erhaltenen Verbindungen ausgewählt sind:
            Isobuttersäure-((*2S,3R,4R*)-4-(3,4-dimethoxybenzyl)-2-(3,4,5-trimethoxyphenyl)-tetrahydrofuran-3-yl)methylester (2632),
            (*Z*)-2-Methyl-2-butensäure-((*2S*,*3R*,*4R*)-4-(4-butylbenzyl)-2-(3,4-dimethoxyphenyl)-tetrahydrofuran-3-yl)methylester (3026),
            (*Z*)-2-Methyl-2-butensäure-((*2S*,*3R*,*4R*)-4-(4-(*tert-*butyl)benzyl)-2-(4-fluorphenyl)-tetrahydrofuran-3-yl)methylester (3010),
            (2*S*,3*R*,4*R*)-3-((Allyloxy)methyl)-4-(3,4-dimethoxybenzyl)-2-(3,4-dimethoxyphenyl)-tetrahydrofuran (2540),
            3-Methyl-2-butensäure-((*2S,3R,4R*)-4-(3,4-dimethoxybenzyl)-2-(3,4,5-trimethoxyphenyl)tetrahydrofuran-3-yl)methylester (2755),
            3-Methylbuttersäure-((*2S,3R,4R*)-4-(3,4-dimethoxybenzyl)-2-(4-fluorphenyl)-tetrahydrofuran-3-yl)methylester (2862),
            (*Z*)-2-Methyl-2-butensäure-((*2S*,*3R*,*4R*)-4-(4-fluorbenzyl)-2-(4-fluorphenyl)tetrahydrofuran-3-yl)methylester (2864),
            Buttersäure-((*2S,3R,4R*)-4-(3,4-dimethoxybenzyl)-2-(3,4,5-trimethoxyphenyl)-tetrahydrofuran-3-yl)methylester (2629),
            (*E*)-2-Methyl-2-butensäure-((*2S,3R,4R*)-4-(3,4-dimethoxybenzyl)-2-(3,4,5-trimethoxyphenyl)tetrahydrofuran-3-yl)methylester (2628),
            (E)-2-Butensäure-((*2S,3R,4R*)-4-(3,4-dimethoxybenzyl)-2-(3,4-dimethoxyphenyl)-tetrahydrofuran-3-yl)methylester (2549),
            (*Z*)-2-Methyl-2-butensäure-((*2S*,*3R*,*4R*)-4-(4-(*tert*-butyl)benzyl)-2-(3,4-dimethoxyphenyl)tetrahydrofuran-3-yl)methylester (2788),
            (*Z*)-2-Methyl-2-butensäure-((*2S*,*3R*,*4R*)-4-(4-(*tert*-butyl)benzyl)-2-(3,4,5-trimethoxyphenyl)tetrahydrofuran-3-yl)methylester (2765),
            3-Methyl-2-butensäure-((*2S,3R,4R*)-4-(3,4-dimethoxybenzyl)-2-(3,4-dimethoxyphenyl)tetrahydrofuran-3-yl)methylester (2738) und
            2-Ethylbuttersäure-((*2S,3R,4R*)-4-(3,4-dimethoxybenzyl)-2-(4-fluorphenyl)tetrahydrofuran-3-yl)methylester (2856);
               und
      b) durch die Verbindungen der Formel (II) die Proliferation von Glattmuskelzellen (SMC) selektiv in höherem Ausmaß als jene von Endothelzellen (EC) gehemmt wird.

Bei allen oben aufgelisteten 43 Verbindungen der Formel (II) wurde, wie erwähnt, überraschenderweise festgestellt, dass sie SMC im Vergleich zu EC selektiv hemmen, und zwar in einem Ausmaß vom bis zu 73fachen ihrer Aktivität gegen EC. Dies ist umso überraschender als:
a) mittels entsprechender, oben beschriebener Auswahl der Substituenten R₁ bis R₇ insgesamt 130 Verbindungen hergestellt wurden, von denen also 2/3 diese Selektivität nicht - und mitunter sogar die umgekehrte Selektivität - zeigen, und vor allem
b) Leoligin selbst die umgekehrte Selektivität zeigt; dies sowohl im Zuge der hierin angeführten Aktivitätstests (mit einem Wirksamkeitsverhältnis von 0,39, d.h. EC werden durch Leoligin etwa 2,5-mal so stark gehemmt wie SMC) als auch bei im Stand der Technik beschriebenen Tests.

So wurde für Leoligin eine IC₅₀ gegenüber SMC von 54,5 µM sowie parallel dazu eine IC₅₀ gegenüber EC von nur 17,9 µM offenbart (A. Knolz, Diplomarbeit, Universität Innsbruck, 2008), was ein IC₅₀-Verhältnis, als Maß für die selektive Hemmung von SMC gegenüber EC, von 0,33 (und einen Kehrwert, als Maß für die selektive Hemmung von EC gegenüber SMC, von 3,0) ergibt. Dies entspricht fast exakt dem Verhältnis von 0,32, das von den Erfindern für die am wenigsten SMC-selektive neue Verbindung (2822; Daten hierin nicht offenbart) bestimmt wurde.

Bei der Auswahl der hierin beanspruchten, SMC selektiv hemmenden Verbindungen anhand ihrer Aktivitätsverhältnisse wurde aufgrund der relativ großen Schwankungen aufgrund der Berechnungsmethode, zum Teil aber auch schon bei den hierfür durchzuführenden biologischen Tests, ein Schwellenwert bei einem Verhältnis von 1,50 gesetzt (siehe die späteren Ausführungsbeispiele): Alle 43 oben genannten Verbindungen hemmten daher SMC zumindest um 50 % stärker als EC.

Vorzugsweise ist die Verbindung freilich aus der folgenden Gruppe ausgewählt, die 32 Verbindungen umfasst, die SMC zumindest doppelt so stark hemmten wie EC, d.h. ein Wirksamkeitsverhältnis von zumindest 2,0 aufweisen:
Isobuttersäure-((*2S,3R,4R*)-4-(3,4-dimethoxybenzyl)-2-(3,4,5-trimethoxyphenyl)-tetrahydrofuran-3-yl)methylester (2632),
(*Z*)-2-Methyl-2-butensäure-((*2S*,*3R*,*4R*)-4-(4-butylbenzyl)-2-(3,4-dimethoxyphenyl)-tetrahydrofuran-3-yl)methylester (3026),
((*2S,3R,4R*)-4-(4-(Trifluormethyl)benzyl)-2-(3,4,5-trimethoxyphenyl)tetrahydrofuran-3-yl)methanol (2760),
(*Z*)-2-Methyl-2-butensäure-((*2S*,*3R*,*4R*)-4-(4-(*tert*-butyl)benzyl)-2-(4-fluorphenyl)-tetrahydrofuran-3-yl)methylester (3010),
(*2S,3R,4R*)-3-((Allyloxy)methyl)-4-(3,4-dimethoxybenzyl)-2-(3,4-dimethoxyphenyl)-tetrahydrofuran (2540),
(*Z*)-2-Methyl-2-butensäure-((*2S*,*3R*,*4R*)-2-(4-fluorphenyl)-4-(4-(trifluormethyl)benzyl)-tetrahydrofuran-3-yl)methylester (3016),
((*2S,3R,4R*)-2-(3,4-Dimethoxyphenyl)-4-(4-methylbenzyl)tetrahydrofuran-3-yl)-methanol (2754),
3-Methyl-2-butensäure-((*2S,3R,4R*)-4-(3,4-dimethoxybenzyl)-2-(3,4,5-trimethoxyphenyl)tetrahydrofuran-3-yl)methylester (2755),
((*2S*,*3R*,*4R*)-4-Benzyl-2-(3,4,5-trimethoxyphenyl)tetrahydrofuran-3-yl)methanol (2756),
(*Z*)-2-Methyl-2-butensäure-((*2S*,*3R*,*4R*)-2-phenyl-4-(4-(trifluormethyl)benzyl)-tetrahydrofuran-3-yl)methylester (3015),
3-Methylbuttersäure-((*2S,3R,4R*)-4-(3,4-dimethoxybenzyl)-2-(4-fluorphenyl)-tetrahydrofuran-3-yl)methylester (2862),
(*Z*)-2-Methyl-2-butensäure-((*2S*,*3R*,*4R*)-4-(4-fluorbenzyl)-2-(4-fluorphenyl)tetrahydrofuran-3-yl)methylester (2864),
Buttersäure-((*2S,3R,4R*)-4-(3,4-dimethoxybenzyl)-2-(3,4,5-trimethoxyphenyl)-tetrahydrofuran-3-yl)methylester (2629),
(*E*)-2-Methyl-2-butensäure-((*2S,3R,4R*)-4-(3,4-dimethoxybenzyl)-2-(3,4,5-trimethoxyphenyl)tetrahydrofuran-3-yl)methylester (2628),
(*Z*)-2-Methyl-2-butensäure-((*2S,3R*,*4R*)-2-(3,4-dimethoxyphenyl)-4-(pyridin-2-yl-methyl)tetrahydrofuran-3-yl)methylester (2823),
(*E*)-2-Butensäure-((*2S*,*3R*,*4R*)-4-(3,4-dimethoxybenzyl)-2-(3,4-dimethoxyphenyl)-tetrahydrofuran-3-yl)methylester (2549),
((*2S,3R,4R*)-4-(Biphenyl-4-ylmethyl)-2-(3,4,5-trimethoxyphenyl)tetrahydrofuran-3-yl)-methanol (2767),
((*2S,3R,4R*)-4-(4-(1,1-Difluorethyl)benzyl)-2-(3,4-dimethoxyphenyl)tetrahydrofuran-3-yl)methanol (3013),
Cyclobutancarbonsäure-((*2S*,*3R*,*4R*)-4-(3,4-dimethoxybenzyl)-2-(4-fluorphenyl)-tetrahydrofuran-3-yl)methylester (2863),
Cyclopentancarbonsäure-((*2S,3R,4R*)-4-(3,4-dimethoxybenzyl)-2-(3,4,5-trimethoxyphenyl)tetrahydrofuran-3-yl)methylester (2635),
Cyclopropancarbonsäure-((*2S,3R,4R*)-4-(3,4-dimethoxybenzyl)-2-(3,4,5-trimethoxyphenyl)tetrahydrofuran-3-yl)methylester (2637),
((*2S,3R,4R*)-2-(3,4-Dimethoxyphenyl)-4-(naphthalin-1-ylmethyl)tetrahydrofuran-3-yl)-methanol (2821),
((*2S,3R,4R*)-4-(4-Butylbenzyl)-2-(3,4-dimethoxyphenyl)tetrahydrofuran-3-yl)methanol (3008),
2-(Adamantan-1-yl)essigsäure-((*2S,3R,4R*)-4-(3,4-dimethoxybenzyl)-2-(3,4-dimethoxyphenyl)tetrahydrofuran-3-yl)methylester (2745),
(*Z*)-2-Methyl-2-butensäure-((*2S*,*3R*,*4R*)-4-(4-(*tert*-butyl)benzyl)-2-(3,4-dimethoxyphenyl)tetrahydrofuran-3-yl)methylester (2788),
(*Z*)-2-Methyl-2-butensäure-((*2S*,*3R*,*4R*)-4-(4-acetylbenzyl)-2-(3,4,5-trimethoxyphenyl)tetrahydrofuran-3-yl)methylester (2771),
(*Z*)-2-Methyl-2-butensäure-((*2S*,*3R*,*4R*)-4-(4-(*tert-*butyl)benzyl)-2-(3,4,5-trimethoxyphenyl)tetrahydrofuran-3-yl)methylester (2765),
3-Methyl-2-butensäure-((*2S*,*3R*,*4R*)-4-(3,4-dimethoxybenzyl)-2-(3,4-dimethoxyphenyl)tetrahydrofuran-3-yl)methylester (2738),
4-(((*2S*,*3R*,*4R*)-4-((((*Z*)-2-Methyl-2-butenoyl)oxy)methyl)-5-(3,4,5-trimethoxyphenyl)-tetrahydrofuran-3-yl)methyl)benzoesäureethylester (2774),
Cyclopenten-1-carbonsäure-((*2S,3R,4R*)-4-(3,4-dimethoxybenzyl)-2-(3,4,5-trimethoxyphenyl)tetrahydrofuran-3-yl)methylester (2636),
Zimtsäure-((*2S,3R,4R*)-4-(3,4-dimethoxybenzyl)-2-(3,4-dimethoxyphenyl)tetrahydrofuran-3-yl)methylester (2739) und
Cyclopentancarbonsäure-((*2S*,*3R*,*4R*)-4-(4-(*tert-*butyl)benzyl)-2-(3,4-dimethoxyphenyl)tetrahydrofuran-3-yl)methylester (2867).

Und besonders bevorzugt ist die Verbindung aus der folgenden Gruppe ausgewählt, die 15 Verbindungen umfasst, die SMC zumindest fünfmal so stark hemmten wie EC, d.h. ein Wirksamkeitsverhältnis von zumindest 5,0 aufweisen, wobei für die bislang beste der getesteten neuen Substanzen, d.h. die jeweils erstgenannte Verbindung (2632), sogar ein Wert von über 73 erzielt wurde:
Isobuttersäure-((*2S,3R,4R*)-4-(3,4-dimethoxybenzyl)-2-(3,4,5-trimethoxyphenyl)-tetrahydrofuran-3-yl)methylester (2632),
(*Z*)-2-Methyl-2-butensäure-((*2S*,*3R*,*4R*)-4-(4-butylbenzyl)-2-(3,4-dimethoxyphenyl)-tetrahydrofuran-3-yl)methylester (3026),
((*2S,3R,4R*)-4-(4-(Trifluormethyl)benzyl)-2-(3,4,5-trimethoxyphenyl)tetrahydrofuran-3-yl)methanol (2760),
(*Z*)-2-Methyl-2-butensäure-((*2S*,*3R*,*4R*)-4-(4-(*tert-*butyl)benzyl)-2-(4-fluorphenyl)-tetrahydrofuran-3-yl)methylester (3010),
(*2S,3R,4R*)-3-((Allyloxy)methyl)-4-(3,4-dimethoxybenzyl)-2-(3,4-dimethoxyphenyl)-tetrahydrofuran (2540),
(*Z*)-2-Methyl-2-butensäure-((*2S*,*3R*,*4R*)-2-(4-fluorphenyl)-4-(4-(trifluormethyl)benzyl)-tetrahydrofuran-3-yl)methylester (3016),
((*2S,3R,4R*)-2-(3,4-Dimethoxyphenyl)-4-(4-methylbenzyl)tetrahydrofuran-3-yl)-methanol (2754),
3-Methyl-2-butensäure-((*2S,3R,4R*)-4-(3,4-dimethoxybenzyl)-2-(3,4,5-trimethoxyphenyl)tetrahydrofuran-3-yl)methylester (2755),
((*2S*,*3R*,*4R*)-4-Benzyl-2-(3,4,5-trimethoxyphenyl)tetrahydrofuran-3-yl)methanol (2756),
(*Z*)-2-Methyl-2-butensäure-((*2S*,*3R*,*4R*)-2-phenyl-4-(4-(trifluormethyl)benzyl)-tetrahydrofuran-3-yl)methylester (3015),
3-Methylbuttersäure-((*2S,3R,4R*)-4-(3,4-dimethoxybenzyl)-2-(4-fluorphenyl)-tetrahydrofuran-3-yl)methylester (2862),
(*Z*)-2-Methyl-2-butensäure-((*2S*,*3R*,*4R*)-4-(4-fluorbenzyl)-2-(4-fluorphenyl)tetrahydrofuran-3-yl)methylester (2864),
Buttersäure-((*2S,3R,4R*)-4-(3,4-dimethoxybenzyl)-2-(3,4,5-trimethoxyphenyl)-tetrahydrofuran-3-yl)methylester (2629),
(*E*)-2-Methyl-2-butensäure-((*2S,3R,4R*)-4-(3,4-dimethoxybenzyl)-2-(3,4,5-trimethoxyphenyl)tetrahydrofuran-3-yl)methylester (2628) und
(*Z*)-2-Methyl-2-butensäure-((*2S*,*3R*,*4R*)-2-(3,4-dimethoxyphenyl)-4-(pyridin-2-yl-methyl)tetrahydrofuran-3-yl)methylester (2823).

Aufgrund der erwähnten Schwankungen ist jedoch nicht auszuschließen, dass sich im Verlauf zukünftiger Aktivitätstests noch weitere, mittels Kombination der obigen Substituenten in Formel (II) erhaltene Verbindungen als entsprechend aktiv herausstellen werden, die bisher unter dem Schwellenwert von 1,5 lagen.

Ein die Verbindung der Formel (II) umfassendes Mittel zur selektiven Hemmung der Proliferation von Glattmuskelzellen (SMC) gegenüber Endothelzellen (EC) ist somit vorzugsweise ein Arzneimittel, das zur Behandlung eines menschlichen oder tierischen Patienten dient, der einer solchen Behandlung bedarf. Besonders bevorzugt dient das Arzneimittel zur Behandlung von oder Prophylaxe gegen Hyperplasie, insbesondere Intimahyperplasie, Stenose oder Restenose.

Die Art der Formulierung und Verabreichung des Arzneimittels ist dabei nicht speziell eingeschränkt, solange die Wirksamkeit der Verbindung der Formel (II) nicht beeinträchtigt wird. So kann die Verabreichung beispielsweise systemisch oder topisch; z.B. oral, rektal, parenteral, intrazisternal, intravaginal, intraperitoneal, intravenös, intraarteriell, intramuskulär, intrakardial, intrapulmonal, intravesikal, intravitreal, subkutan, sublingual, intranasal oder transdermal mittels Pflastern erfolgen - je nachdem, an welcher Stelle des Körpers die Hyperplasie bekämpft werden soll.

In bevorzugten Ausführungsformen dient das Arzneimittel zur Beschichtung oder Imprägnierung eines Stents oder anderen Implantats, der/das in der Folge in den Körper des Patienten transplantiert wird. Das Implantat kann künstlich erzeugt oder aber ein Gewebe, Organ oder Teil eines Organs, insbesondere ein Abschnitt einer Vene, sein, das/der einem Patienten entnommen wurde und nach Beschichtung mit dem Arzneimittel dem Patienten reimplantiert wird.

Besonders bevorzugt dient das Arzneimittel zur Beschichtung von eingangs erwähnten, so genannten "drug eluting", d.h. arzneimittelbeschichteten, Stents, die z.B. in ein von Stenose betroffenes Blutgefäß des Patienten eingesetzt werden, um dieses aufzuweiten. Das Arzneimittel kann in der Folge wirksam eine Restenose durch "Einwachsen" von Glattmuskelzellen in das Lumen des Stents verhindern. Aufgrund der selektiven Wirkung der Verbindungen der vorliegenden Erfindung wird gleichzeitig die Heilung der beim Einsetzen des Stents üblicherweise verletzten *Tunica intima,* d.h. der monozellulären Schicht aus Endothelzellen, nicht oder kaum gehemmt.

Außerdem stellt die Offenbarung daher eine für eine solche Behandlung von oder Prophylaxe gegen Hyperplasie, Stenose oder Restenose mittels selektiver Hemmung der Proliferation von Glattmuskelzellen (SMC) in höherem Ausmaß als jener von Endothelzellen (EC) geeignete pharmazeutische Zusammensetzung bereit, die eine Verbindung wie zuvor definiert sowie einen pharmazeutisch annehmbaren Träger oder Exzipienten und gegebenenfalls ein oder mehrere Adjuvantien und/oder einen oder mehrere andere Wirkstoffe umfasst. Ein Fachmann auf dem Gebiet der Pharmazie ist problemlos in der Lage, ohne übermäßiges Experimentieren, sondern durch Standard-Routineversuche geeignete Formulierungen für den jeweiligen Zweck zu entwickeln. Anleitungen dazu finden sich in zahlreichen pharmazeutischen Standardwerken, wie z.B. "Remington: Essentials of Pharmazeutics", L. Felton (Hrsg.), Pharmaceutical Press, Gurnee, IL (2013).

Weiters betrifft die Offenbarung die Verwendung einer Verbindung der Formel (II) wie hierin zuvor definiert zur selektiven Hemmung der Proliferation von Glattmuskelzellen (SMC) gegenüber Endothelzellen (EC) ex vivo bzw. in vitro, wie z.B. in diversen Assayformaten.

Darüber hinaus stellt die Offenbarung ein Verfahren zum Beschichten eines Körperimplantats durch Aufbringen eines Arzneimittels auf zumindest eine Oberfläche des Implantats bereit, das dadurch gekennzeichnet ist, dass als Arzneimittel eine Verbindung der Formel (II) wie hierin zuvor definiert aufgebracht wird.

Bei dem Implantat handelt es sich vorzugsweise um einen arzneimittelbeschichteten ("drug eluting") Stent, der mit einem solchen Arzneimittel beschichtet ist. Die hierzu notwendigen Schritte sind dem Fachmann bekannt, können aber beispielsweise auch dem "Handbook of Coronary Stents", P. W. Serruys, B. Rensing, S. W. Serruys (Hrsg.), Informa Healthcare (November 2001), oder dem "Handbook of Drug-eluting Stents", P. W. Serruys und A. H. Gershlick (Hrsg.), Informa Healthcare (Juni 2005), entnommen werden. Ein solcher Stent stellt einen weiteren Aspekt der Offenbarung dar.

Die vorliegende Erfindung stellt nun ein Verfahren zur Herstellung von Verbindungen der Formel (II) wie hierin zuvor definiert bereit, d.h. sowohl von völlig neuen Verbindungen als auch von Verbindungen, die unter die Definition von Formel (Ia) aus WO 2010/007169 A1 (wenn X für O steht) fallen, wobei das Verfahren durch die folgenden Reaktionsschritte a) bis h) gekennzeichnet ist:
a) die an sich bekannte Umsetzung eines mit den entsprechenden Resten R₄ bis R₆ substituierten Benzaldehyds der Formel (1) mit Vinylmagnesiumbromid zum entsprechenden racemischen α-Vinylbenzylalkohol *rao-*(2): 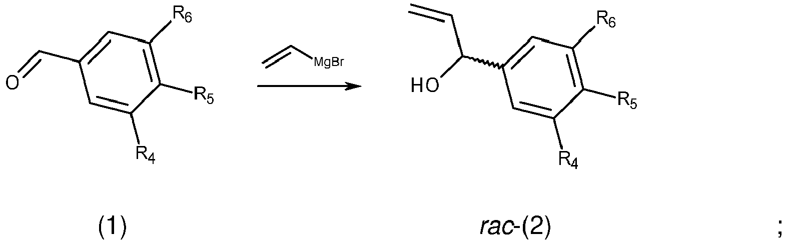
b) kinetische Racematspaltung von *rac-*(2) mittels enzymatischer Katalyse zum Erhalt des (S)-Enantiomers (S)-(2) unter gleichzeitiger Bildung von (R)-Rs-(2) als abzutrennendes Nebenprodukt, worin R₈ vorzugsweise für einen Acylrest steht: 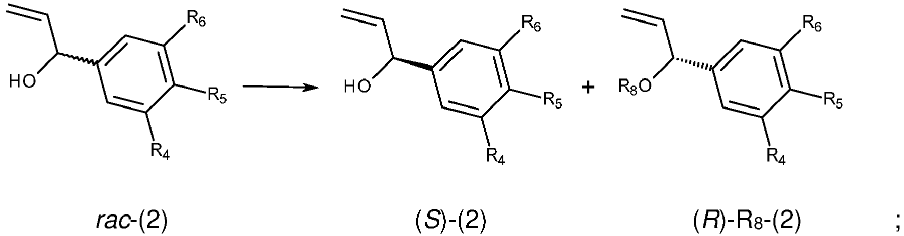
c) anschließende Veretherung und Oxidation von (*S*)-(2) in beliebiger Reihenfolge, d.h. entweder:
   c1) Veretherung von (S)-(2) mit Propargylbromid zum Propargylether (3): 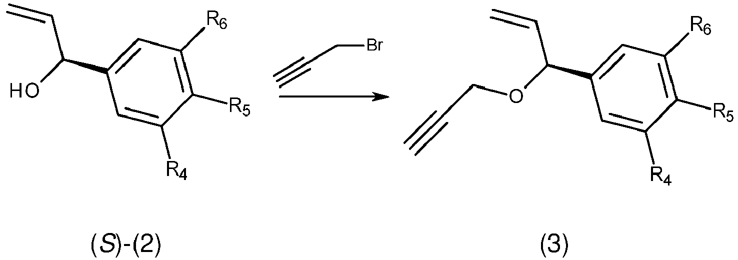 und darauf folgende Oxidation von (3) zum Propargylether-Epoxid (5): 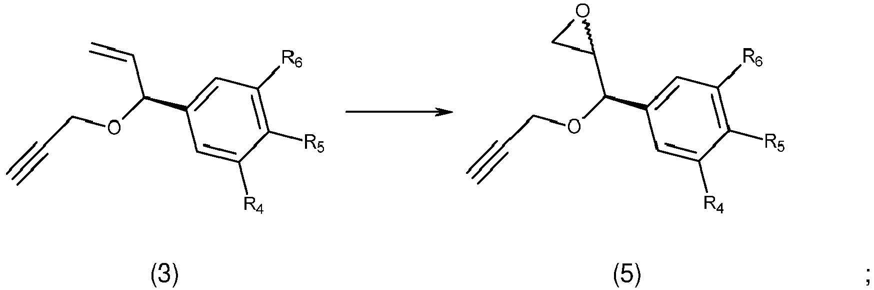 oder umgekehrt:
   c2) Oxidation von (S)-(2) zum Epoxid (4): 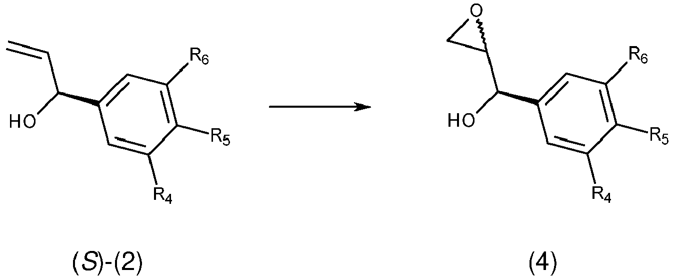 und darauf folgende Veretherung von (4) mit Propargylbromid zum Propargylether-Epoxid (5): 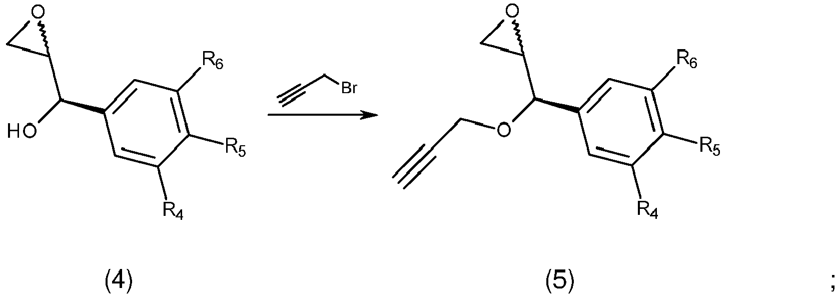
d) Zyklisierung von (5) mithilfe eines Zyklisierungsmittels zum Tetrahydrofuranylmethanol (6): 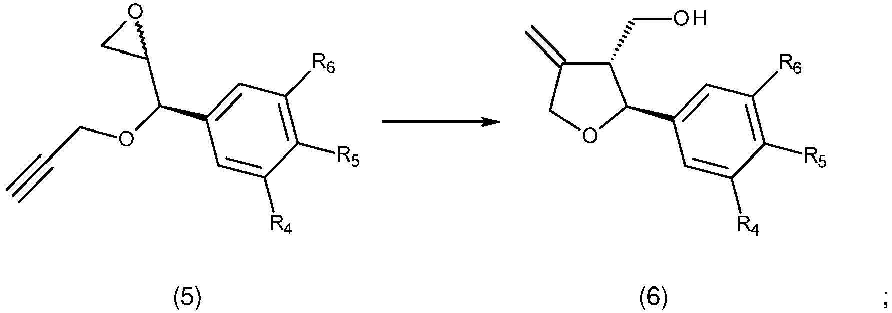
e) Schützen der Alkoholfunktionalität von (6) mit der tert-Butyldimethylsilyl- (TBDMS-) Gruppe zum geschützten Methanol (7): 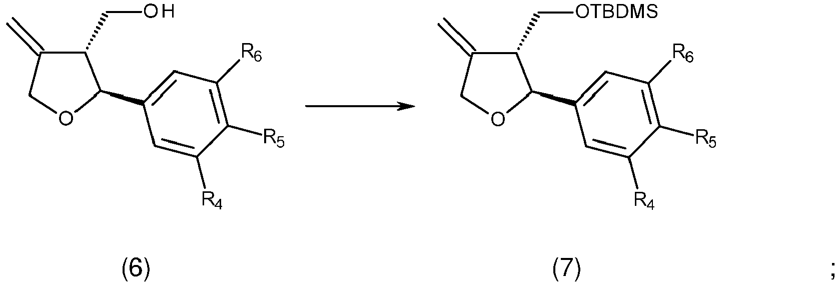
f) Einführung eines mit den entsprechenden Resten R₁ bis R₃ substituierten Phenyl- oder 2-Pyridylrests (7b) durch Reaktion mit der exozyklischen Methylengruppe von (7) zum geschützten Produkt (8): 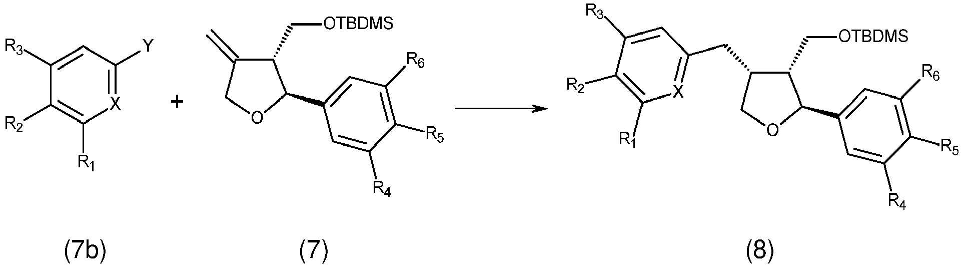 worin X jeweils für CH oder N steht und Y für eine Abgangsgruppe steht;
g) Abspaltung der Schutzgruppe von (8) zum freien Alkohol (9): 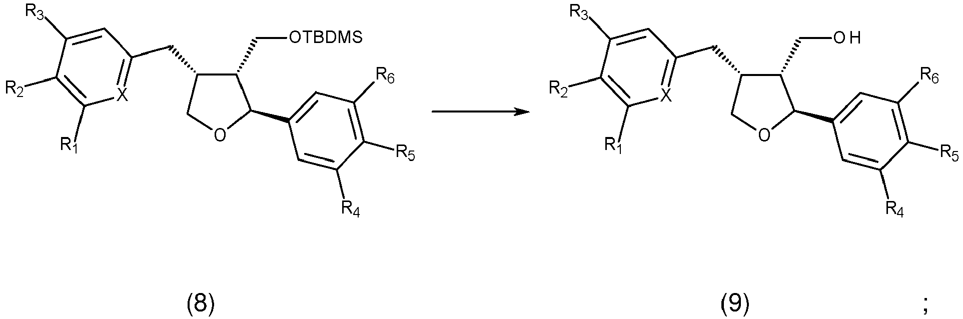
h) wenn R₇ ≠ OH ist, die gleichzeitige oder anschließende Veretherung oder Veresterung der freien OH-Gruppe, um Verbindung (10) mit dem entsprechenden Rest R₇ zu erhalten:

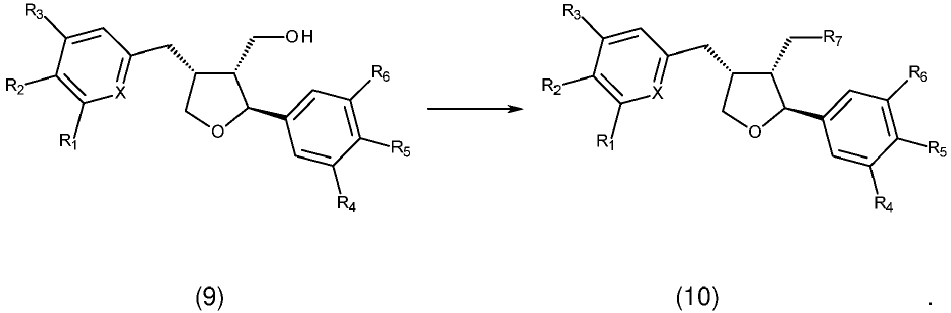

Dieser Syntheseweg bietet gegenüber dem in WO 2010/007169 A1 und Roy et al. (s.o.) offenbarten Herstellungsverfahren mehrere entscheidende Vorteile, vor allem die nachstehend unter 1) bis 6) angeführten:
1) Bereits im zweiten Syntheseschritt b) erfolgt eine Racematspaltung, um das gewünschte (S)-Isomer des Zwischenprodukts (2) zu erhalten, das die mit den Resten R₄ bis R₆ substituierte, später an Position 2 des Tetrahydrofurans sitzende Phenylgruppe in der richtigen räumlichen Ausrichtung enthält.
2) Die Zyklisierung in Schritt d) erfolgt im Gegensatz zu WO 2010/007169 A1 und Roy et al. (s.o.) vor der Anbindung des mit R₁ bis R₃ substituierten Arylrests an das ringgeschlossene Tetrahydrofuran. Dies ermöglicht eine deutlich einfachere Variation der Substituenten R₁ bis R₇, da ein zyklisiertes und verestertes Zwischenprodukt (7), das eine definierte Kombination aus R₄ bis R₆ umfasst, in einem einzigen oder sehr wenigen Schritten mit unterschiedlichen Verbindungen (7b), die ihrerseits jeweils eine bestimmte Kombination aus R₁ bis R₃ umfassen, zu einer Vielzahl von Zwischenprodukten (8) kombiniert werden kann.

Diese können in der Folge (nach Abspaltung der Schutzgruppe) durch eine einfache Veresterungsreaktion mit einer breiten Palette von Säureresten zu jeweils einem neuen Derivat (10) der Formel (II) umgesetzt werden. Und für den Fall, dass die OH-Schutzgruppe zusammen mit dem ethanolischen Sauerstoff bereits den späteren Substituenten R₇ bildet, kann dies sogar entfallen, da dann bereits das Zwischenprodukt (8) der Formel (II) entspricht. Die Erzeugung einer großen Bibliothek von auf ihre Wirksamkeit zu testenden Derivaten der Formel (II) ist somit gemäß vorliegender Erfindung mit einem Bruchteil des Syntheseaufwands möglich.
3) Weiters liefert die Zyklisierung in Schritt d) zwischen Epoxid und Alkinylgruppe unter Ausbildung einer exozyklischen Methylengruppe kein 5:1-Gemisch racemischer Diastereomere wie die in WO 2010/007169 A1 und Roy et al. (s.o.) beschriebene Zyklisierung zwischen Epoxid und phenylsubstituierter Alkenylgruppe.

Die im Verfahren der vorliegenden Erfindung durchgeführte Zyklisierung wurde ebenfalls bereits von der Arbeitsgruppe um Subhas Chandra Roy beschrieben: für einen Alkylsubstituenten am Tetrahydrofuran in G. Maiti und S. C. Roy, J. Chem. Soc., Perkin Trans. 1, 403-404 (1996) (kurz: Maiti et al.), und für diverse Substituenten, darunter auch das zu Leoligin bzw. Dimethyllariciresinol führende Dimethoxyphenyl, in P. K. Mandal, G. Maiti, S. C. Roy, J. Org. Chem. 63, 2829-2834 (1998) (kurz: Mandal et al.).

Für 2-Alkylsubstituenten an den dabei entstehenden Tetrahydrofuranen werden in den beiden letzteren Publikationen Diastereomeren-Verhältnisse von 5:1 und in Mandal et al. auch von 6:1 angegeben, während sperrigere Gruppierungen wie (substituiertes) Phenyl oder Naphthyl im Wesentlichen nur das gewünschte Diastereomer mit 2,3-trans-Konfiguration der Substituenten an den beiden chiralen Zentren ergaben. Dies konnten die vorliegenden Erfinder mit ihren Synthesen, in denen durchwegs mit R₄ bis R₆ substituiertes Phenyl als Substituent zum Einsatz kam, bestätigen.

Neben der Sperrigkeit des Substituenten an Position 2 des Tetrahydrofurans trägt aber natürlich auch die Abwesenheit des dritten chiralen Zentrums an C4 zur Stereoselektivität der Zyklisierungsreaktion bei. Dieses wird gemäß vorliegender Erfindung ja erst im späteren Schritt f) in das Molekül eingeführt.
4) Durch die Verwendung einer sperrigen Schutzgruppe in Schritt e) wird die Stereoselektivität bei der nachfolgenden Funktionalisierung der exozyklischen Methylengruppe enorm erhöht. Neben der vorzugsweise als Schutzgruppe eingesetzten tert-Butyldimethylsilyl-Gruppe, die sich neben ihrem Raumbedarf auch durch Kostengünstigkeit, chemische Stabilität und dennoch leichte Abspaltbarkeit auszeichnet, können auch andere Silyl-Schutzgruppen, wie z.B. Triisopropylsilyl- (TIPS) und tert-Butyldiphenylsilyl- (TBDPS), oder generell andere Hydroxschutzgruppen, wie z.B. acylische, acetalische, ketalische, benzylische oder alkylische OH-Schutzgruppen, eingesetzt werden, wenn diese auch mitunter schlechtere Ergebnisse im nachfolgenden Schritt f) bewirken können.
5) Nicht nur, aber speziell wenn R₇ einen relativ sperrigen Substituenten darstellt, kann anstelle einer Schutzgruppe auch sofort der zu R₇ führende Säurerest mit der freien OH-Gruppe verestert werden, was die Synthese um zwei Schritte verkürzt, da die Entfernung der Schutzgruppe und die neuerliche Veresterung in den Schritten g) und h) entfallen können.

Dasselbe gilt sinngemäß auch, wenn die OH-Gruppe nicht zu verestern, sondern zu verethern ist, um R₇ zu bilden. In den bisherigen Beispielen der Erfindung wurde der Alkohol allerdings nur mit Allyl bzw. Propargyl verethert, die beide zu wenig sperrig sind, um eine ausgeprägte dirigierende Wirkung bei der Addition in Schritt f) entfalten zu können, und zudem unerwünschte Nebenreaktionen in Schritt f) eingehen können. Beispielsweise mit tert-Butyl- oder (Hetero-)Aryl-Resten könnte jedoch auch die Veretherung von Schritt h) in Schritt e) vorgezogen und die Schritte g) und h) entfallen gelassen werden.
6) Ein wesentlicher Schritt und Vorteil im Verfahren der vorliegenden Erfindung ist die Funktionalisierung der exozyklischen Doppelbindung in Schritt f). Wie bereits oben erwähnt, erhöht eine sperrige OH-Schutzgruppe (bzw. ein sperriger Ester oder Ether als Substituent R₇) die Stereoselektivität dieses Schritts. Unter Ausnutzung der Anwesenheit dieser Schutzgruppe sind mehrere Möglichkeiten der stereoselektiven Funktionalisierung gegeben: i) Prä-Funktionalisierung der Doppelbindung mit einem geeigneten Reagens, dessen Additionsprodukt *in situ* oder nach Aufarbeitung mit einem geeigneten Reaktionspartner zur Einführung des mit R₁ bis R₃ substituierten Arylrests umgesetzt werden kann, wie z.B. eine Hydrometallierung der Doppelbindung mit nachfolgender Kupplungsreaktion; ii) Epoxidierung der Doppelbindung mit nachfolgender Ringöffnung durch ein geeignetes Nucleophil und anschließender Entfernung der so entstandenen OH-Gruppe; iii) Funktionalisierung unter Erhalt der Doppelbindung, z.B. mittels der Heck-Reaktion oder CH-Aktivierung, insbesondere sp2-sp2-CH-Aktivierung, mit nachfolgender Reduktion; oder iv) Funktionalisierung der Doppelbindung unter gleichzeitiger Überführung in eine Einfachbindung, z.B. durch reduktive Heck-Reaktion oder Direkt-Funktionalisierung, sofern der Übergang zur Einfachbindung mit der gewünschten Stereochemie bewirkt werden kann.

Weitere mögliche und bevorzugte Varianten der Schritte des erfindungsgemäßen Verfahrens werden nachstehend näher beschrieben.

Gemäß einer bevorzugten Ausführungsform der Erfindung werden in Schritt b) als Enzym Amano-Lipase PS und als deren Donor-Komponente Vinylacetat oder Isopropenylacetat eingesetzt, so dass der Rest R₈ ein Acetylrest ist und als Nebenprodukt (R)-Acetyl-(2) anfällt.

Ganz allgemein zeichnen sich enzymatisch katalysierte Reaktionen durch hohe Stereoselektivität aus - eine Eigenschaft, die sich die vorliegende Erfindung in Schritt b) zunutze macht, um bereits in einem frühen Stadium der Synthese die Stereochemie eines der drei Chiralitätszentren am späteren Tetrahydrofuran-Ring festzulegen. Da außerdem das unerwünschte Enantiomer das reagierende und das erwünschte Enantiomer das zurückbleibende ist, kann ein Enatiomeren-Überschuss ("enantiomeric excess", e. e.) von nahezu 100 % erzielt werden.

Vorzugsweise wird dabei Lipase PS von Amano Enzyme Inc. als Enzym mit Vinyl- oder Isopropenylacetat als Donor oder Co-Substrat eingesetzt, da hiermit die besten Ausbeuten erzielt werden konnten. Das Lipase-Präparat umfasst ein auf Diatomeenerde immobilisiertes lipolytisches Enzym, das in Gegenwart von Vinylacetat die Acetylgruppe selektiv auf das (*R*)-Isomer von rac-(2) überträgt, wonach das dabei gebildete (*R*)-Acetyl-(2) vom gewünschten Alkohol (*S*)-(2) leicht abzutrennen ist, z.B. mittels Flashchromatographie, und das auch in größeren Reaktionsansätzen (> 50 g).

Als Alternative zu Lipase PS könnten beispielsweise auch Lipase CALB-L (immobilisierte *Candida-antarctica-Lipase* B, auch als Novozymes 435 im Handel erhältlich) oder andere, für diesen Zweck geeignete Enzyme, insbesondere andere Lipasen, Esterasen, Proteasen oder Acyl-Transferasen, eingesetzt werden, die dem Fachmann geläufig sind. In der Literatur ist eine entsprechende Veresterung mittels Novozymes 435 beispielsweise in Stambasky et al., J. Org. Chem. 73(22), 9148-9150 (2008), beschrieben.

Wie oben erwähnt können die nachfolgende Veretherung des Benzylalkohols zum Propargylether und die Oxidation der Vinylgruppe zum Epoxid in beliebiger Reihenfolge durchgeführt werden. In Schritt c1) wird die obige Reihenfolge eingehalten, im alternativen Schritt c2) wird Zwischenprodukt (*S*)-(2) hingegen zuerst oxidiert und danach verethert.

Die Propargylierung erfolgt in beiden Fällen vorzugsweise mit Propargylbromid, wie dies z.B. auch bereits von Mandal et al. (s.o.) beschrieben wird. Alternativ dazu können allerdings auch andere den Propargylrest positivierende Substituenten eingesetzt werden, wie z.B. Chlorid, lodid, Tosylat oder Mesylat, wie dies für Williamson-Veretherungen allgemein bekannt ist. Die Erzeugung des Alkoholats des Benzylalkohols (3) bzw. (4) kann dabei wie üblich z.B. mittels starker Basen, vorzugsweise NaH oder KH, oder auch Lithiumdiisopropylamid (LDA), Butyllithium (BuLi) oder Kaliumtert-butanolat (KO-t-Bu, KTB), erfolgen.

In Schritt c1) wird dabei die nach der Propargylierung erfolgende Oxidation vorzugsweise mit m-Chlorperbenzoesäure als Oxidationsmittel durchgeführt, wie dies ebenfalls von Mandal et al. (s.o.) beschrieben wird, da dies eine bewährte und vergleichsweise kostengünstige Reaktion darstellt. Diese liefert zwar ein Diastereomerengemisch der beiden möglichen Epoxide, allerdings spielt die Stereochemie am in β-Position zum Phenylrest liegenden Kohlenstoffatom keine Rolle, da in der nachfolgenden Zyklisierung an dieser Stelle ein trigonal-planares C-Radikal gebildet und die gewünschte 2,3-trans-Selektivität ausschließlich durch den Arylrest in 2-Position bedingt wird.

Erfindungsgemäß bevorzugt ist allerdings die umgekehrte Reihenfolge, d.h. die Durchführung von Schritt c2), wobei die vor der Propargylierung erfolgende Oxidation als Sharpless-Epoxidierung mit (*S,S*)-Diethyltartrat, (-)-DET, als Co-Reagens, tert-Butylhydroperoxid, TBHP, als Oxidationsmittel und Titan(IV)-isopropanolat, Ti(O-i-Pr)₄, als Katalysator durchgeführt wird, da diese bei elektronenreichen Aromaten bessere Ausbeuten ermöglicht als die Oxidation mit m-Chlorperbenzoesäure. Die anschließende Propargylierung erfolgt wiederum vorzugsweise mit Propargylbromid.

Bei der darauf folgenden Zyklisierung des propargylierten Epoxids (5) in Schritt d) wird vorzugsweise, wie aus Mandal et al. bekannt, Titanocendichlorid (Dichlorobis-(cyclopentadienyl)titan(IV), Cp₂TiCl₂) als Zyklisierungsmittel eingesetzt, da diese Reaktionsführung, wie zuvor erwähnt, im Wesentlichen nur das gewünschte jeweilige Stereoisomer ergibt. Alternativ dazu, wenn auch nicht bevorzugt, kann auch ein Trialkylstannan in Kombination mit Azobisisobutyronitril, AIBN, als radikalisches Zyklisierungsmittel dienen, wenn das Epoxid zuvor säurekatalystiert in Anwesenheit von Bromid geöffnet oder statt der Epoxidierung eine (formale) Addition von Hypobromiger Säure an die vinylische Doppelbindung durchgeführt wird, um in β-Position einen Br-Substituenten einzuführen.

Wie bereits erwähnt kann, wenn R₇ ≠ OH ist, in Schritt e) zum Schützen der OH-Gruppe diese in bevorzugten Ausführungsformen mit jenem Acylrest verestert - oder mit jenem Alkyl- oder Arylrest verethert - werden, der zusammen mit dem Sauerstoffatom den jeweiligen Rest R₇ ergibt, wonach die späteren Schritte g) und h) entfallen können. Dies gilt insbesondere für sperrige Reste, damit diese bei der späteren Addition an die exozyklische Methylengruppe eine dirigierende Wirkung entfalten können, um im Wesentlichen nur das gewünschte Stereoisomer zu erhalten.

In Schritt f) wird gemäß bevorzugten Ausführungsformen der vorliegenden Erfindung eine Suzuki-Kupplung durchgeführt, bei der die exozyklische Methylengruppe von (7) zunächst in einem Schritt f1) intermediär mit einem Organoboran stereoselektiv zum Zwischenprodukt (7a) präfunktionalisiert, wobei insbesondere eine Hydroborierung als bevorzugte Ausführungsform der bereits zuvor erwähnten Hydrometallierung durchgeführt wird, wonach (7a) unmittelbar darauf in einem Schritt f2) mit Verbindung (7b), worin X für ein Halogen oder Pseudohalogen (z.B. Triflat oder Nonaflat) steht, umgesetzt wird, um den Borylrest durch den mit R₁ bis R₃ substituierten Phenyl- oder 2-Pyridylrest zu ersetzen.

Dieses Verfahren der Präfunktionalisierung hat den Vorteil, dass die gewünschte Stereoselektivität unabhängig - weil allein durch die Schutzgruppe bewirkt - vom sterischen Anspruch des zu kuppelnden Partners (7b) ist. Vor allem stellt die Hydroborierung als Präfunktionalisierung eine verlässliche und einfache Methode dar, um die exozyklische Doppelbindung unter milden Bedingungen reagieren zu lassen. Auch die danach folgende Suzuki-Kupplung ist sehr unempfindlich gegenüber den elektronischen Eigenschaften der Verbindung (7b).

Mechanistisch erfolgt die Addition des Borans über einen vieratomigen Übergangszustand, bei dem sich die im Bruch befindliche B-H-Bindung parallel zur C-C-Doppelbindung ausrichtet, und zwar aus sterischen Gründen regioselektiv mit dem Dialkylboryl-Teil über dem distalen Kohlenstoffatom und dem hydridischen Wasserstoff über dem proximalen Kohlenstoffatom der Doppelbindung. Das hat zur Folge, dass ausschließlich das Intermediat (7a) mit der entsprechenden Regioselektivität gebildet wird. Hinsichtlich der 3,4-cis-Stereoselektivität der Reaktion hängt das Ergebnis vom sterischen Anspruch der OH-Schutzgruppe ab, wobei mit der bevorzugten Ausführungsform TBDMS Diastereoselektivitäten von bis zu 97:3 erzielt wurden.

Bei weniger sperrigen Schutzgruppen steigt die Wahrscheinlichkeit eines Angriffs mit resultierender trans-Konfiguration, sodass bei der Addition Stereoisomerengemische in (mitunter deutlich) schlechteren Verhältnissen erhalten werden.

Aus diesem Grund wird in Schritt f1) die Verbindung der Formel (7) besonders bevorzugt mit 9-Borabicyclo[3.3.1]nonan, 9-BBN, umgesetzt, um intermediär das Boran (7a) zu erhalten: 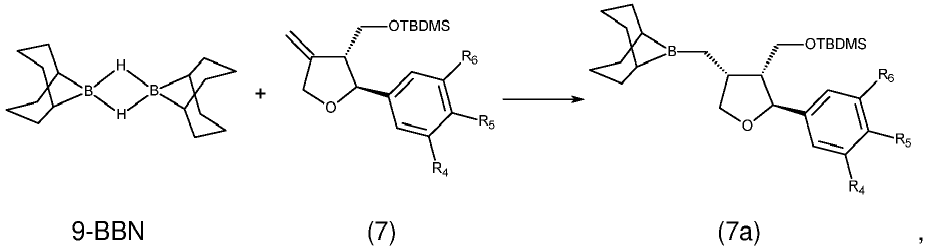 das im unmittelbar folgenden Schritt f2) mit dem entsprechend substituierten Phenyl oder 2-Pyridylbromid (7b) unter Verwendung von 1,1'-Bis(diphenylphosphino)ferro-cendichloropalladium(II)-Dichlormethan-Komplex, Pd(dppf)Cl₂.CH₂Cl₂, als Katalysator zu Verbindung (8) umgesetzt wird. Diese Reaktion wird allgemein z.B. in Chen et al., Org. Lett. 12(7), 1377-1379 (2010), beschrieben.

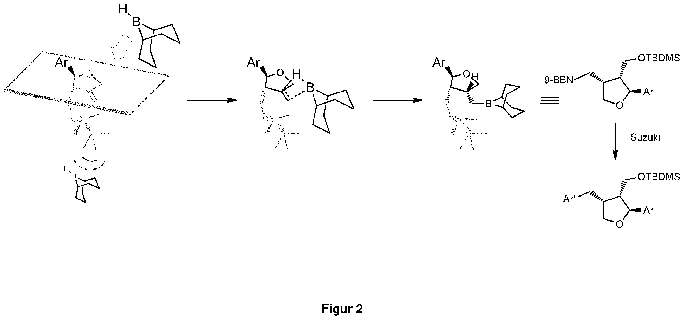

Aufgrund des voluminösen Borylrests und der Sperrigkeit der (in Bezug auf die Blattebene) "nach unten" weisenden Schutzgruppe kann der Angriff auf die Doppelbindung im Wesentlichen nur "von oben" erfolgen. Da der Hydrid-Wasserstoff mit dem proximalen Kohlenstoffatom reagiert, wird dieser demnach oberhalb der Ebene dieses planar sp2-hybridisierten Ring-Kohlenstoffatoms gebunden, wobei sich die Hybridisierung des Ring-Kohlenstoffatoms von sp2 zur tetraedrischen sp3-Form ändert. Die Bindung zum distalen Kohlenstoffatom klappt dadurch zwangsläufig "nach unten", wodurch sich die gewünschte 3,4-cis-Konfiguration ausbildet. Eine schematische Darstellung dieses Reaktionsmechanismus findet sich in Fig. 2, wobei TBDMS als Schutzgruppe angenommen wurde.

Anschließend erfolgt die eigentliche Suzuki-Kupplung, d.h. die Verknüpfung der hydroborylierten Verbindung (7a) mit dem Halogenaromaten (7b), d.h. hierin vorzugsweise einem entsprechend substituierten Phenyl- oder 2-Pyridylbromid oder -iodid, obwohl prinzipiell auch andere Abgangsgruppen, wie z.B. Triflat, sowie andere Aromaten, wie z.B. Naphthyl oder weitere Heteroaryle, als Verbindung (7b) in analoger Weise eingesetzt werden können.

Die dabei erhaltene Verbindung (8) entspricht entweder bereits der Formel (II), wenn zuvor in Schritt e) bereits der gewünschte Acyl-, Akyl- oder Arylrest als "Schutzgruppe" an die OH-Gruppe gebunden wurde, oder es folgt in Schritt g) die Abspaltung der Alkohol-Schutzgruppe zum freien Alkohol (9). Dies erfolgt vorzugsweise mit Tetra-n-butylammoniumfluorid, TBAF, da dies eine verlässliche Methode zur Entfernung von Silyl-Schutzgruppen ist, die mit den neuen Verbindungen kompatible Reaktionsbedingungen umfasst.

Die freie OH-Gruppe von (9) wird abschließend in Schritt h) mit dem gewünschten Acyl-, Alkyl- oder Arylrest umgesetzt, was erneut eine breite Palette von in einem einzigen Schritt herstellbaren neuen Verbindungen der Formel (II) ermöglicht.

Bei geeigneter Auswahl der Reaktanten und Reaktionsführung können auch mehrere aufeinander folgende Schritte des erfindungsgemäßen Verfahrens, so z.B. die Schritte f) und g) oder auch die Schritte g) und h), als Eintopfreaktion durchgeführt werden.

Vorzugsweise wird zur Veresterung der OH-Gruppe in Schritt e) oder h) eine Mitsunobu-Veresterung oder eine Steglich-Veresterung mit einer freien Säure, die als Schutzgruppe und/oder zur Bildung des jeweiligen Rests R₇ dient, durchgeführt, wobei die Mitsunobu-Veresterung noch bevorzugter in Gegenwart von 1,1'-(Azodicar-bonyl)dipiperidin, ADD, oder Diethylazodicarboxylat, DEAD, und Triphenylphosphin, PPh₃ durchgeführt wird und die Steglich-Veresterung in Gegenwart von N-(3-Dimethylaminopropyl)-N'-ethylcarbodiimid, EDC, oder N,N'-Dicyclohexylcarbodiimid, DCC, und 4-Dimethylaminopyridin, DMAP, durchgeführt wird.

Dies ermöglicht im Vergleich zu anderen gängigen Versterungsreaktionen, dass je nach den chemischen Anforderungen entweder der Alkohol (Mitsunobu) oder die Carbonsäure (Steglich) aktiviert werden kann, wenn etwa Inkompatibilitäten der Reaktanten mit einer der Reaktionsbedingungen bestehen, z.B. bei der Aktivierung von Angelikasäure.

### KURZBESCHREIBUNG DER ZEICHNUNGEN

Die beiliegenden Zeichnungen zeigen Folgendes:
Fig. 1 den literaturbekannten Syntheseweg zur Herstellung von Leoligin und Derivaten davon;
Fig. 2 den Mechanismus der Hydroborierung in Schritt f) des erfindungsgemäßen Verfahrens; und
die Fig. 3a und 3b den Syntheseweg gemäß dem erfindungsgemäßen Verfahren im Überblick.

Nachstehend wird die vorliegende Erfindung anhand von nichteinschränkenden Beispielen und Vergleichsbeispielen detaillierter beschrieben, wobei sich die Bezeichnung "Vergleichsbeispiel" nur auf die oben erkäuterte Wirkung der jeweiligen Verbindung auf die Zellproliferation bezieht. Für die einzelnen Verfahrensschritte des hierin offenbarten erfindungsgemäßen Herstellungsverfahren sind alle der als "Synthesebeispiele", "Beispiele" und "Vergleichsbeispiele" bezeichneten nachstehenden Synthesen erfindungsgemäße Beispiele.

### BEISPIELE

### Allgemeine Anmerkungen

*Chemikalien* wurden bei handelsüblichen Anbietern erworben und ohne weitere Reinigung eingesetzt, sofern nicht anders angegeben.

*Trockene Lösungsmittel* wurden, sofern nichts anderes angegeben ist, erhalten, indem vorgetrocknetes Material mittels eines Lösungsmittelabgabesystems durch eine aktiviertes Aluminiumoxid enthaltende Patrone geleitet und unter trockenem Stickstoff gelagert wurde.

*Trockenes und sauerstofffreies THF* wurde durch Rückflusserhitzen des Lösungsmittels über Natrium/Benzophenon unter Argon und anschließende Destillation erhalten.

### Kühlung

Ein Cryostat RKT20 Lauda wurde zum Bereitstellen niederer Temperaturen für längere Niedertemperaturreaktionen verwendet.

### Präparative Chromatographie

Flash-Säulenchromatographie wurde auf einem Büchi-Sepacore-MPLC-System unter Verwendung von Kieselgel 60 (40 bis 63 µm) von Merck durchgeführt. Präparative HPLC wurde auf einem Shimadzu-LC-8A-Gerät mit einem SIL-10AP-Autosampler, einem SPD-20-Detektor und einem FRC-10A-Fraktionssammler durchgeführt.

### Spezifische Drehung

Ein Anton-Parr-MCP500-Polarimeter wurde zum Messen von spezifischen Drehungswerten verwendet.

### Hochauflösende Massenspektroskopie (E. Rosenberg, CTA, TU Wien)

Proben wurde mittels LC-IT-TOF-MS unter Verwendung von ESI (Positivionenmodus) oder APCI-PI analysiert, und die quasimolekularen M+H⁺- oder M+Na⁺-Ionen wurden zur Massenbestimmung herangezogen. LC: Shimadzu Prominence, bestehend aus einer Lösungsmittelentgasungseinheit (DGU-20 A3), einer Binärgradientenpumpe (2x LC-20AD), einem Autoinjektor (SIL-20A), einem Säulenofen (CTO-20AC), einem Steuermodul (CBM-20A) und einem Diodenarraydetektor (SPD-M20A). MS: Shimadzu IT-TOF-MS mit chemischer Ionisierung unter Atmosphärendruck und Elektrosprayschnittstelle.

### NMR-Spektroskopie

¹H- und ¹³C-NMR-Spektren wurden aus CDCl₃- oder DMSO-d₆-Lösungen auf einem Bruker AC 200 (200 MHz) oder auf einem Bruker-Avance-UltraShield-400-Spektrometer (400 MHz) aufgenommen. Chemische Verschiebungen werden in ppm angegeben.

### Abkürzungen

- akt. MS: aktivierte Molekülsiebe
- ADD: 1,1'-(Azodicarbonyl)dipiperidin
- 9-BBN: 9-Borabicyclo[3.3.1]nonan
- DEAD: Diethylazodicarboxylat
- DET: Diethyltartrat
- DIAD: Diisopropylazodicarboxylat
- DIPEA: Diisopropylethylamin
- 4-DMAP: 4-Dimethylaminopyridin
- DMSO: Dimethylsulfoxid
- DPPA: Diphenylphosphorazidat
- dppf: 1,1'-Bis(diphenylphosphino)ferrocen
- EDCl.HCl: *N*-(3-Dimethylaminopropyl)-*N*'-ethylcarbodiimid-hydrochlorid
- PE: Petrolether
- *m*CPBA: *meta*-Chlorperbenzoesäure
- MTBE: Methyl-*tert*-butylether
- TBAF: Tetra-*n*-butylammoniumfluorid
- TBDMS: *tert*-Butyldimethylsilyl
- TBHP: *tert*-Butylhydroperoxid
- THF: Tetrahydrofuran

### Allgemeines Reaktionsschema

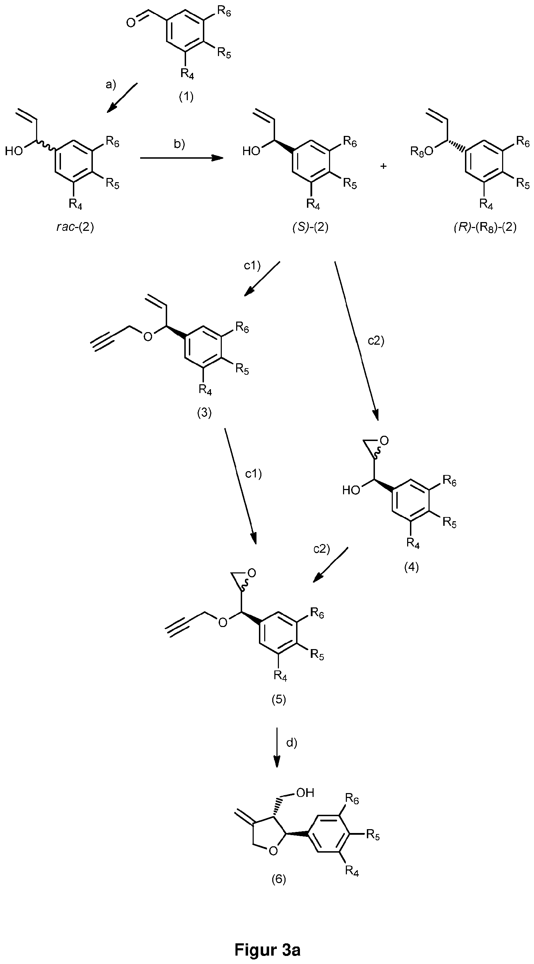

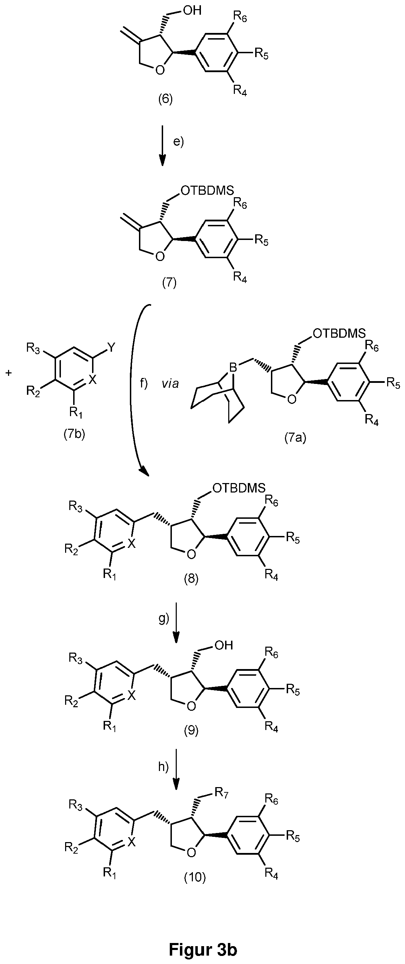

Ein die oben beschriebenen allgemeinen Reaktionsschritte a) bis h) veranschaulichendes Reaktionsschema ist in den Fig. 3a und 3b dargestellt.

### Synthesebeispiele 1 bis 15

Nachstehend wird zunächst die Synthese von Zwischenprodukten der Formeln (2) bis (7) beschrieben. Die Kennzeichnung der Verbindungen erfolgt durch Angabe der jeweiligen Formelziffer 1 bis 7 (ohne Klammern), jeweils gefolgt von einem unterschiedlichen Kleinbuchstaben "a" bis "e" für jeweils eine bestimmte Kombination der Reste R₄ bis R₆ in Formel (II). Die Buchstaben stehen dabei für die folgenden Substitutionsmuster:

| | |
|---|---|
| "a" | R₄ = R₅ = OCH₃, R₆ = H |
| "b" | R₄ = R₅ = R₆ = OCH₃ |
| "c" | R₄ = R₆ = H, R₅ = OCH₃ |
| "d" | R₄ = R₅ = R₆ = H |
| "e" | R₄ = R₆ = H, R₅ = F |

Für manche Verbindungen geht der Formelziffer noch eine Angabe zur Stereochemie voraus, d.h. entweder "(*R*)-" oder "(*S*)-" für das jeweilige Enantiomer oder "*rac*-" für ein racemisches Gemisch.

Anschließend folgt der chemische Name der jeweiligen Verbindung.

### Synthesebeispiel 1

Beispielhaft dargestellt ist die Synthese von **6a** über die Zwischenprodukte *rac-***2a***,* (*S*)-**2a, 3a** und **5a,** ausgehend von **1a:**

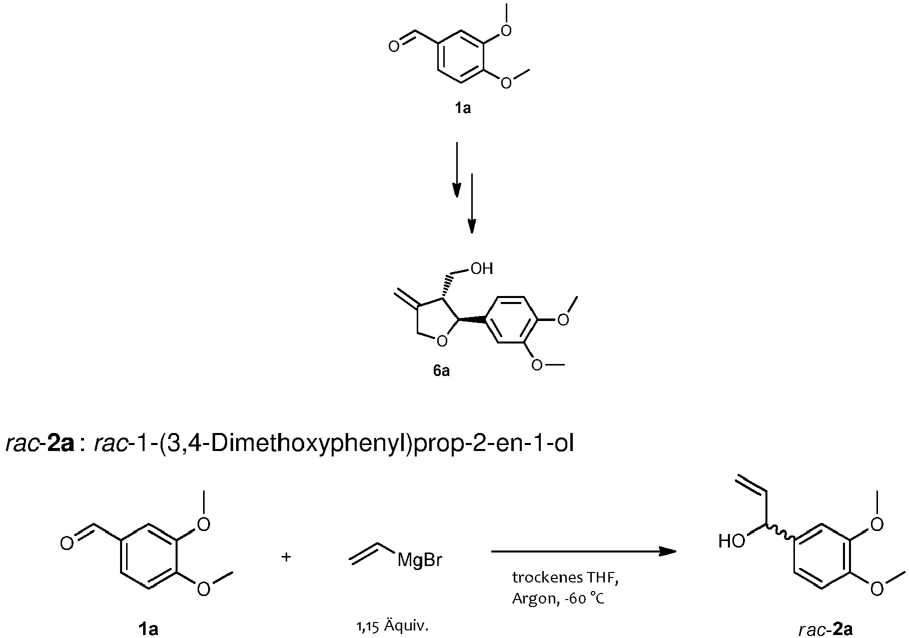

Eine gerührte Lösung von 3,4-Dimethoxybenzaldehyd **1a** (73,1 g, 440,0 mmol, 1,00 Äquiv.) in trockenem THF (600 ml) unter Argon wurde auf -60 °C gekühlt, hierzu wurde Vinylmagnesiumbromid-Lösung (1 M in THF, 506 ml, 506,0 mmol, 1,15 Äquiv.) über einen Tropftrichter binnen 1,8 h zugesetzt, während die Temperatur innerhalb von ±1 °C gehalten wurde. Dann wurde das Gemisch innerhalb von 2 h auf -10 °C erwärmen gelassen, bevor eine gesättigte wässrige NH₄Cl-Lösung (100 ml) binnen 5 min zugetropft wurde, während für zusätzliche Kühlung gesorgt wurde, um zu verhindern, dass die Temperatur während der exothermen Hydrolyse über +10 °C anstieg. Um die Magnesiumsalze zu lösen, wurde Wasser (450 ml) zugesetzt und das Produkt mit Et₂O (1× 500 ml, 5x 250 ml) extrahiert. Die vereinigten organischen Phasen wurden mit gesättigter wässriger NaHCOs-Lösung (1× 150 ml) und gesättigter Kochsalzlösung (1x 100 ml) versetzt, gefolgt von Trocknung mit Na₂SO₄. Die Lösung wurde durch einen Pfropfen aus Kieselgel (15 g, mit Et₂O vorkonditioniert) filtriert, und die Lösungsmittel wurden im Vakuum entfernt, um *rac-***2a** als hellgelbes Öl (85,4 g, 99 %) zur direkten Verwendung in der nächsten Stufe zu erhalten.

### Synthesebeispiel 2

### (S)-2a: (S)-1-(3,4-Dimethoxyphenyl)prop-2-en-1-ol

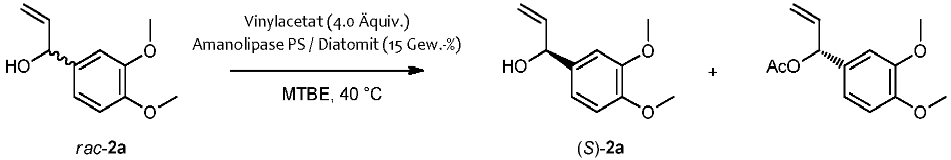

Zu einer mechanisch gerührten Lösung von Alkohol *rac-***2a** (85,4 g, 439,7 mmol, 1,00 Äquiv.) und Vinylacetat (151,5 g, 162 ml, 1,76 mol, 4,00 Äquiv.) in MTBE (2,4 I) bei 40 °C wurde Amanolipase PS (immobilisiert auf Diatomit, 12,82 g, 15 Gew.-%) zugesetzt. Die resultierende Suspension wurde bei dieser Temperatur 44,5 h lang gerührt, bevor das Gemisch durch Celite 545 filtriert und das Lösungsmittel im Vakuum entfernt wurde. Zur Flash-Säulenchromatographie wurde das Rohmaterial wie folgt in Chargen geteilt:
1.) 10,4 g Rohprodukt, Kieselgel (9 g Vorsäule, 90 g Trennsäule), 50 ml/min, EtOAc in PE: 15 % für 30 min, dann 15 auf 40 % binnen 80 min.
2.) 15,0 g Rohprodukt, Kieselgel (9 g Vorsäule, 90 g Trennsäule), 50 ml/min, EtOAc in PE: 15 % für 55 min, dann 15 auf 40 % binnen 40 min.
3.) 20,5 g roh, Kieselgel (40 g & 90 g Trennsäulen), 50 ml/min, EtOAc in PE: 15 % für 40 min, dann 15 auf 25 % binnen 5 min, dann 25 bis 55 % binnen 40 min.
4.) 53,0 g roh, Kieselgel (2 × 90 g Trennsäulen), 50 ml/min, EtOAc in PE: 11 % für 35 min, dann 15 % für 35 min, dann 15 auf 25 % binnen 5 min, 25 auf 65 % binnen 40 min.

Dies ergab (*S*)-**2a** als hellgelbes Öl, das im Stehen zu einem farblosen Pulver (34,4 g, 40 %) kristallisierte.

### Synthesebeispiel 3

### 3a: (S)-1,2-Dimethoxy-4-(1-(prop-2-in-1-yloxy)allyl)benzol

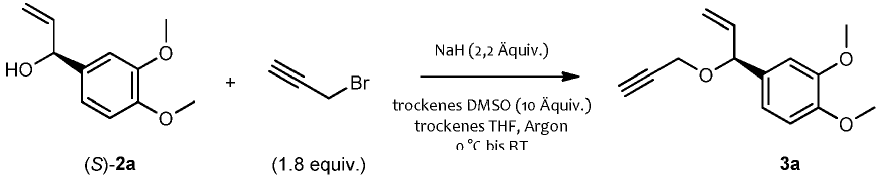

Zu NaH (etwa 60%ige Dispersion in Mineralöl, 15,55 g, 388,7 mmol, 2,20 Äquiv.) in trockenem THF (300 ml) unter Argon wurde trockenes DMSO (125 ml, 1,76 mol, 10,00 Äquiv.) zugesetzt, und die resultierende Suspension wurde unter Verwendung eines Eisbads gekühlt, dann wurde eine Lösung von (*S*)-**2a** (34,32 g, 176,7 mmol, 1,00 Äquiv.) in trockenem THF (300 ml) über einen Tropftrichter innerhalb von 30 min zugesetzt. Danach wurde das Rühren bei dieser Temperatur 15 min lang fortgesetzt, bevor eine Lösung von Propargylbromid (80 % in Toluol, 35,4 ml, 318,1 mmol, 1,80 Äquiv.) innerhalb von 30 min zugesetzt wurde. Um die so entstandene Aufschlämmung aufzulockern, wurde mehr trockenes THF (300 ml) zugesetzt, dann das Eisbad entfernt und die Reaktion 13 h lang gerührt. Das Gemisch, weiterhin unter Argon, wurde dann neuerlich in einem Eisbad gekühlt und durch Zutropfen wässriger HCl-Lösung (1 M, 100 ml) binnen 10 min hydrolysiert. Der Großteil des THF wurde dann im Vakuum (40 °C) entfernt, gefolgt vom Zusatz von Wasser (400 ml), und das Rohprodukt mit Et₂O (4x 400ml) extrahiert, die vereinigten organischen Phasen wurden mit gesättigter Kochsalzlösung (1× 400 ml) versetzt, mit Na₂SO₄ getrocknet, filtriert, und das Lösungsmittel wurde im Vakuum entfernt, um rohes **3a** (47,23 g) zur direkten Verwendung in der nächsten Stufe zu erhalten.

### Synthesebeispiel 4

### 5a: 2-((R)-(3,4-Dimethoxyphenyl)(prop-2-in-1-yloxy)methyl)oxiran

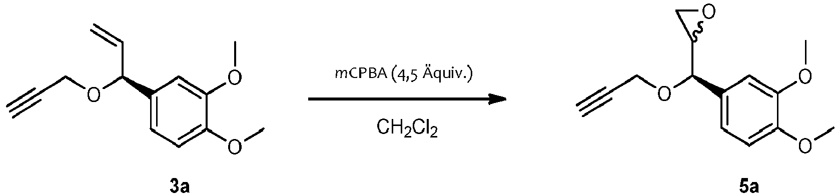

Eine gerührte Lösung von rohem **3a** (47,22 g) in CH₂Cl₂ (500 ml) wurde in einem Eisbad gekühlt, und *m*CPBA (etwa 77%ig, 178,2 g, 795,2 mmol, 4,5 Äquiv.) wurde in kleinen Portionen binnen 30 min zugesetzt. Die Reaktion wurde auf Raumtemperatur erwärmen gelassen, während das Rühren weitere 17 h lang fortgesetzt wurde. Dann wurde ausreichend wässrige Na₂SO₃-Lösung zugesetzt, um restliche Peroxysäure zu zerstören, was Kühlung unter Verwendung eines Eisbads erforderte. Darauf folgte eine wässrige Lösung von K₃PO₄ (185 g) in Wasser (750 ml), um den pH auf 8 zu bringen. Das Rohprodukt wurde mit Et₂O (1 × 750 ml, 5 × 250 ml) extrahiert, die vereinigten organischen Phasen wurden mit gesättigter Kochsalzlösung (250 ml) versetzt, mit Na₂SO₄ getrocknet, und das Lösungsmittel aus dem Filtrat im Vakuum entfernt, um rohes **5a** (50,09 g) zur direkten Verwendung in der nächsten Stufe zu erhalten.

### Synthesebeispiel 5

### 6a: ((2S,3R)-2-(3,4-Dimethoxyphenyl)-4-methylentetrahydrofuran-3-yl)methanol

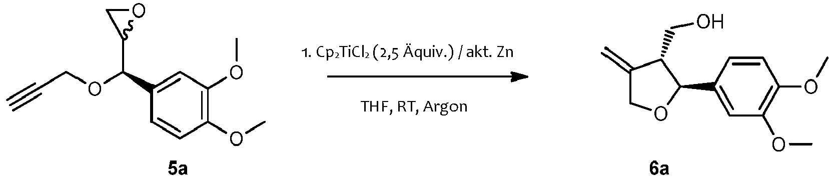

Zu trockenem und sauerstofffreiem THF (2,5 L) wurde unter Argon Bis(cyclopentadienyl)titan(IV)-dichlorid (108,6 g, 436,1 mmol, 2,5 Äquiv.) und aktivierter Zinkstaub (79,8 g, 1,22 mol, 7,0 Äquiv.) zugesetzt, und die resultierende Suspension wurde bei Raumtemperatur 1 h lang heftig gerührt. Dann wurde der restliche Zink 5 min lang absetzen gelassen, woraufhin die grüne Lösung über eine Kanüle in eine schnell gerührte Lösung des rohen Epoxids **5a** (49,42 g) in trockenem und sauerstofffreiem THF (1,2 I) bei Raumtemperatur innerhalb von 3 h übertragen wurde. Danach wurde das Rühren weitere 1,25 h lang fortgesetzt, dann wurde vorsichtig verdünnte H₂SO₄ (10 %, 1 I) zugesetzt und der Großteil des THF im Vakuum entfernt (bis 150 mbar bei 40 °C). Das Rohprodukt wurde dann mit Et₂O extrahiert, die vereinigten organischen Phasen mit gesättigter wässriger NaHCOs-Lösung (500 ml), gesättigter Kochsalzlösung (250 ml) extrahiert, mit Na₂SO₄ getrocknet, filtriert und das Lösungsmittel aus dem Filtrat im Vakuum entfernt.

Flash-Säulenchromatographie wurde mit der gesamten Charge der Reihe nach wie folgt durchgeführt:
1.) Kieselgel (2 × 90 g Trennsäulen), 40 ml/min, EtOAc in PE: 25 auf 50 % binnen 45 min, dann 50 auf 100 % binnen 180 min.
2.) Kieselgel (1 × 90 g Trennsäule), 40 ml/min, EtOAc in PE: 15 auf 50 % binnen 45 min, dann 50 auf 100 % binnen 180 min.

Das ergab **6a** als braunes Öl (8,62 g, 20 % für 3 Stufen).

**¹H-NMR (200 MHz, CDCl₃):** δ 1,63 (bs, 1H), 2,71-2,86 (m, 1H), 3,63-4,00 (m, 8H), 4,42 (dq, *J* = 13,4, 2,2 Hz, 1H), 4,62 (d, *J* = 13,4 Hz, 1H), 4,79 (d, *J* = 7,5 Hz, 1H), 5,07 (q, *J* = 2,3 Hz, 1H), 5,12 (q, *J* = 2,2 Hz, 1H), 6,79-6,99 (m, 3H).

**¹³C-NMR (50 MHz, CDCl₃):** δ 54,0, 56,0, 56,0, 62,0, 71,5, 83,5, 105,1, 109,4, 111,0, 119,0, 133,6, 148,9, 149,0, 149,3.

### Synthesebeispiele 6 bis 8

Die Verbindungen **6c, 6d** und **6e** wurden analog zu **6a** über die entsprechenden Zwischenprodukte *rac-***2***,* (*S*)-**2, 3** und **5**, ausgehend von **1c,** *rac-***2d** bzw. **1e** erhalten:

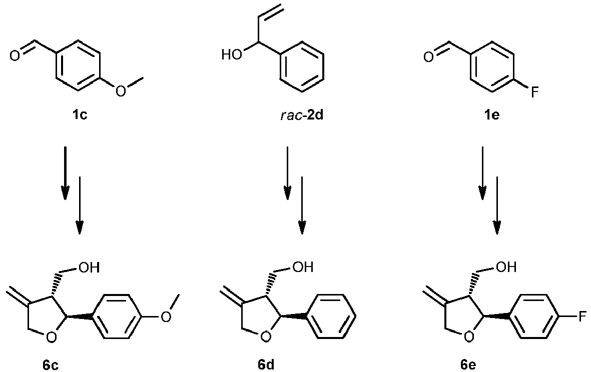

### Synthesebeispiele 9 und 10

Verbindung **6b** wurde über die Zwischenprodukte *rac-***2b***,* (*S*)-**2b**, **4b** und **5b,** ausgehend von **1b** erhalten. Die Synthese von *rac-***2b***,* (*S*)-**2b** und **6b** erfolgte analog zu jener von *rac-***2a***,* (*S*)-**2a** bzw. **6a.** Die Synthese von **4b** und **5b** ist nachstehend angeführt.

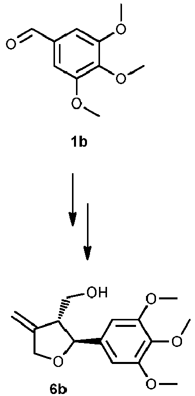

### Synthesebeispiel 9

### 4b: (1R)-Oxiran-2-yl(3,4,5-trimethoxyphenyl)methanol

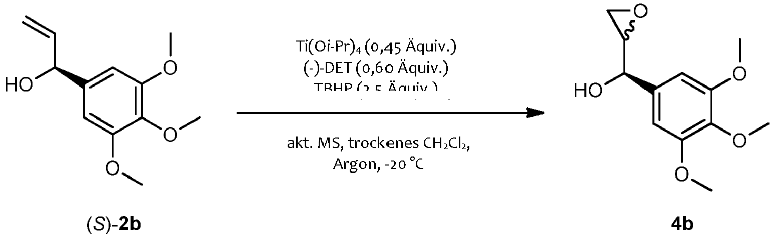

Trockenes CH₂Cl₂ (150 ml), (-)-DET (2,789 g, 12,53 mmol, 0,6 Äquiv.) und (*S*)-**2b** (5,085 g, 22,67 mmol, 1,0 Äquiv.) wurden (zusätzlich) über aktivierten Molekülsieben über Nacht in Argonatmosphäre getrocknet (-)-DET wurde in getrocknetem CH₂Cl₂ (1,0 ml) gelöst und über einen Kryostat auf -20 °C gekühlt. Ti(O*i*-Pr)₄ (3,00 ml, 10,145 mmol, 0,45 Äquiv.) in trockenem CH₂Cl₂ (70 ml, 0,14 M) wurde zugesetzt, und das Reaktionsgemisch wurde 15 min lang gerührt. Dann wurde langsam TBHP (5,5 M in Decan, 10,3 ml, 56,68 mmol, 2,5 Äquiv.) zugesetzt. Nach 30 min wurde eine Lösung von (*S*)-**2b** in CH₂Cl₂ zugesetzt, und das resultierende Gemisch wurde bei -20 °C 70 h lang gerührt. Danach wurde eine Lösung von Na₂SO₃ (20 g in 100 ml Wasser) ebenso zugesetzt wie 1000 ml CH₂Cl₂ und 500 ml Wasser. Die wässrige Phase wurde mit CH₂Cl₂ (4 × 500 ml) extrahiert, und die vereinigten organischen Phasen wurden über Na₂SO₄ getrocknet und filtriert. Das Lösungsmittel wurde im Vakuum entfernt, und Flash-Säulenchromatographie wurde durchgeführt (Rohprodukt adsorbiert an BULK Isolute^{®} HM-N, 90 g Kieselgel, Durchflussgeschwindigkeit 50 ml/min, isokratisch 8 % EtOAc in PE 15 min lang, dann Gradient 8 auf 50 % binnen 25 min, dann 50 auf 100 % binnen 10 min, dann isokratisch 100 % 10 min lang). Das ergab **4b** als orangefarbenes Öl (4,431 mg, 81 %).

### Synthesebeispiel 10

### 5b: 2-((R)-(Prop-2-in-1-yloxy)(3,4,5-trimethoxyphenyl)methyl)oxiran

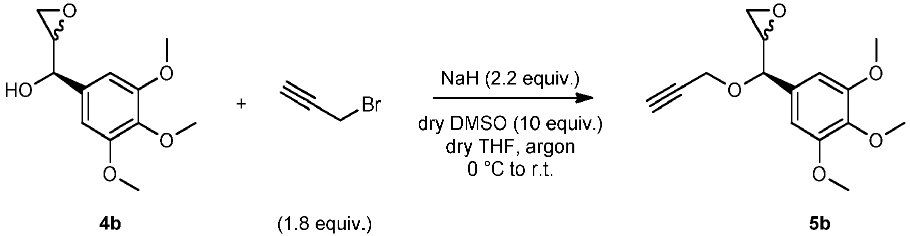

Zu einer Mineralöldispersion von NaH (etwa 60%ig, 1,62 g, 40,57 mmol, 2,2 Äquiv.) wurde unter Argon trockenes THF zugesetzt (z.B. um eine 1,0 M Suspension in Bezug auf NaH zu erhalten) und in einem Eisbad auf 0 °C gekühlt, worauf Zusatz von trockenem DMSO (13,1 ml, 184 mmol, 10 Äquiv.) mittels Spritze folgte. Darauf folgte Zutropfen von **5b** (4,431 g, 18,44 mmol, 1,0 Äquiv.) als Lösung in trockenem THF (0,4 M), anschließend 15 min langes Rühren der Reaktion bei 0 °C, und schließlich Propargylbromid (80%ige Lösung in Toluol, 3,7 ml, 33,2 mmol, 1,8 Äquiv.) als Lösung in trockenem THF, beide mittels Spritze. Das Eisbad wurde entfernt, und das Gemisch wurde 48 h lang bei Raumtemperatur gerührt. Danach wurde die Reaktion wieder auf 0 °C gekühlt und durch Zutropfen von wässriger HCl (1 M, 1,0 Äquiv.) gequencht. Der Großteil des THF wurde abgedampft, dann wurde Wasser zugesetzt. Das Gemisch wurde mit Et₂O (4x) extrahiert, die vereinigten organischen Phasen wurden mit gesättigter Kochsalzlösung versetzt, mit Na₂SO₄ getrocknet, filtriert und die Lösungsmittel abgedampft. Dann wurde Flash-Säulenchromatographie durchgeführt (90 g Kieselgel, Durchflussgeschwindigkeit 50 ml/min, Gradient, isokratisch 3 % EtOAc in PE 10 min lang, dann 3 auf 30 % binnen 20 min, dann 30 auf 100 % binnen 20 min, dann isokratisch 100 % 10 min lang). Das ergab **5b** als gelbliches Öl (4,211 g, 82 %).

### Synthesebeispiel 11

Beispielhaft dargestellt ist die Synthese von **7a** aus **6a:**
**7a:** *tert-*Butyl(((2S,3R)-2-(3,4-dimethoxyphenyl)-4-methylentetrahydrofuran-3-yl)methoxy)dimethylsilan

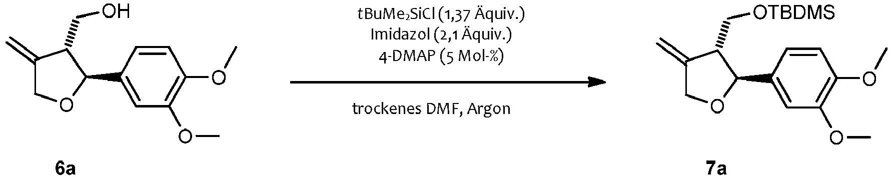

Zu einer gerührten Lösung von **6a** (1,723 g, 6,885 mmol, 1,00 Äquiv.), Imidazol (984 mg, 14,459 mmol, 2,10 Äquiv.) und 4-Dimethylaminopyridin (42 mg, 0,344 mmol, 0,05 Äquiv.) in trockenem DMF (40 ml) unter Argon wurde *tert*-Butylchlordimethylsilan-Lösung (3 M in THF, 3,14 ml, 9,42 mmol, 1,37 Äquiv.) zugetropft, und das Gemisch wurde bei Raumtemperatur 12,5 h lang gerührt. Dann wurde Et₂O (100 ml) zugesetzt, gefolgt von gesättigter wässriger NH₄Cl-Lösung (40 ml). Die Phasen wurden getrennt, die wässrige Phase mit Et₂O (3 × 50 ml) extrahiert, die vereinigten organischen Phasen wurden mit einer gesättigten wässrigen Lösung von NaHCOs (25 ml), gesättigter Kochsalzlösung (25 ml) versetzt, mit Na₂SO₄ getrocknet, filtriert und die Lösungsmittel im Vakuum entfernt, um rohes **7a** (2,63 g) zur direkten Verwendung zur Hydroborierung / Suzuki-Kupplung in der nächsten Stufe zu erhalten.

### Synthesebeispiele 12 bis 15

Die Verbindungen **7b, 7c, 7d** und **7e** wurden analog zu **7a** aus **6b, 6c, 6d** bzw. **6e** erhalten und direkt zur Hydroborierung / Suzuki-Kupplung in der nächsten Stufe eingesetzt.

### Beispiele 1 bis 6 und Vergleichsbeispiele 1 bis 8

Die in den nachfolgenden Abschnitten synthetisierten Verbindungen sind jene von biologischem Interesse (d.h. entsprechend der Formel (II)), und die meisten von ihnen wurden (bereits) einem Screening auf biologischem Aktivität unterzogen, woraufhin sie eine 4-stellige "Bio-ID" zugewiesen bekamen (Leoligin z.B. hat die ID "2418"), die nach dem chemischen Namen der Verbindung angeführt ist. Ist keine "Bio-ID" angegeben, wurde die betreffende Verbindung entweder noch nicht getestet oder zeigte nicht die gewünschte Selektivität der Zellhemmung und stellt somit ein Vergleichsbeispiel in Bezug auf die Aktivität dar. Weiters wird auch jede Verbindung, die in den (später näher erläuterten) Aktivitätstests ein Aktivitätsverhältnis von unter 1,50 aufwies, als Vergleichsbeispiel bezeichnet, wenngleich - wie zuvor erwähnt - zukünftige Tests möglicherweise doch ihre Eignung für die Zwecke der Offenbarung belegen könnten.

Die Anbindung des die Substituenten R₁ bis R₃ tragenden Arylrests erfolgte dabei durchwegs durch Suzuki-Kupplung nach anfänglicher Hydroborierung. Die darauf folgende Abspaltung der OH-Schutzgruppe wurde in der Regel als Eintopfreaktion, d.h. ohne Isolierung des geschützten Zwischenprodukts der Formel (8), durchgeführt.

Die Bezeichnung der Verbindungen erfolgt durch Angabe der Formelziffer 9, gefolgt von einer weiteren, prinzipiell fortlaufend ansteigenden Zahl. Fehlende Verbindungsnummern in dieser Reihe zeigen an, dass die jeweiligen Verbindungen (bisher) nicht die gewünschte Selektivität der Zellhemmung gezeigt haben (oder noch nicht getestet wurden) und weder als Ausgangsmaterial für Verbindungen mit der gewünschten Aktivität benötigt noch mittels einer Referenzsynthese hergestellt wurden.

Die Versuchsbeschreibung ist für gewöhnlich in "Herstellung" und "Aufarbeitung und Reinigung" unterteilt und bezieht sich in vielen Fällen auf ein Beispiel weiter oben in der Liste, falls Herstellung und/oder Aufarbeitung und Reinigung identisch oder analog waren. Für gewöhnlich sind ¹H-NMR, HR-MS und spezifische Drehung oder alternativ dazu ¹H-NMR, ¹³C-NMR und spezifische Drehung bei Verbindungen angegeben, für die biologische Ergebnisse verfügbar sind. Ist das Herstellungsverfahren in einem bestimmten Beispiel explizit angeführt (d.h. nicht auf ein identisches oder analoges Verfahren referenziert), sind ¹H-NMR, ¹³C-NMR, HR-MS und spezifische Drehung angeführt.

In dieser ersten Gruppe von Beispielen und Vergleichsbeispielen wurde ausschließlich das Zwischenprodukt 7a mit R₄ = R₅ = OCH₃ und R₆ = H derivatisiert.

### Vergleichsbeispiel 1

### 9-1: ((2S,3R,4R)-4-(3,4-Dimethoxybenzyl)-2-(3,4-dimethoxyphenyl)tetrahydrofuran-3-yl)methanol (Dimethyllariciresinol)

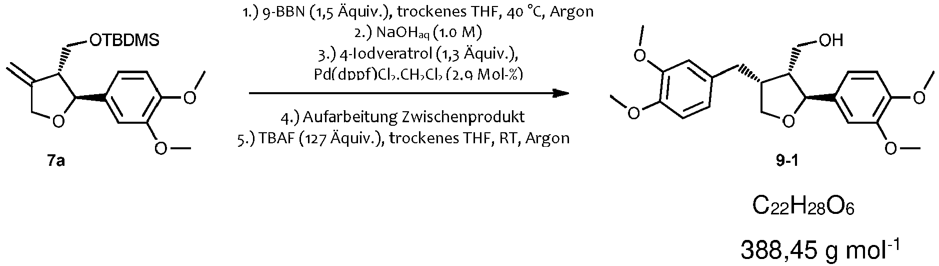

Herstellung: Ein Reaktionsgefäß wurde mit einem Rührstäbchen und rohem Ausgangsmaterial **7a** (2,51 g, 6,88 mmol, 1,0 Äquiv.) befüllt und dann evakuiert und unter Anwendung eines Standard-Schlenk-Verfahrens (3x) mit Argon wiederbelüftet. Dann wurde eine Lösung von 9-BBN (0,5 M in THF, 20,7 ml, 10,3 mmol, 1,5 Äquiv.) mittels Spritze zugesetzt, die Reaktion 16,5 h lang bei 40 °C gerührt und dann auf Raumtemperatur abkühlen gelassen. Danach wurde eine entgaste wässrige Lösung von NaOH (2 M, 20 ml) vorsichtig zugesetzt, und das Rühren wurde weitere 15 min lang fortgesetzt. Als nächstes wurden 4-lodveratrol (2,36 g, 8,95 mmol, 1,30 Äquiv.) und Pd(dppf)Cl₂.CH₂Cl₂ (161 mg, 0,198 mmol, 2,9 Mol-%) zugesetzt, und das resultierende zweiphasige Gemisch wurde bei Raumtemperatur 25 h lang kräftig gerührt. Dann wurden Et₂O (200 ml) und gesättigte Kochsalzlösung (50 ml) zugesetzt, die Phasen wurden getrennt, die wässrige Phase mit Et₂O (4 × 50 ml) extrahiert, die vereinigten organischen Phasen mit Na₂SO₄ getrocknet und filtriert. Unter Verwendung eines neuen Reaktionsgefäßes wurde das Lösungsmittel abgedampft, zu dem Rückstand wurde ein Rührstäbchen gegeben, dann wurde das Gefäß evakuiert und mit Argon wiederbelüftet. Zur Schutzgruppenentfernung wurde eine Lösung von TBAF (1,0 M in THF, 8,25 ml, 8,25 mmol, 1,2 Äquiv.) mittels Spritze zugesetzt und das Gemisch schließlich 18 h lang bei Raumtemperatur gerührt.

Aufarbeitung und Reinigung: Et₂O (200 ml) und gesättigte Kochsalzlösung (50 ml) wurden zugesetzt, die Phasen wurden getrennt und die wässrige Phase mit Et₂O (4x 50 ml) und EtOAc (2x 50 ml) extrahiert. Die vereinigten organischen Phasen wurden mit Na₂SO₄ getrocknet, filtriert und die Lösungsmittel abgedampft. Die Zielverbindung wurde mittels Flash-Säulenchromatographie in zwei aufeinanderfolgenden Durchgängen (erster Durchgang: 90 g Kieselgel mit 9 g vor Säule, Durchflussgeschwindigkeit 40 ml/min, isokratisch bei 30 % EtOAc in PE 3 min lang, dann Gradient 30 auf 100 % binnen 60 min; zweiter Durchlauf: 90 g Kieselgel, Durchflussgeschwindigkeit 40 ml/min, Gradient 45 auf 85 % EtOAc in PE binnen 60 min) gereinigt, um ein leicht gefärbtes Öl zu erhalten (1,04 g, 39 %).

**¹H-NMR (200 MHz, CDCl₃):** δ 1,52 (bs, 1H), 2,42 (quint, J= 6,9 Hz, 1H), 2,56 (dd, *J* = 12,7, 10,4 Hz, 1H), 2,66-2,85 (m, 1H), 2,94 (dd, *J* = 12,8, 4,7 Hz, 1H), 3,73-3,98 (m, 2H), 3,76 (dd, *J* = 8,5, 5,9 Hz, 1H), 3,87 (s, 9H), 3,88 (s, 3H), 4,07 (dd, *J* = 8,5, 6,4 Hz, 1H), 4,81 (d, *J* = 6,5, 1H), 6,70-6,81 (m, 3H), 6,81-6,91 (m, 3H).

**¹³C-NMR (50 MHz, CDCl₃):** δ 33,4, 42,5, 52,7, 56,0, 56,1, 61,1, 73,1, 82,9, 109,0, 111,1, 111,4, 112,0, 118,2, 120,6, 133,1, 135,5, 147,6, 148,5, 149,1, 149,2.

### Vergleichsbeispiel 2

### 9-2: ((2S,3R,4R)-4-(3,4-Dimethoxybenzyl)-2-(3,4,5-trimethoxyphenyl)tetrahydrofuran-3-yl)methanol

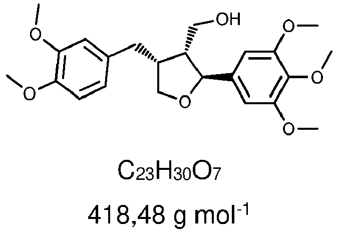

Herstellung analog zu jener von **9-1** unter Verwendung von rohem Ausgangsmaterial **7b** (1,48 g, 3,62 mmol, 1,0 Äquiv.) und 22,5-, 24- bzw. 20-stündiges Rühren in den Stufen der Hydroborierung, der Bindung bzw. der Schutzgruppenentfernung.

Aufarbeitung und Reinigung: Et₂O (100 ml) und gesättigte Kochsalzlösung (25 ml) wurden zugesetzt, und die wässrige Phase wurde mit Et₂O (4 × 30 ml) und EtOAc (2 × 35 ml) extrahiert. Die vereinigten organischen Phasen wurden mit Na₂SO₄ getrocknet, filtriert und die Lösungsmittel abgedampft. Die Zielverbindung wurde mittels Flash-Säulenchromatographie 90 g Kieselgel, Durchflussgeschwindigkeit 40 ml/ min, isokratisch bei 30 % EtOAc in PE 5 min lang, dann Gradient 30 auf 100 % binnen 45 min) gereinigt, um ein gelbes Öl zu erhalten (631 mg, 42 %).
**[α]_{D}²⁰:** +7,1 (c 2,14, MeOH)

**HR-MS (ESI)** ber. für M+Na⁺: 441,1884, gef.: 441,1903.

**¹H-NMR (200 MHz, CDCl₃):** δ 1,68 (bs, 1H), 2,32-2,82 (m, 3H), 2,92 (dd, *J* = 12,8, 4,6 Hz, 1H), 3,83 (s, 3H), 3,85 (s, 15H), 4,07 (dd, *J* = 8,4, 6,5 Hz, 1H), 4,84 (d, *J* = 6,1 Hz, 1H), 6,56 (s, 2H), 6,67-6,83 (m, 3H).

**¹³C-NMR (50 MHz, CDCl₃):** δ 33,1, 42,2, 52,4, 55,8, 56,1, 60,8, 60,9, 73,0, 82,9, 102,5, 111,2, 111,8, 120,4, 132,9, 137,1, 138,7, 147,4, 148,9, 153,2.

### Vergleichsbeispiel 3

### 9-3: ((2S,3R,4R)-4-Benzyl-2-(3,4-dimethoxyphenyl)tetrahydrofuran-3-yl)methanol

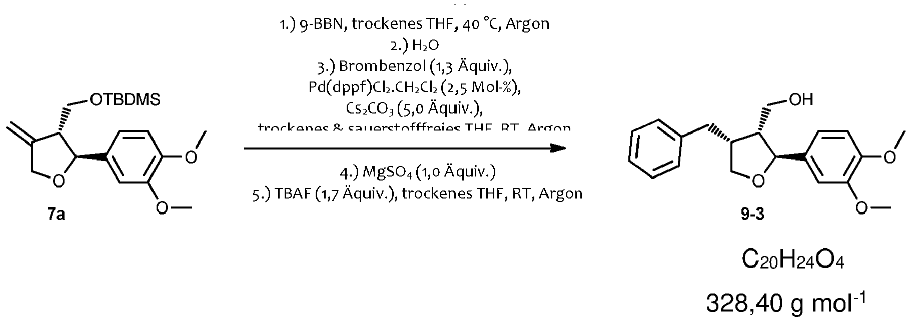

Herstellung: Ein Reaktionsgefäß wurde mit einem Rührstäbchen und rohem Ausgangsmaterial **7a** (715,9 mg, 1,964 mmol) befüllt und dann evakuiert und mit Argon unter Anwendung eines Standard-Schlenk-Verfahrens (3x) wiederbelüftet. Dann wurde eine Lösung von 9-BBN (0,5 M in THF, 5,89 ml, 2,95 mmol) mittels Spritze zugesetzt, die Reaktion wurde 21 h lang bei 40 °C gerührt und dann auf Raumtemperatur abkühlen gelassen. Anschließend wurde Wasser (35 µl, 2,0 mmol) zugesetzt, und das Rühren wurde 2 h lang fortgesetzt, um überschüssiges 9-BBN zu zersetzen.

Dann wurde dieses Gemisch durch Einspülen von Argon in die Lösung durch eine Nadel gereinigt, und anschließend wurde trockenes und sauerstofffreies THF zugesetzt, um ein Gesamtvolumen von 10,0 ml zu erhalten, d.h. eine 0,196 M Lösung des borylierten Zwischenprodukts, was die Verwendung von Aliquoten zur nachfolgenden Kupplung ermöglichte.

Ein Aliquot (0,97 ml, 0,19 mmol, 1,0 Äquiv.) dieser Lösung wurde dann mittels Spritze in ein eigenes Gefäß übertragen, das mit einem Rührstäbchen, Brombenzol (38,8 mg, 0,247 mmol, 1,3 Äquiv.), Pd(dppf)Cl₂.CH₂Cl₂ (3,9 mg, 4,8 µmοl, 2,5 Mol-%) und Cs₂CO₃ (310 mg, 0,950 mmol, 5,0 Äquiv.) unter Argon beladen war und dann 36 h lang bei Raumtemperatur gerührt. Danach wurde MgSO₄ (23 mg, 0,19 mmol, 1,0 Äquiv.) zugesetzt, und das Rühren wurde 1,5 h lang fortgesetzt, um Restwasser zu entfernen. Zur Schutzgruppenentfernung wurde eine Lösung von TBAF (1,0 M in THF, 0,32 ml, 0,32 mmol, 1,7 Äquiv.) mittels Spritze zugesetzt und das Gemisch schließlich 32 h lang bei Raumtemperatur gerührt.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 20 auf 70 % EtOAc in PE binnen 30 min) gereinigt, um ein hellbraunes Öl zu erhalten (39,3 mg, 63 %).
**[α]_{D}²³:** +18,3 (c 3,80, MeOH)

**HR-MS (ESI)** ber. für M+Na⁺: 351,1567, gef.: 351,1577.

**¹H-NMR (200 MHz, CDCl₃):** δ 1,70 (bs, 1H), 2,40 (quint, *J* = 6,8 Hz, 1H), 2,61 (dd, *J* = 12,5, 10,2 Hz, 1H), 2,67-2,87 (m, 1H), 2,96 (dd, *J* = 12,6, 4,6 Hz, 1H), 3,69-3,98 (m, 3H), 3,85 (s, 3H), 3,87 (s, 3H), 4,05 (dd, *J* = 8,5, 6,4 Hz, 1H), 4,83 (d, *J* = 6,3 Hz, 1H), 6,77-6,94 (m, 3H), 7,14-7,35 (m, 5H).

**¹³C-NMR (50 MHz, CDCl₃):** 33,7, 42,2, 52,5, 56,0, 56,0, 61,0, 73,0, 82,9, 109,0, 111,1, 118,1, 126,3, 128,7, 128,7, 135,6, 140,5, 148,4, 149,1.

### Beispiel 1

### 9-5: ((2S,3R,4R)-4-(4-(tert Butyl)benzyl)-2-(3,4-dimethoxyphenyl)tetrahydrofuran-3-yl)methanol (2749)

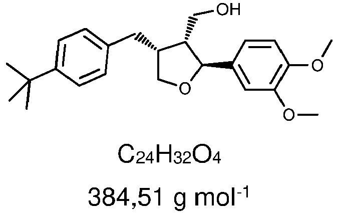

Herstellung identisch mit jener von **9-3,** mit der Ausnahme, dass 1-Brom-4-(tert-butyl)benzol (52,6 mg, 0,247 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 20 auf 70 % EtOAc in PE binnen 30 min) gereinigt, um ein hellbraunes Öl zu erhalten (49,1 mg, 67 %).
**[α]_{D}²⁵:** +12,0 (c 4,91, MeOH)

**HR-MS (ESI)** ber. für M+Na⁺: 407,2193, gef.: 407,2183.

**¹H-NMR (200 MHz, CDCl₃):** δ 1,31 (s, 9H), 1,68 (bs, 1H), 2,40 (quint, *J* = 6,7 Hz, 1H), 2,59 (dd, *J* = 12,5, 9,8 Hz, 1H), 2,67-2,86 (m, 1H), 2,92 (dd, *J* = 12,6, 4,8 Hz, 1H), 3,69-3,98 (m, 3H), 3,86 (s, 3H), 3,87 (s, 3H), 4,08 (dd, *J* = 8,5, 6,4 Hz, 1H), 4,83 (d, *J* = 6,2 Hz, 1H), 6,78-6,94 (m, 3H), 7,12 (d, *J* = 8,2 Hz, 2H), 7,31 (d, *J* = 8,2 Hz, 2H).

### Vergleichsbeispiel 4

### 9-6: Ethyl 4-(((3R,4R,5S)-5-(3,4-dimethoxyphenyl)-4-(hydroxymethyl)tetrahydrofuran-3-yl)methyl)benzoat

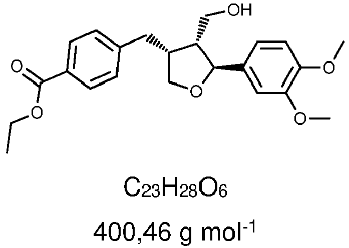

Herstellung identisch mit jener von **9-3,** mit der Ausnahme, dass Ethyl-4-bromben-zoat (56,6 mg, 0,247 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 30 auf 52 % EtOAc in PE binnen 17 min, dann auf 74 % binnen 9 min, dann isokratisch bei 74 %) gereinigt, um ein hellbraunes Öl zu erhalten (47,3 mg, 62 %).
**[α]_{D}²³**: +11,8 (c 4,64, MeOH)

**HR-MS (ESI)** ber. für M+Na⁺: 423,1778, gef.: 423,1795.

**¹H-NMR (200 MHz, CDCl₃):** δ 1,38 (t, *J* = 7,1 Hz, 3H), 1,86 (t, *J* = 4,6 Hz, 1H), 2,42 (quint, *J* = 6,7 Hz, 1H), 2,59-2,88 (m, 2H), 3,04 (dd, *J* = 11,9 Hz, 3,3 Hz, 1H), 3,71 (dd, *J* = 8,6 Hz, 6,0 Hz, 1H), 3,74-3,99 (m, 2H), 3,86 (s, 3H), 3,87 (s, 3H), 4,03 (dd, *J* = 8,6, 6,2 Hz, 1H), 4,36 (q, *J* = 7,1 Hz, 2H), 4,82 (d, *J* = 6,4 Hz, 1H), 6,78-6,94 (m, 3H), 7,27 (d, *J* = 8,2 Hz, 2H), 7,97 (d, *J* = 8,2 Hz, 2H).

### Beispiel 2

### 9-9: ((2S,3R,4R)-2-(3,4-Dimethoxyphenyl)-4-(4-methylbenzyl)tetrahydrofuran-3-yl)-methanol (2754)

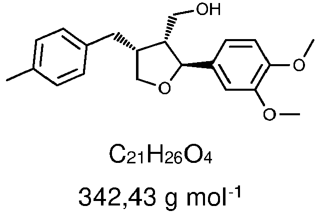

Herstellung identisch mit jener von 9-3, mit der Ausnahme, dass 4-Bromtoluol (42,2 mg, 0,247 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 20 auf 70 % EtOAc in PE binnen 30 min) gereinigt, um ein hellbraunes Öl zu erhalten (43,2 mg, 66 %).
**[α]_{D}²³:** +15,0 (c 4,34, MeOH)

**HR-MS (ESI)** ber. für M+Na⁺: 365,1723, gef.: 365,1727.

**¹H-NMR (200 MHz, CDCl₃):** δ 1,72 (t, *J* = 5,0 Hz, 1H), 2,31 (s, 3H), 2,39 (quint, *J* = 6,7 Hz, 1H), 2,57 (dd, *J* = 12,6, 10,0 Hz, 1H), 2,65-2,85 (m, 1H), 2,91 (dd, *J* = 12,6, 4,8 Hz, 1H), 3,66-3,98 (m, 3H), 3,85 (s, 3H), 3,87 (s, 3H), 4,05 (dd, *J* = 8,5, 6,4 Hz, 1H), 4,82 (d, *J* = 6,3 Hz, 1H), 6,77-6,90 (m, 3H), 7,09 (s, 4H).

### Vergleichsbeispiel 5

### 9-12: ((2S,3R,4R)-2-(3,4-Dimethoxyphenyl)-4-(3-fluorbenzyl)tetrahydrofuran-3-yl)-methanol

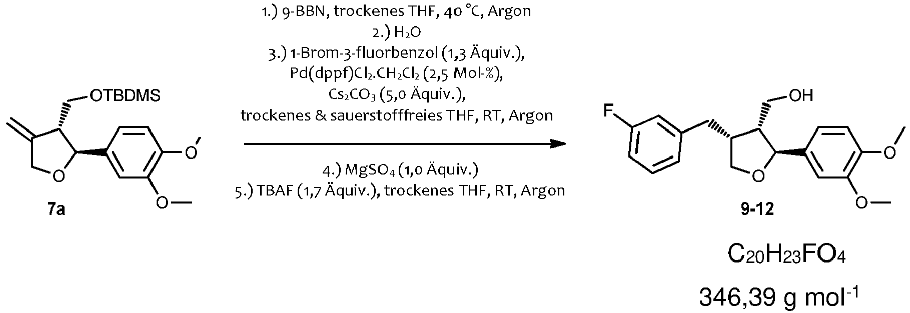

Herstellung: Ein Reaktionsgefäß wurde mit einem Rührstäbchen und rohem Ausgangsmaterial **7a** (843,2 mg, 2,313 mmol) beladen und dann evakuiert und mit Argon unter Anwendung eines Standard-Schlenk-Verfahrens (3x) wiederbelüftet. Dann wurde eine Lösung von 9-BBN (0,5 M in THF, 6,94 ml, 3,47 mmol) mittels Spritze zugesetzt, die Reaktion wurde 35 h lang bei 40 °C gerührt und dann auf Raumtemperatur abkühlen gelassen. Anschließend wurde Wasser (42 µl, 2,3 mmol) zugesetzt, und das Rühren wurde 2 h lang fortgesetzt, um überschüssiges 9-BBN zu zersetzen.

Dann wurde dieses Gemisch durch Einspülen von Argon in die Lösung durch eine Nadel gereinigt, und anschließend wurde trockenes und sauerstofffreies THF zugesetzt, um ein Gesamtvolumen von 13,0 ml zu erhalten, d.h. eine 0,178 M Lösung des borylierten Zwischenprodukts, was die Verwendung von Aliquoten zur nachfolgenden Kupplung ermöglichte.

Ein Aliquot (1,07 ml, 0,19 mmol, 1,0 Äquiv.) dieser Lösung wurde dann unter Argon mittels Spritze in ein eigenes Gefäß übertragen, das mit einem Rührstäbchen, 1-Brom-3-fluorbenzol (43,2 mg, 0,247 mmol, 1,3 Äquiv.), Pd(dppf)Cl₂.CH₂Cl₂ (3,9 mg, 4,8 µmol, 2,5 Mol-%) und Cs₂CO₃ (310 mg, 0,950 mmol, 5,0 Äquiv.) beladen war und dann 50 h lang bei Raumtemperatur gerührt. Danach wurde MgSO₄ (23 mg, 0,19 mmol, 1,0 Äquiv.) zugesetzt, und das Rühren wurde 1,5 h lang fortgesetzt, um Restwasser zu entfernen. Zur Schutzgruppenentfernung wurde eine Lösung von TBAF

(1,0 M in THF, 0,32 ml, 0,32 mmol, 1,7 Äquiv.) mittels Spritze zugesetzt und das Gemisch schließlich 24 h lang bei Raumtemperatur gerührt.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 15 auf 80 % EtOAc in PE binnen 30 min) gereinigt, um ein farbloses Öl zu erhalten (50,9 mg, 77 %).
**[α]_{D}²⁵:** +21,0 (c 2,22, MeOH)

**HR-MS (ESI)** ber. für M+Na⁺: 369,1473, gef.: 369,1476.

**¹H-NMR (200 MHz, CDCl₃):** δ 1,78 (bs, 1H, OH), 2,40 (quint, *J* = 6,7 Hz, 1H), 2,61 (dd, *J* = 12,4, 10,6 Hz, 1H), 2,64-2,85 (m, 1H), 2,97 (dd, *J* = 12,5, 4,1 Hz, 1H), 3,71 (dd, *J* = 8,5, 6,2 Hz, 1H), 3,76-3,90 (m, 2H), 3,86 (s, 3H), 3,87 (s, 3H), 4,05 (dd, *J* = 8,5, 6,3 Hz, 1H), 4,81 (d, *J* = 6,4 Hz, 1H), 6,78-7,00 (m, 6H), 7,31-7,18 (m, 1H).

**¹³C-NMR (50 MHz, CDCl₃):** δ 33,4 (d, *J* = 1,4 Hz), 42,1, 52,4, 56,0, 56,0, 60,9, 72,8, 82,8, 108,9, 111,0, 113,2 (d, *J* = 21,0 Hz), 115,6 (d, *J* = 21,0 Hz), 118,1, 124,4 (d, *J* = 2,8 Hz), 130,1 (d, *J* = 8,4 Hz), 135,4, 143,2 (d, *J* = 7,1 Hz), 148,5, 149,1, 163,0 (d, *J* = 245,8 Hz).

### Vergleichsbeispiel 6

### 9-13: ((2S,3R,4R)-2-(3,4-Dimethoxyphenyl)-4-(pyridin-2-ylmethyl)tetrahydrofuran-3-yl)methanol

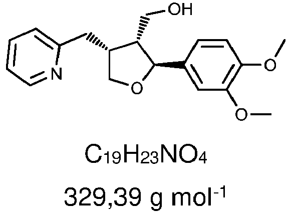

Herstellung identisch mit jener von **9-12,** mit der Ausnahme, dass 2-Brompyridin (39,0 mg, 0,247 mmol, 1,3 Äquiv.) als Arylhalogenid-Kupplungspartner verwendet wurde und das Wiederholen der Schritte 4 und 5 zur Schutzgruppenentfernung als unvollständig erachtet wurde.

Danach wurde Et₂O (5 ml) zu dem Reaktionsinhalt zugesetzt, und das Gemisch wurde mit wässriger HCl (1 M, 2 × 5 ml) extrahiert. Zu den vereinigten wässrigen Phasen wurde dann CH₂Cl₂ (10 ml) zugesetzt, gefolgt von der vorsichtigen Zugabe von festem Na₂CO₃ (1,5 g). Ein weiteres Mal wurde die wässrige Phase mit CH₂Cl₂ (10 ml) extrahiert, die vereinigten Extrakte wurden mit Na₂SO₄ getrocknet und die Lösungsmittel abgedampft. Die Zielverbindung wurde zunächst mittels Flash-Säulenchromatographie (18 g Kieselgel, Durchflussgeschwindigkeit 20 ml/min, 100 % EtOAc) und dann mittels präparativer HPLC (Phenomenex^{®} Luna 10u C18(2) 100 A, 250 × 21,20 mm, Durchflussgeschwindigkeit 20,0 ml/min, isokratisch bei 38 % MeOH in Wasser) gereinigt, um ein farbloses Öl zu erhalten (36,7 mg, 59 %).
**[α]_{D}²⁵:** +22,1 (c 1,91, MeOH)

**HR-MS (ESI)** ber. für M+H⁺: 330,1700, gef.: 330,1698.

**¹H-NMR (200 MHz, CDCl₃):** δ 2,41-2,57 (m, 1H), 2,72-2,89 (m, 2H), 3,24 (dd, *J* = 15,5, 9,9 Hz, 1H), 3,69 (dd, *J* = 11,6, 4,1 Hz, 1H), 3,79 (dd, *J* = 8,4, 6,5 Hz, 1H), 3,86 (s, 3H), 3,87 (s, 3H), 3,91-4,01 (m, 1H), 4,25 (dd, *J* = 8,5, 6,3 Hz, 1H), 4,66 (d, *J* = 6,4 Hz, 1H), 6,77-6,90 (m, 3H), 7,11-7,24 (m, 2H), 7,64 (td, *J* = 7,7, 1,8 Hz, 1H), 8,47 (d, *J* = 4,9 Hz, 1H).

### Beispiel 3

### 9-14: ((2S,3R,4R)-2-(3,4-Dimethoxyphenyl)-4-(naphthalen-1-ylmethyl)tetrahydrofuran-3-yl)methanol (2821)

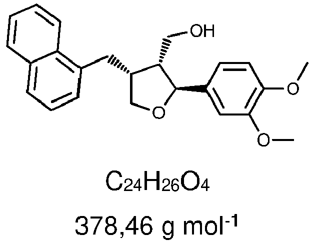

Herstellung identisch mit jener von **9-12,** mit der Ausnahme, dass 1-Bromnaphthalin (51,1 mg, 0,247 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 15 auf 80 % EtOAc in PE binnen 45 min) gereinigt und dann aus iPrOH (11 ml) umkristallisiert. Die heiße Lösung wurde von unlöslichem Material dekantiert, abkühlen gelassen und die Kristallisation dann durch Lagern bei -25 °C vollendet. Entfernung der Mutterlauge und Spülen mit Pentan (etwa 3 ml) ergaben farblose Kristalle (43,7 mg, 61 %).
**[α]_{D}²⁰:** +39,5 (c 0,78, DMSO-d6)

**HR-MS (ESI)** ber. für M+Na⁺: 401,1723, gef.: 401,1741.

**¹H-NMR (200 MHz, CDCl₃):** δ 2,37 (quint, *J* = 6,9 Hz, 1H), 2,64-2,98 (m, 2H), 3,48-3,90 (m, 4H), 3,73 (s, 6H), 4,73 (d, *J* = 7,4 Hz, 1H), 4,89 (t, *J* = 4,4 Hz, 1H), 6,76-6,98 (m, 3H), 7,33-7,60 (m, 4H), 7,79 (d, *J* = 7,3 Hz, 1H), 7,92 (m, 1H), 8,19 (m, 1H).

### Beispiel 4

### 9-17: ((2S,3R,4R)-2-(3,4-Dimethoxyphenyl)-4-(4-(trifluormethyl)benzyl)tetrahydrofuran-3-yl)methanol (3005)

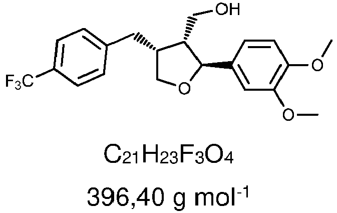

Herstellung identisch mit jener von **9-12,** mit der Ausnahme, dass 1-Brom-4-(trifluormethyl)benzol (55,6 mg, 0,247 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 15 auf 80 % EtOAc in PE binnen 45 min) gereinigt, um ein nahezu farbloses Öl zu erhalten (57,2 mg, 76 %).
[α]_{D}²⁵: +18,7 (c 2,68, MeOH)

**HR-MS (ESI)** ber. für M+Na⁺: 419,1441, gef.: 419,1437.

**¹H-NMR (200 MHz, CDCl₃):** δ 1,79 (bs, 1H), 2,42 (quint, *J*= 6,7 Hz, 1H), 2,60-2,86 (m, 2H), 3,04 (d, *J* = 11,4 Hz, 1H), 3,71 (dd, *J*= 8,6, 5,9 Hz, 1H), 3,76-3,97 (m, 2H), 3,86 (s, 3H), 3,87 (s, 3H), 4,03 (dd, *J*= 8,6, 6,2 Hz, 1H), 4,82 (d, *J* = 6,4 Hz, 1H), 6,78-6,91 (m, 3H), 7,31 (d, *J* = 8,0 Hz, 2H), 7,55 (d, *J* = 8,2 Hz, 2H).

### Vergleichsbeispiel 7

### 9-18: ((2S,3R,4R)-2-(3,4-Dimethoxyphenyl)-4-(3,4,5-trimethoxybenzyl)tetrahydrofuran-3-yl)methanol

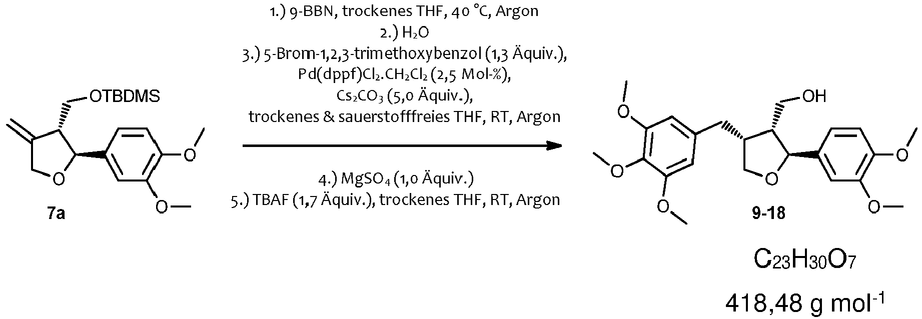

Herstellung: Ein Reaktionsgefäß wurde mit einem Rührstäbchen und rohem Ausgangsmaterial **7a** (658,7 mg, 1,807 mmol) beladen und dann evakuiert und mit Argon unter Anwendung eines Standard-Schlenk-Verfahrens (3x) wiederbelüftet. Dann wurde eine Lösung von 9-BBN (0,5 M in THF, 5,42 ml, 2,71 mmol) mittels Spritze zugesetzt, die Reaktion 35 h lang bei 40 °C gerührt und dann auf Raumtemperatur abkühlen gelassen. Dann wurde Wasser (33 µl, 1,8 mmol) zugesetzt, und das Rühren wurde eine weitere Stunde lang fortgesetzt, um überschüssiges 9-BBN zu zersetzen.

Dann wurde dieses Gemisch durch Einspülen von Argon in die Lösung durch eine Nadel gereinigt, und anschließend wurde trockenes und sauerstofffreies THF zugesetzt, um ein Gesamtvolumen von 7,0 ml zu erhalten, d.h. eine 0,258 M Lösung des borylierten Zwischenprodukts, was die Verwendung von Aliquoten zur nachfolgenden Kupplung ermöglichte.

Ein Aliquot (0,60 ml, 0,16 mmol, 1,0 Äquiv.) dieser Lösung wurde dann mittels Spritze in ein eigenes Gefäß übertragen, das mit einem Rührstäbchen, 5-Brom-1,2,3-tri-methoxybenzol (49,8 mg, 0,202 mmol, 1,3 Äquiv.), Pd(dppf)Cl₂.CH₂Cl₂ (3,2 mg, 3,9 µmol, 2,5 Mol-%) und Cs₂CO₃ (253 mg, 0,775 mmol, 5,0 Äquiv.) unter Argon beladen war, und dann 37 h lang bei Raumtemperatur gerührt. Danach wurde MgSO₄ (19 mg, 0,16 mmol, 1,0 Äquiv.) zugesetzt, und das Rühren wurde 2 h lang fortgesetzt, um Restwasser zu entfernen. Zur Schutzgruppenentfernung wurde eine Lösung von TBAF (1,0 M in THF, 0,26 ml, 0,26 mmol, 1,7 Äquiv.) mittels Spritze zugesetzt und das Gemisch schließlich 12 h lang bei Raumtemperatur gerührt.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 30 auf 100 % EtOAc in PE binnen 50 min) und dann präparativer HPLC (Phenomenex^{®} Luna 10u C18(2) 100 A, 250 x 21,20 mm, Durchflussgeschwindigkeit 20,0 ml/min, Gradient 45 bis 55 % MeOH in Wasser binnen 60 min) gereinigt, um ein farbloses Öl zu erhalten (26,7 mg, 41 %).
**[α]_{D}²³**: +15,1 (c 1,31, iPrOH)

**HR-MS (APCI-PI)** ber. für M+Na⁺: 441,1884, gef.: 441,1893.

**¹H-NMR (200 MHz, CDCl₃):** δ 1,60 (bs, 1H), 2,36-2,63 (m, 2H), 2,66-2,85 (m, 1H), 2,95 (dd, *J* = 12,8, 4,5 Hz, 1H), 3,72-3,99 (m, 3H), 3,83 (s, 3H), 3,84 (s, 6H), 3,87 (s, 3H), 3,89 (s, 3H), 4,07 (dd, *J* = 8,5, 6,3 Hz, 1H), 4,81 (d, *J* = 6,7 Hz, 1H), 6,41 (s, 2H), 6,79-6,92 (m, 3H).

**¹³C-NMR (50 MHz, CDCl₃):** δ 34,1, 42,4, 52,7, 56,0, 56,0, 56,2, 60,9, 61,0, 72,9, 82,8, 105,7, 109,1, 111,1, 118,1, 135,5, 136,3, 136,5, 148,5, 149,2, 153,3.

### Beispiel 5

### 9-20: ((2S,3R,4R)-4-(4-Butylbenzyl)-2-(3,4-dimethoxyphenyl)tetrahydrofuran-3-yl)-methanol (3008)

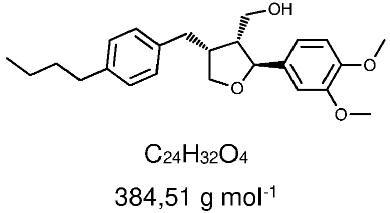

Herstellung identisch mit jener von **9-18,** mit der Ausnahme, dass 1-Brom-4-butylbenzol (42,9 mg, 0,202 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 20 auf 70 % EtOAc in PE binnen 40 min) gereinigt, um ein nahezu farbloses Öl zu erhalten (37,0 mg, 62 %).
**[α]_{D}²³:** +4,7 (c 1,88, iPrOH)

**HR-MS (APCI-PI)** ber. für M+Na⁺: 407,2193, gef.: 407,2194.

**¹H-NMR (200 MHz, CDCl₃):** δ 0,92 (t, *J* = 7,2 Hz, 3H), 1,35 (sext, *J* = 7,2 Hz, 2H), 1,48-1,69 (m, 3H), 2,40 (quint, *J* = 6,7 Hz, 1H), 2,50-2,65 (m, 3H), 2,66-2,85 (m, 1H), 2,92 (dd, *J* = 12,6, 4,8 Hz, 1H), 3,68-3,98 (m, 3H), 3,86 (s, 3H), 3,87 (s, 3H), 4,06 (dd, *J* = 8,5, 6,4 Hz, 1H), 4,83 (d, *J* = 6,3 Hz, 1H), 6,78-6,90 (m, 3H), 7,00-7,14 (m, 4H).

### Vergleichsbeispiel 8

### 9-21: ((2S,3R,4R)-4-(3,5-Dimethoxybenzyl)-2-(3,4-dimethoxyphenyl)tetrahydrofuran-3-yl)methanol

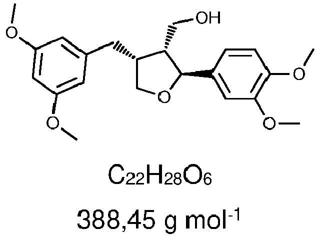

Herstellung identisch mit jener von **9-18,** mit der Ausnahme, dass 1-Brom-3,5-di-methoxybenzol 43,7 mg, 0,202 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 25 bis 75 % EtOAc in PE binnen 40 min) gereinigt, um ein bräunliches Öl zu erhalten (35,1 mg, 58 %).
**[α]_{D}²³:** +13,6 (c 1,69, iPrOH)

**HR-MS (APCI-PI)** ber. für M+Na⁺: 411,1778, gef.: 411,1756.

**¹H-NMR (200 MHz, CDCl₃):** δ 1,67 (bs, 1H), 2,41 (quint, *J* = 6,8 Hz, 1H), 2,55 (dd, *J* = 12,7, 10,1 Hz, 1H), 2,67-2,86 (m, 1H), 2,92 (dd, *J* = 12,7, 4,8 Hz, 1H), 3,64-3,99 (m, 3H), 3,77 (s, 6H), 3,86 (s, 3H), 3,88 (s, 3H), 4,07 (dd, *J* = 8,5, 6,4 Hz, 1H), 4,81 (d, *J* = 6,4 Hz, 1H), 6,29-6,39 (m, 3H), 6,78-6,95 (m, 3H).

### Beispiel 6

### 9-22: ((2S,3R,4R)-4-(4-(1,1 -Difluorethyl)benzyl)-2-(3,4-dimethoxyphenyl)tetrahydrofuran-3-yl)methanol (3013)

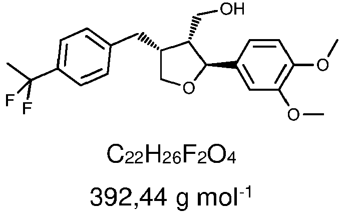

Herstellung identisch mit jener von **9-18,** mit der Ausnahme, dass 1-Brom-4-(1,1-di-fluorethyl)benzol (44,5 mg, 0,202 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 20 auf 70 % EtOAc in PE binnen 40 min) gereinigt, um ein bräunliches Öl zu erhalten (30,5 mg, 50 %).
**[α]_{D}²³:** +10,4 (c 1,52, iPrOH)

**HR-MS (APCI-PI)** ber. für M+Na⁺: 415,1691, gef.: 415,1667.

**¹H-NMR (200 MHz, CDCl₃):** δ 1,65 (bs, 1H), 1,91 (t, *J* = 18,1 Hz, 3H), 2,41 (quint, *J* = 6,7 Hz, 1H), 2,56-2,90 (m, 2H), 3,00 (dd, *J* = 12,2, 3,7 Hz, 1H), 3,63-3,99 (m, 3H), 3,86 (s, 3H), 3,88 (s, 3H), 4,05 (dd, *J* = 8,6, 6,3 Hz, 1H), 4,83 (d, *J* = 6,3 Hz, 1H), 6,78-6,94 (m, 3H), 7,24 (d, *J* = 7,9 Hz, 2H), 7,43 (d, *J* = 8,2 Hz, 2H).

### Vergleichsbeispiele 9 bis 11

In dieser zweiten Beispielsgruppe wurde das Zwischenprodukt 7e mit R₄ = R₆ = H und R₅ = F derivatisiert.

### Vergleichsbeispiel 9

### 9-24: ((2S,3R,4R)-4-(3,4-Dimethoxybenzyl)-2-(4-fluorphenyl)tetrahydrofuran-3-yl)-methanol

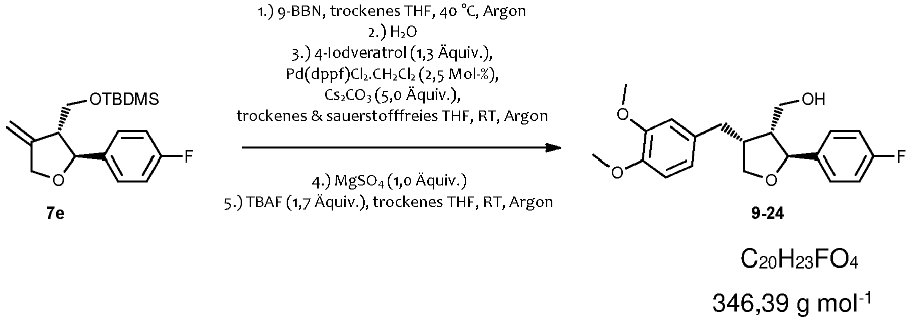

Herstellung: Ein Reaktionsgefäß wurde mit einem Rührstäbchen und rohem Ausgangsmaterial **7e** (89 %, 169,3 mg, 0,467 mmol) beladen und dann evakuiert und mit Argon unter Anwendung eines Standard-Schlenk-Verfahrens (3x) wiederbelüftet. Dann wurde eine Lösung von 9-BBN (0,5 M in THF, 1,40 ml, 0,70 mmol) mittels Spritze zugesetzt, die Reaktion 19 h lang bei 40 °C gerührt und dann auf Raumtemperatur abkühlen gelassen. Anschließend wurde Wasser (9 µl, 0,5 mmol) zugesetzt, und das Rühren wurde 15 min lang fortgesetzt, um überschüssiges 9-BBN zu zersetzen.

Dann wurde dieses Gemisch durch Einspülen von Argon in die Lösung durch eine Nadel gereinigt, und anschließend wurde trockenes und sauerstofffreies THF zugesetzt, um ein Gesamtvolumen von 2,7 ml zu erhalten, d.h. eine 0,173 M Lösung des borylierten Zwischenprodukts, was die Verwendung von Aliquoten zur nachfolgenden Kupplung ermöglichte.

Ein Aliquot (0,90 ml, 0,156 mmol, 1,0 Äquiv.) dieser Lösung wurde dann mittels Spritze in ein eigenes Gefäß übertragen, das mit einem Rührstäbchen, 4-lodveratrol (53,5 mg, 0,202 mmol, 1,3 Äquiv.), Pd(dppf)Cl₂.CH₂Cl₂ (3,2 mg, 3,9 µmol, 2,5 Mol-%) und Cs₂CO₃ (254 mg, 0,779 mmol, 5,0 Äquiv.) unter Argon beladen war, und dann 19,5 h lang bei Raumtemperatur gerührt. Danach wurde MgSO₄ (19 mg, 0,16 mmol, 1,0 Äquiv.) zugesetzt, und das Rühren wurde 1,5 h lang fortgesetzt, um Restwasser zu entfernen. Zur Schutzgruppenentfernung wurde eine Lösung von TBAF (1,0 M in THF, 0,26 ml, 0,26 mmol, 1,7 Äquiv.) mittels Spritze zugesetzt und das Gemisch schließlich 12 h lang bei Raumtemperatur gerührt.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 25 auf 70 % EtOAc in PE binnen 40 min) gereinigt, um ein gelb-oranges Öl zu erhalten (31,5 mg, 58 %).
**[α]_{D}²⁰:** +10,4 (c 0,69, MeOH)

**HR-MS (APCI-PI)** ber. für M+H⁺: 347,1653, gef.: 347,1668.

**¹H-NMR (200 MHz, CDCl₃):** δ 1,46 (bs, 1H, OH), 2,38 (quint, *J* = 6,8 Hz, 1H), 2,56 (dd, *J* = 12,6, 10,2 Hz, 1H), 2,65-2,83 (m, 1H), 2,92 (dd, *J* = 12,7, 4,7 Hz, 1H), 3,77 (dd, *J* = 8,6, 6,2 Hz, 1H), 3,86 (s, 3H), 3,86 (s, 3H), 3,79-4,00 (m, 2H), 4,07 (dd, *J* = 8,5, 6,3 Hz, 1H), 4,87 (d, *J* = 6,2 Hz, 1H), 6,68-6,84 (m, 3H), 6,95-7,08 (m, 2H), 7,24-7,35 (m, 2H).

**¹³C-NMR (50 MHz, CDCl₃):** δ 33,1, 42,4, 52,8, 56,0, 56,0, 60,7, 73,1, 82,4, 111,4, 112,0, 115,3, (d, *J* = 21,4 Hz), 120,5, 127,4 (d, *J* = 8,1 Hz), 133,0, 139,0 (d, *J* = 3,0 Hz), 147,5, 149,0, 162,2 (d, *J* = 245,4 Hz).

### Vergleichsbeispiel 10

### 9-25: ((2S,3R,4R)-2-(4-Fluorphenyl)-4-(4-methoxybenzyl)tetrahydrofuran-3-yl)-methanol

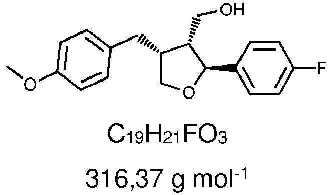

Herstellung identisch mit jener von **9-24,** mit der Ausnahme, dass 4-Bromanisol (37,8 mg, 0,202 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 15 auf 65 % EtOAc in PE binnen 35 min) gereinigt, um ein hellbraunes Öl zu erhalten (28,1 mg, 58 %).
**[α]_{D}²³:** +8,3 (c 1,08, MeOH)

**¹H-NMR (200 MHz, CDCl₃):** δ 1,46 (bs, 1H), 2,35 (quint, *J* = 6,5 Hz, 1H), 2,57 (dd, *J* = 12,5, 9,9 Hz, 1H), 2,61-2,81 (m, 1H), 2,89 (dd, *J* = 12,5, 4,6 Hz, 1H), 3,69-3,83 (m, 2H), 3,79 (s, 3H), 3,93 (dd, *J* = 10,6, 6,9 Hz, 1H), 4,06 (dd, *J* = 8,6, 6,4 Hz, 1H), 4,89 (d, *J* = 6,0 Hz, 1H), 6,79-6,88 (m, 2H), 6,96-7,15 (m, 4H), 7,24-7,34 (m, 2H).

### Veraleichsbeispiel 11

### 9-26: ((2S,3R,4R)-4-(4-Fluorbenzyl)-2-(4-fluorphenyl)tetrahydrofuran-3-yl)methanol

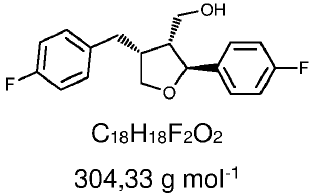

Herstellung identisch mit jener von **9-24,** mit der Ausnahme, dass 1-Brom-4-fluorbenzol (35,4 mg, 0,202 mmol, 1,3 Äquiv.) als Arylhalogenid-Kupplungspartner verwendet wurde.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 10 auf 60 % EtOAc in PE binnen 35 min) gereinigt, um ein hellbraunes Öl zu erhalten (22,6 mg, 48 %).
**[α]_{D}²³:** +12,9 (c 0,77, MeOH)

**¹H-NMR (200 MHz, CDCl₃):** δ 1,47 (bs, 1H), 2,36 (quint, *J* = 6,5 Hz, 1H), 2,52-2,81 (m, 2H), 2,94 (dd, *J* = 12,3, 4,0 Hz, 1H), 3,66-3,98 (m, 3H), 4,05 (dd, *J* = 8,6, 6,3 Hz, 1H), 4,88 (d, *J* = 6,0 Hz, 1H), 6,91-7,08 (m, 4H), 7,09-7,19 (m, 2H), 7,26-7,34 (m, 2H).

### Vergleichsbeispiel 12

In diesem Beispiel wurde das Zwischenprodukt 7c mit R₄ = R₆ = H und R₅ = OCH₃ derivatisiert.

### 9-27: ((2S,3R,4R)-4-(3,4-Dimethoxybenzyl)-2-(4-methoxyphenyl)tetrahydrofuran-3-yl)methanol

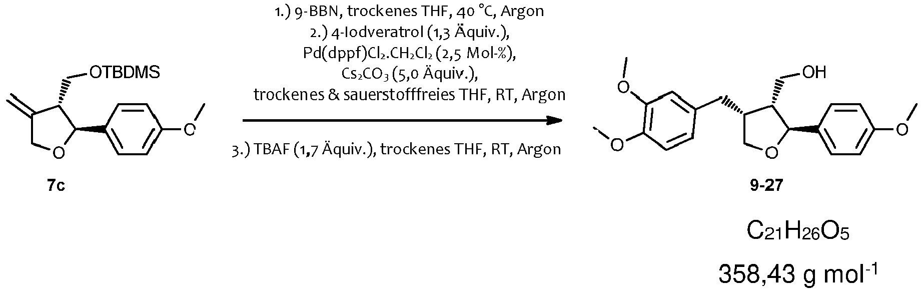

Herstellung: Ein Reaktionsgefäß wurde mit einem Rührstäbchen und rohem Ausgangsmaterial **7c** (137,2 mg, 0,410 mmol) beladen und dann evakuiert und mit Argon unter Anwendung eines Standard-Schlenk-Verfahrens (3x) wiederbelüftet. Dann wurde eine Lösung von 9-BBN (0,5 M in THF, 1,25 ml, 0,61 mmol) mittels Spritze zugesetzt, die Reaktion wurde 18 h lang bei 40 °C gerührt und dann auf Raumtemperatur abkühlen gelassen.

Dann wurde dieses Gemisch durch Einspülen von Argon in die Lösung durch eine Nadel gereinigt, und anschließend wurde trockenes und sauerstofffreies THF zugesetzt, um ein Gesamtvolumen von 3,0 ml zu erhalten, d.h. eine 0,137 M Lösung des borylierten Zwischenprodukts, was die Verwendung von Aliquoten zur nachfolgenden Kupplung ermöglichte.

Ein Aliquot (1,50 ml, 0,21 mmol, 1,0 Äquiv.) dieser Lösung wurde dann mittels Spritze in ein eigenes Gefäß übertragen, das mit einem Rührstäbchen, 4-lodveratrol (70,4 mg, 0,267 mmol, 1,3 Äquiv.), Pd(dppf)Cl₂.CH₂Cl₂ (4,2 mg, 5,1 µmol, 2,5 Mol-%) und Cs₂CO₃ (334 mg, 1,025 mmol, 5,0 Äquiv.) unter Argon beladen war, und dann 77 h lang bei Raumtemperatur gerührt. Zur Schutzgruppenentfernung wurde eine Lösung von TBAF (1,0 M in THF, 0,35 ml, 0,35 mmol, 1,7 Äquiv.) mittels Spritze zugesetzt und das Gemisch schließlich 14 h lang bei Raumtemperatur gerührt.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde zweimal mittels Flash-Säulenchromatographie (18 g Kieselgel, Durchflussgeschwindigkeit 20 ml/min, Gradient 30 auf 80 % EtOAc in PE binnen 40 min) gereinigt, um ein nahezu farbloses Öl zu erhalten (21,4 mg, 29 %).
**[α]_{D}²⁵:** +11,8 (c 1,14, iPrOH)

**HR-MS (APCI-PI)** ber. für M+Na⁺: 381,1672, gef.: 381,1661.

**¹H-NMR (200 MHz, CDCl₃):** δ 1,55 (bs, 1H), 2,40 (quint, *J* = 6,8 Hz, 1H), 2,55 (dd, *J* = 12,6, 10,5 Hz, 1H), 2,65-2,84 (m, 1H), 2,93 (dd, *J* = 12,8, 4,6 Hz, 1H), 3,68-3,98 (m, 3H), 3,79 (s, 3H), 3,86 (s, 6H), 4,05 (dd, *J* = 8,5, 6,4 Hz, 1H), 4,80 (d, *J* = 6,6 Hz, 1H), 6,68-6,83 (m, 3H), 6,87 (d, *J* = 8,6 Hz, 2H), 7,25 (d, *J* = 8,7 Hz, 2H).

**¹³C-NMR (50 MHz, CDCl₃):** δ 33,3, 42,5, 52,8, 55,4, 56,0, 56,0, 61,0, 73,0, 82,7, 111,4, 112,0, 114,0, 120,6, 127,2, 133,1, 135,1, 147,5, 149,1, 159,1.

### Beispiele 7 bis 9 und Vergleichsbeispiele 13 bis 17

In dieser Gruppe erfolgte die Derivatisierung des Zwischenprodukts 7b mit R₄ = R₅ = R₆ = OCH₃.

### Beispiel 7

### 9-28: ((2S,3R,4R)-4-Benzyl-2-(3,4,5-trimethoxyphenyl)tetrahydrofuran-3-yl)methanol (2756)

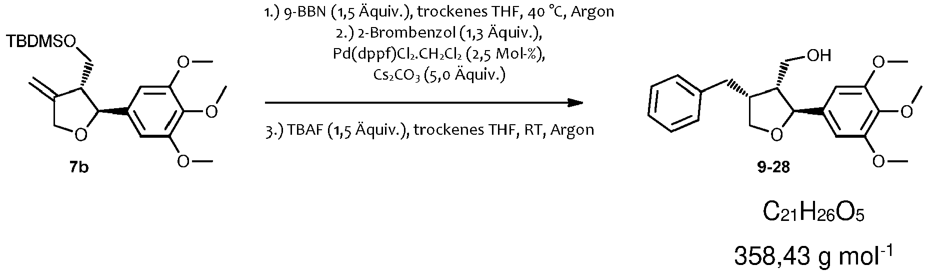

Herstellung: Ein Reaktionsgefäß wurde mit einem Rührstäbchen und rohem Ausgangsmaterial **7b** (52,0 mg, 0,132 mmol, 1,0 Äquiv.) beladen und dann evakuiert und mit Argon unter Anwendung eines Standard-Schlenk-Verfahrens (3x) wiederbelüftet. Dann wurde eine Lösung von 9-BBN (0,5 M in THF, 0,40 ml, 0,20 mmol) mittels Spritze zugesetzt, die Reaktion 24 h lang bei 40 °C gerührt und dann auf Raumtemperatur abkühlen gelassen. Die Lösung wurde mittels Spritze unter Argon in ein gesondertes Gefäß übertragen, das mit einem Rührstäbchen, Brombenzol (26,9 mg, 0,172 mmol, 1,3 Äquiv.), Pd(dppf)Cl₂.CH₂Cl₂ (2,7 mg, 3,3 µmol, 2,5 Mol-%) und Cs₂CO₃ (215 mg, 0,66 mmol, 5,0 Äquiv.) beladen war, und dann 27 h lang bei Raumtemperatur gerührt. Zur Schutzgruppenentfernung wurde eine Lösung von TBAF (1,0 M in THF, 0,20 ml, 0,20 mmol, 1,5 Äquiv.) mittels Spritze zugesetzt und das Gemisch schließlich bei Raumtemperatur 22 h lang gerührt.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein orangefarbenes Öl zu erhalten (22,2 mg, 36 %).
**[α]_{D}²⁰:** +7,8 (c 1,96, MeOH)

**HR-MS (ESI)** ber. für M+Na⁺: 381,1672, gef.: 381,1667.

**¹H-NMR (200 MHz, CDCl₃):** δ 1,91 (bs, 1H), 2,31-3,01 (m, 4H), 3,81 (s, 3H), 3,84 (s, 6H), 4,85 (d, *J* = 5,9 Hz, 1H), 6,54 (s, 2H), 7,12-7,35 (m, 5H).

**¹³C-NMR (50 MHz, CDCl₃):** δ 33,5, 42,1, 52,4, 56,1, 60,8, 60,8, 73,0, 83,0, 102,5, 126,2, 128,6, 138,8, 140,4, 153,3. Ein C_{q} nicht sichtbar.

### Vergleichsbeispiel 13

### 9-29: ((2S,3R,4R)-4-(4-(tert-Butyl)benzyl)-2-(3,4,5-trimethoxyphenyl)tetrahydrofuran-3-yl)methanol (2757)

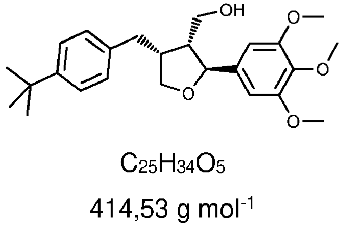

Herstellung analog zu jener von **9-28**, unter Verwendung des rohen Ausgangsmaterials **7b** (57,7 mg, 0,206 mmol, 1,0 Äquiv.) und von 1-Brom-4-(*tert*-butyl)benzol (57,1 mg, 0,268 mmol, 1,3 Äquiv.) als Arylhalogenid-Kupplungspartner.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein nahezu farbloses Öl zu erhalten (41,6 mg, 49 %).
**[α]_{D}²⁰:** +11,0 (c 1,70, MeOH)

**¹H-NMR (200 MHz, CDCl₃):** δ 1,30 (s, 9H), 2,32-2,48 (m, 1H), 2,58 (dd, *J* = 12,4, 9,6 Hz, 1H), 2,68-2,82 (m, 1H), 2,91 (dd, *J* = 12,4, 4,9 Hz, 1H), 3,75 (dd, *J* = 8,6, 6,8 Hz, 1H), 3,81 (s, 3H), 3,84 (s, 6H), 3,93 (dd, *J* = 10,5, 6,8 Hz, 1H), 3,93 (dd, *J* = 8,6, 6,4 Hz, 1H), 4,85 (d, *J* = 5,7 Hz, 1H), 6,55 (s, 2H), 7,11 (d, *J* = 8,3 Hz, 2H), 7,30 (d, *J* = 8,3 Hz, 2H).

**¹³C-NMR (50 MHz, CDCl₃):** δ 31,3, 32,9, 34,3, 42,0, 52,3, 56,1, 60,8, 60,9, 73,1, 83,0, 102,5, 145,4, 128,2, 137,2, 138,9, 149,0, 153,2. Ein C_{q} nicht sichtbar.

### Vergleichsbeispiel 14

### 9-30: ((2S,3R,4R)-4-(4-Methoxybenzyl)-2-(3,4,5-trimethoxyphenyl)tetrahydrofuran-3-yl)methanol

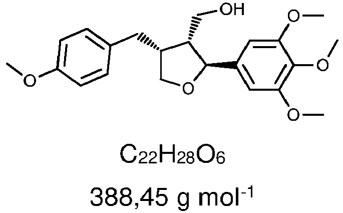

Herstellung analog zu jener von **9-28**, unter Verwendung des rohen Ausgangsmaterials **7b** (52,0 mg, 0,132 mmol, 1,0 Äquiv.) und 4-Bromanisol (32,1 mg, 0,172 mmol, 1,3 Äquiv.) als Arylhalogenid-Kupplungspartner.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein oranges Öl zu erhalten (19,3 mg, 28 %).
**[α]_{D}²⁰:** +3,4 (c 0,72, MeOH)

**HR-MS (ESI)** ber. für M+Na⁺: 411,1778, gef.: 411,1785.

**¹H-NMR (200 MHz, CDCl₃):** δ 1,54 (bs, 1H), 2,31-2,96 (m, 4H), 3,76-3,99 (m, 3H), 3,78 (s, 3H), 3,82 (s, 3H), 3,84 (s, 6H), 4,00-4,11 (m, 1H), 4,84 (d, *J* = 5,9 Hz, 1H), 6,54 (s, 2H), 6,77-6,78 (m, 2H), 7,04-7,15 (m, 2H).

### Beispiel 8

### 9-32: ((2S,3R,4R)-4-(4-(Trifluormethyl)benzyl)-2-(3,4,5-trimethoxyphenyl)tetrahydrofuran-3-yl)methanol (2760)

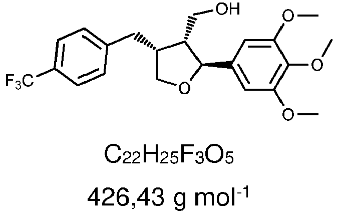

Herstellung analog zu jener von **9-28**, unter Verwendung des rohen Ausgangsmaterials **7b** (52,0 mg, 0,132 mmol, 1,0 Äquiv.) und 1-Brom-4-(trifluormethyl)benzol (38,6 mg, 0,172 mmol, 1,3 Äquiv.) als Arylhalogenid-Kupplungspartner.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein gelbliches Öl zu erhalten (40,2 mg, 62 %).
**[α]_{D}²⁰:** +15,5 (c 0,1,72, MeOH)

**HR-MS (ESI)** ber. für M+Na⁺:449,1546, gef.: 449,1551.

**¹H-NMR (200 MHz, CDCl₃):** δ 1,74 (bs, 1H), 2,33-2,49 (m, 1H), 2,57-3,10 (m, 3H), 3,63-4,10 (m, 4H), 3,81 (s, 3H), 3,84 (s, 6H), 4,83 (d, *J* = 5,9 Hz, 1H), 6,54 (s, 2H), 7,30 (d, *J* = 8,0 Hz, 2H), 7,54 (d, *J* = 8,0 Hz, 2H).

### Vergleichsbeispiel 15

### 9-33: ((2S,3R,4R)-4-(4-Methylbenzyl)-2-(3,4,5-trimethoxyphenyl)tetrahydrofuran-3-yl)methanol

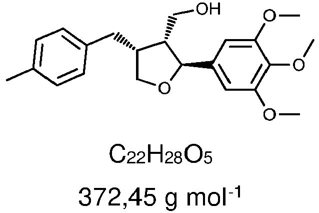

Herstellung analog zu jener von **9-28,** unter Verwendung des rohen Ausgangsmaterials **7b** (57,7 mg, 0,206 mmol, 1,0 Äquiv.) und 1-lodtoluol (58,4 mg, 0,268 mmol, 1,3 Äquiv.) als Arylhalogenid-Kupplungspartner.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein gelbliches Öl zu erhalten (33,2 mg, 43 %).
**[α]_{D}²⁰:** +12,3 (c 1,22, MeOH)

**HR-MS (ESI)** ber. für M+Na⁺: 395,1829, gef.: 395,1829.

**¹H-NMR (200 MHz, CDCl₃):** δ 1,73 (bs, 1H), 2,31 (s, 3H), 2,33-2,79 (m, 4H), 2,83-2,98 (dd, *J* = 12,5, 4,7 Hz, 1H), 3,68-3,98 (m, 3H), 3,81 (s, 3H), 3,84 (s, 6H), 4,00-4,11 (m, 1H), 4,84 (d, *J* = 5,7 Hz, 1H), 6,54 (s, 2H), 7,08 (s, 4H).

### Beispiel 9

### 9-34: ((2S,3R,4R)-4-([1,1'-Biphenyl]-4-ylmethyl)-2-(3,4,5-trimethoxyphenyl)tetrahydrofuran-3-yl)methanol (2767)

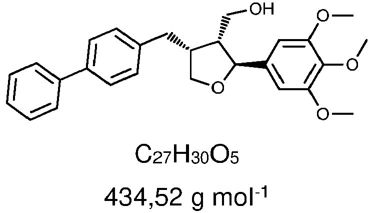

Herstellung analog zu jener von **9-28,** unter Verwendung des rohen Ausgangsmaterials **7b** (57,7 mg, 0,206 mmol, 1,0 Äquiv.) und 4-Brom-1,1'-biphenyl (62,4 mg, 0,268 mmol, 1,3 Äquiv.) als Arylhalogenid-Kupplungspartner.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein gelbes Öl zu erhalten (40,3 mg, 45 %).
**[α]_{D}²⁰:** +7,1 (c 1,72, MeOH)

**¹H-NMR (200 MHz, CDCl₃):** δ 1,78 (bs, 1H), 2,35-2,52 (m, 1H), 2,58-2,90 (m, 2H), 2,93-3,06 (m, 1H), 3,72-3,89 (m, 3H), 3,82 (s, 3H), 3,85 (s, 6H), 3,91-4,02 (m, 1H), 4,87 (d, *J* = 5,7 Hz, 1H), 6,56 (s, 2H), 7,19-7,64 (m, 9H).

**¹³C-NMR (50 MHz, CDCl₃):** δ 33,1, 42,0, 52,3, 56,1, 60,8, 60,9, 73,0, 83,0, 102,5, 126,9, 127,1, 127,2, 128,7, 129,0, 137,0, 138,9, 139,1, 139,4, 140,7, 153,2.

### Vergleichsbeispiel 16

### 9-36: 1-(4-(((3R,4R,5S)-4-(Hydroxymethyl)-5-(3,4,5-trimethoxyphenyl)tetrahydrofuran-3-yl)methyl)phenyl)ethanon

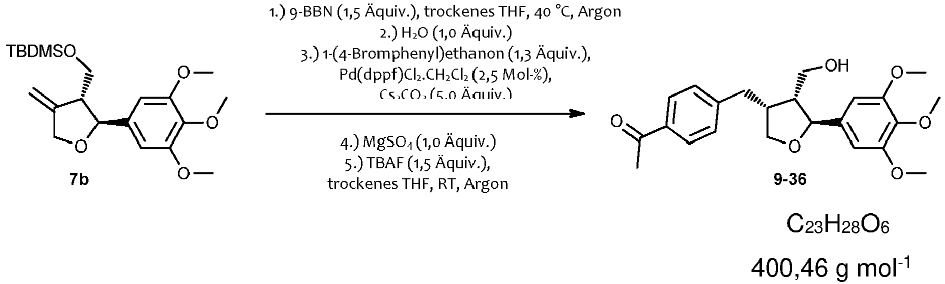

Herstellung: Ein Reaktionsgefäß wurde mit einem Rührstäbchen und rohem Ausgangsmaterial **7b** (57,7 mg, 0,206 mmol, 1,0 Äquiv.) beladen und dann evakuiert und mit Argon unter Anwendung eines Standard-Schlenk-Verfahrens (3x) wiederbelüftet. Dann wurde eine Lösung von 9-BBN (0,5 M in THF, 0,62 ml, 0,31 mmol) mittels Spritze zugesetzt, die Reaktion 24 h lang bei 40 °C gerührt und dann auf Raumtemperatur abkühlen gelassen. Anschließend wurde Wasser (4 µl, 0,21 mmol, 1,0 Äquiv.) zugesetzt, und das Rühren wurde 30 min lang fortgesetzt, um überschüssiges 9-BBN zu zersetzen, bevor die Lösung mittels Spritze unter Argon in ein gesondertes Gefäß übertragen wurde, das mit einem Rührstäbchen, 1-(4-Bromphenyl)ethanon (53,3 mg, 0,268 mmol, 1,3 Äquiv.), Pd(dppf)Cl₂.CH₂Cl₂ (4,2 mg, 5,2 µmol, 2,5 Mol-%) und Cs₂CO₃ (336 mg, 1,03 mmol, 5,0 Äquiv.) beladen war, und dann 27 h lang bei Raumtemperatur gerührt. Danach wurde MgSO₄ (25 mg, 0,21 mmol, 1,0 Äquiv.) zugesetzt, und das Rühren wurde 30 min lang fortgesetzt, um Restwasser zu entfernen. Zur Schutzgruppenentfernung wurde eine Lösung von TBAF (1,0 M in THF, 0,31 ml, 0,31 mmol, 1,5 Äquiv.) mittels Spritze zugesetzt, und das Gemisch wurde schließlich 22 h lang bei Raumtemperatur gerührt.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein gelbes Öl zu erhalten (19,5 mg, 24 %).
**[α]_{D}²⁰:** +10,9 (c 1,55, MeOH)

**HR-MS (ESI)** ber. für M+Na⁺: 423,1778, gef.: 423,1785.

**¹H-NMR (200 MHz, CDCl₃):** δ 1,73 (bs, 1H), 2,30-2,59 (m, 1H), 2,65 (s, 3H), 2,59-2,83 (m, 2H), 2,96-3,08 (m, 1H), 3,64-3,95 (m, 3H), 3,80 (s, 3H), 3,83 (s, 6H), 3,97-4,06 (m, 1H), 4,82 (d, *J* = 6,1 Hz, 1H), 6,53 (s, 2H), 7,28 (d, *J* = 8,4 Hz, 2H), 7,88 (d, *J* = 8,4 Hz, 2H).

**¹³C-NMR (50 MHz, CDCl₃):** δ 26,5, 36,3, 41,9, 52,3, 50,1, 60,8, 60,7, 72,7, 82,9, 102,5, 128,7, 128,8, 135,3, 137,1, 138,6, 146,3, 153,2, 197,8.

### Vergleichsbeispiel 17

### 9-37: Ethyl-4-(((3R,4R,5S)-4-(hydroxymethyl)-5-(3,4,5-trimethoxyphenyl)tetrahydrofuran-3-yl)methyl)benzoat

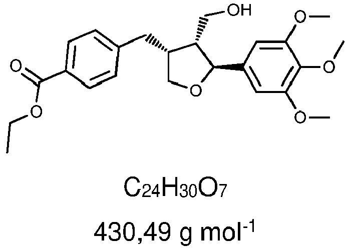

Herstellung analog zu jener von **9-36,** unter Verwendung des rohen Ausgangsmaterials **7b** (57,7 mg, 0,206 mmol, 1,0 Äquiv.) und Ethyl-4-brombenzoat (61,4 mg, 0,268 mmol, 1,3 Äquiv.) als Arylhalogenid-Kupplungspartner.

Aufarbeitung und Reinigung: Zu dem heterogenen Reaktionsinhalt wurden Et₂O und Wasser zugesetzt, die Phasen wurden getrennt und die wässrige Phase mit Et₂O (4x) und EtOAc (2x) extrahiert. Die vereinigten organischen Phasen wurden mit Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 15 auf 70 % EtOAc in PE binnen 45 min) gereinigt, um ein gelbliches Öl zu erhalten (40,0 mg, 45 %).
**[α]_{D}²⁰:** +7,7 (c 2,02, MeOH)

**HR-MS (ESI)** ber. für M+Na⁺: 453,1884, gef.: 453,1886.

**¹H-NMR (400 MHz, CDCl₃):** δ 1,39 (t, *J* = 7,4 Hz, 3H), 1,94 (bs, 1H), 2,38-2,46 (m, 1H), 2,69 (dd, *J* = 12,9, 11,0 Hz, 1H), 2,72-2,82 (m, 1H), 3,07 (dd, *J* = 8,6, 4,3 Hz, 1H), 3,72 (dd, *J* = 8,2, 6,7 Hz, 1H), 3,83 (s, 3H), 3,85 (s, 6H), 3,92 (dd, *J* = 10,4, 6,8 Hz, 1H), 4,04 (dd, *J* = 8,6, 6,7 Hz, 1H), 4,37 (q, *J* = 7,4 Hz, 2H), 4,85 (d, *J* = 5,9, 1H), 6,55 (s, 2H), 7,27 (d, *J* = 8,2 Hz, 2H), 7,97 (d, *J* = 8,2 Hz, 2H).

### Vergleichsbeispiele 18 bis 20

In dieser Gruppe erfolgte die Derivatisierung des Zwischenprodukts 7d mit R₄ = R₅ = R₆ = H.

### Vergleichsbeispiel 18

### 9-39: ((2S,3R,4R)-4-(3,4-Dimethoxybenzyl)-2-phenyltetrahydrofuran-3-yl)methanol

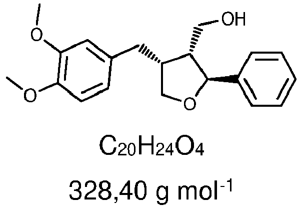

Herstellung analog zu jener von **9-36,** unter Verwendung des rohen Ausgangsmaterials **7d** (81 %, 54,8 mg, 0,146 mmol, 1,0 Äquiv.) und 4-lodveratrol (50,1 mg, 0,190 mmol, 1,3 Äquiv.) als Arylhalogenid-Kupplungspartner, und 42- anstatt 27-stündigem Rühren während des Kupplungsschrittes.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 15 auf 40 % EtOAc in PE binnen 35 min) gereinigt, um ein farbloses Öl zu erhalten (24,5 mg, 51 %).
**[α]_{D}²⁰:** +10,1 (c 7,33, MeOH)

**HR-MS (ESI)** ber. für M+Na⁺: 351,1567, gef.: 351,1569.

**¹H-NMR (200 MHz, CDCl₃):** δ 1,71 (bs, 1H), 2,41 (quint, *J* = 6,6 Hz, 1H), 2,56 (dd, *J* = 12,7, 10,3 Hz, 1H), 2,64-2,83 (m, 1H), 2,92 (dd, *J* = 12,7, 4,6 Hz, 1H), 3,78 (dd, *J* = 8,5, 6,2 Hz, 1H), 3,86 (s, 6H), 3,81-3,99 (m, 2H), 4,08 (dd, *J* = 8,5, 6,4 Hz, 1H), 4,89 (d, *J* = 6,2 Hz, 1H), 6,66-6,83 (m, 3H), 7,20-7,40 (m, 5H).

### Vergleichsbeispiel 19

### 9-40: ((2S,3R,4R)-4-(4-Methoxybenzyl)-2-phenyltetrahydrofuran-3-yl)methanol

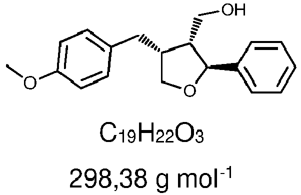

Herstellung analog zu jener von **9-36,** unter Verwendung des rohen Ausgangsmaterials **7d** (81 %, 56,3 mg, 0,150 mmol, 1,0 Äquiv.) und 4-Bromanisol (36,4 mg, 0,195 mmol, 1,3 Äquiv.) als Arylhalogenid-Kupplungspartner, und 42- statt 27-stündigem Rühren während des Kupplungsschrittes.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 10 bis 40 % EtOAc in PE binnen 35 min) gereinigt, um ein farbloses Öl zu erhalten (32,7 mg, 73 %).
**[α]_{D}²³:** +12,8 (c 1,40, MeOH)

**¹H-NMR (200 MHz, CDCl₃):** δ 1,71 (bs, 1H), 2,40 (quint, *J* = 6,4 Hz, 1H), 2,57 (dd, *J* = 12,5, 10,0 Hz, 1H), 2,64 -2,82 (m, 1H), 2,90 (dd, *J* = 12,5, 4,6 Hz, 1H), 3,70-3,84 (m, 2H), 3,78 (s, 3H), 3,93 (dd, *J* = 10,8, 6,9 Hz, 1H), 4,08 (dd, *J* = 8,5, 6,4 Hz, 1H), 4,90 (d, *J* = 6,0 Hz, 1H), 6,78-6,88 (m, 2H), 7,06-7,15 (m, 2H), 7,21 -7,39 (m, 5H).

### Vergleichsbeispiel 20

### 9-41: ((2S,3R,4R)-2-Phenyl-4-(4-(trifluormethyl)benzyl)tetrahydrofuran-3-yl)methanol

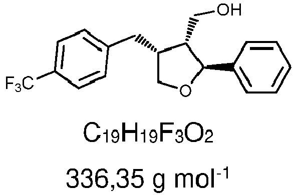

Herstellung analog zu jener von **9-36,** unter Verwendung des rohen Ausgangsmaterials **7d** (81 %, 44,5 mg, 0,118 mmol, 1,0 Äquiv.) und 1-Brom-4-(trifluormethyl)benzol (34,6 mg, 0,154 mmol, 1,3 Äquiv.) als Arylhalogenid-Kupplungspartner und 42- statt 27-stündigem Rühren während des Kupplungsschrittes.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 10 auf 40 % EtOAc in PE binnen 35 min) gereinigt, um ein farbloses Öl zu erhalten (17,4 mg, 44 %).
**[α]_{D}²³:** +15,3 (c 1,61, MeOH)

**¹H-NMR (200 MHz, CDCl₃):** δ 1,59 (bs, 1H), 2,42 (quint, *J* = 6,4 Hz, 1H), 2,62-2,86 (m, 2H), 2,99-3,10 (m, 1H), 3,67-3,99 (m, 3H), 4,06 (dd, *J* = 8,5, 6,2 Hz, 1H), 4,90 (d, *J* = 6,1 Hz, 1H), 7,21-7,40 (m, 7H), 7,55 (d, *J* = 8,1 Hz, 2H).

### Vergleichsbeispiele 21 und 22

In dieser Beispielsgruppe wurde erneut das Zwischenprodukt 7e mit R₄ = R₆ = H und R₅ = F derivatisiert.

### Vergleichsbeispiel 21

### 9-42: ((2S,3R,4R)-4-(4-(tert-Butyl)benzyl)-2-(4-fluorphenyl)tetrahydrofuran-3-yl)-methanol

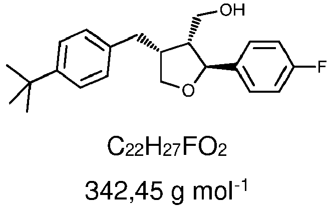

Herstellung analog zu jener von **9-36,** unter Verwendung des rohen Ausgangsmaterials **7e** (41,3 mg, 0,128 mmol, 1,0 Äquiv.) und 1-Brom-4-(*tert*-butyl)benzol (35,5 mg, 0,166 mmol, 1,3 Äquiv.) als Arylhalogenid-Kupplungspartner und 42- statt 27-stündigem Rühren während des Kupplungsschrittes.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 10 bis 80 % EtOAc in PE binnen 60 min) gereinigt, um ein farbloses Öl zu erhalten (13,5 mg, 31 %).
**[α]_{D}²³:** +6,8 (c 1,07, MeOH) α

**¹H-NMR (200 MHz, CDCl₃):** δ 1,31 (s, 9H), 1,49 (bs, 1H), 2,36 (quint, *J* = 6,5, 1H), 2,52-2,96 (m, 3H), 3,69-3,84 (m, 2H), 3,93 (dd, *J* = 10,8, 6,9 Hz, 1H), 4,09 (dd, *J* = 8,6, 6,3 Hz, 1H), 4,90 (d, *J* = 5,8 Hz, 1H), 6,95-7,15 (m, 4H), 7,26 -7,35 (m, 4H).

### Vergleichsbeispiel 22

### 9-43: ((2S,3R,4R)-2-(4-Fluorphenyl)-4-(4-(trifluormethyl)benzyl)tetrahydrofuran-3-yl)-methanol

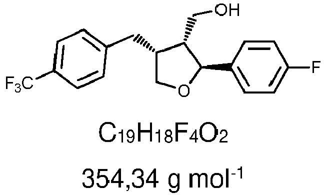

Herstellung analog zu jener von **9-36,** unter Verwendung des rohen Ausgangsmaterials **7e** (39,0 mg, 0,121 mmol, 1,0 Äquiv.) und 1-Brom-4-(trifluormethyl)benzol (35,4 mg, 0,157 mmol, 1,3 Äquiv.) als Arylhalogenid-Kupplungspartner, und 42- statt 27-stündigem Rühren während des Kupplungsschrittes.

Aufarbeitung und Reinigung: Der heterogene Reaktionsinhalt wurde filtriert und mit CH₂Cl₂ (20 ml) gespült, die Lösungsmittel wurden abgedampft, und die Zielverbindung wurde mittels Flash-Säulenchromatographie (18 g Kieselgel, Durchflussgeschwindigkeit 20 ml/min, Gradient 15 auf 40 % EtOAc in PE binnen 35 min) gereinigt, um ein nahezu farbloses Öl zu erhalten (17,5 mg, 41 %).
**[α]_{D}²³:** +12,0 (c 1,36, MeOH)

**¹H-NMR (200 MHz, CDCl₃):** δ 1,84 (bs, 1H), 2,36 (quint, *J* = 6,5 Hz, 1H), 2,59-2,84 (m, 2H), 3,02 (d, *J* = 9,0 Hz, 1H), 3,65-3,95 (m, 3H), 4,03 (dd, *J* = 8,5, 6,3 Hz, 1H), 4,87 (d, *J* = 6,1 Hz, 1H), 6,94-7,07 (m, 2H), 7,21-7,34 (m, 4H), 7,54 (d, *J* = 8,1 Hz, 2H).

### Beispiele 10 bis 22 und Vergleichsbeispiele 23 bis 34

In dieser und den beiden folgenden Beispielsgruppen werden nun schließlich freie Alkohole der Formel (9) durch Veresterung bzw. Veretherung in Verbindungen der Formel (10) übergeführt (mit R7 ≠ OH). Zunächst werden Mitsunobu-Veresterungen offenbart. Die Bezeichnung der erhaltenen Verbindungen ist analog zu jenen der Formel (9), d.h. die Formelzahl 10, gefolgt von einer weiteren, prinzipiell fortlaufend ansteigenden Zahl (mit entsprechenden fehlenden Verbindungen in der Reihe).

### Vergleichsbeispiel 23

### 10-1: (Z)-((2S,3R,4R)-4-Benzyl-2-(3,4-dimethoxyphenyl)tetrahydrofuran-3-yl)methyl-2-methylbut-2-enoat

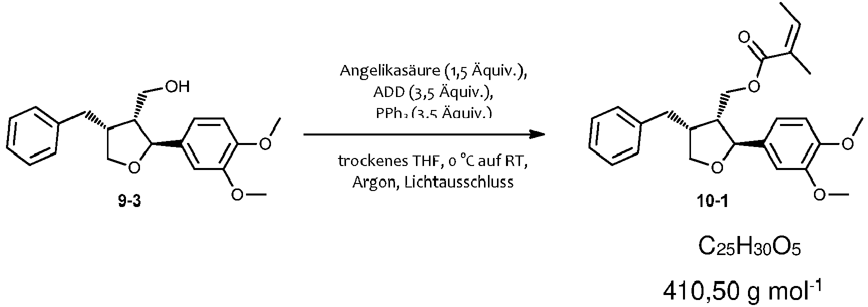

Herstellung: Ein Reaktionsgefäß wurde mit einem Rührstäbchen, Ausgangsmaterial **9-3** (24,4 mg, 0,074 mmol, 1,0 Äquiv.), Angelikasäure (11,2 mg, 0,111 mmol, 1,5 Äquiv.) und PPh₃ (68,2 mg, 0,260 mmol, 3,5 Äquiv.) beladen und dann evakuiert und mit Argon unter Anwendung eines Standard-Schlenk-Verfahrens (3x) wiederbelüftet. Trockenes THF (0,75 ml) wurde dann zugesetzt, und die Lösung wurde in einem Eisbad auf 0 °C gekühlt. Zu dem gerührten Gemisch wurde dann eine Lösung von ADD (65,6 mg, 0,260 mmol, 3,5 Äquiv.) in trockenem THF (1,0 ml) mittels Spritze binnen etwa 1 min zugesetzt, und die Reaktion wurde 22 h lang gerührt und dabei unter Lichtausschluss langsam auf Raumtemperatur erwärmen gelassen.

Aufarbeitung und Reinigung: Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, Gradient 1 auf 35 % EtOAc in PE binnen 30 min) gereinigt, um ein farbloses Öl zu erhalten (26,4 mg, 86 %).
**[α]_{D}²⁵:** +31,6 (c 2,64, MeOH)

**HR-MS (ESI)** ber. für M+Na⁺: 433,1985, gef.: 433,1968.

**¹H-NMR (200 MHz, CDCl₃):** δ 1,85-1,91 (m, 3H), 2,00 (dq, *J* = 7,3, 1,5 Hz, 3H), 2,70-2,53 (m, 2H), 2,89-2,70 (m, 1H), 2,95 (dd, *J* = 12,5, 4,1 Hz, 1H), 3,77 (dd, *J* = 8,6, 6,3 Hz, 1H), 3,87 (s, 3H), 3,88 (s, 3H), 4,07 (dd, *J* = 8,6, 6,2 Hz, 1H), 4,28 (dd, *J* = 11,4, 7,0 Hz, 1H), 4,41 (dd, *J* = 11,4, 7,0 Hz, 1H), 4,85 (d, *J* = 6,3 Hz, 1H), 6,10 (qq, *J* = 7,2, 1,4 Hz, 1H), 6,92-6,79 (m, 3H), 7,36-7,13 (m, 5H).

**¹³C-NMR (50 MHz, CDCl₃):** δ 16,0, 20,7, 33,7, 42,5, 49,3, 56,0, 56,1, 62,3, 72,8, 83,0, 108,9, 111,1, 118,1, 126,4, 127,5, 128,7, 128,7, 135,1, 139,1, 140,2, 148,6, 149,2, 167,8.

### Beispiel 10

### 10-4: (Z)-((2S,3R,4R)-4-(4-(tert-Butyl)benzyl)-2-(3,4-dimethoxyphenyl)tetrahydrofuran-3-yl)methyl-2-methylbut-2-enoat (2788)

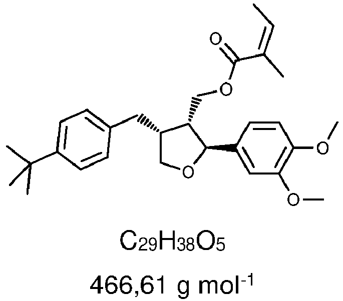

Herstellung analog zu jener von **10-1,** unter Verwendung von Ausgangsmaterial **9-5** (31,1 mg, 0,081 mmol, 1,00 Äquiv.).

Aufarbeitung und Reinigung: Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, isokratisch bei 7 % EtOAc in PE 3 min lang, dann Gradient 7 auf 35 % binnen 30 min) gereinigt, um ein farbloses Öl zu erhalten (25,4 mg, 67 %).
**[α]_{D}²⁰:** +21,4 (c 1,52, MeOH)

**HR-MS (APCI-PI)** ber. für M+Na⁺: 489,2611, gefunden 489,2676.

**¹H-NMR (200 MHz, CDCl₃):** δ 1,31 (s, 9H), 1,85-1,90 (m, 3H), 2,00 (dq, *J* = 7,2, 1,5 Hz, 3H), 2,53-2,86 (m, 3H), 2,91 (dd, *J* = 12,4, 4,2 Hz, 1H), 3,80 (dd, *J* = 8,5, 6,3 Hz, 1H), 3,87 (s, 3H), 3,88 (s, 3H), 4,09 (dd, *J* = 8,6, 6,3 Hz, 1H), 4,28 (dd, *J* = 11,3, 7,0 Hz, 1H), 4,42 (dd, *J* = 11,4, 7,0 Hz, 1H), 4,84 (d, *J* = 6,3 Hz, 1H), 6,10 (qq, *J* = 7,2, 1,4 Hz, 1H), 6,79-6,92 (m, 3H), 7,10 (d, *J* = 8,3 Hz, 2H), 7,32 (d, *J* = 8,3 Hz, 2H).

### Beispiel 11

### 10-8: Ethyl-4-(((3R,4R,5S)-5-(3,4-Dimethoxyphenyl)-4-((((Z)-2-methylbut-2-enoyl)-oxy)methyl)tetrahydrofuran-3-yl)methyl)benzoat (2792)

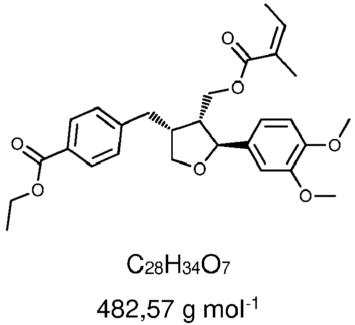

Herstellung analog zu jener von **10-1,** unter Verwendung von Ausgangsmaterial **9-6** (26,7 mg, 0,067 mmol, 1,00 Äquiv.).

Aufarbeitung und Reinigung: Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, Gradient 5 auf 40 % EtOAc binnen 30 min) gereinigt, um ein farbloses Öl zu erhalten (28,8 mg, 89 %).
**[α]_{D}²⁵:** +18,4 (c 1,66, MeOH)

**HR-MS (APCI-PI)** ber. für M+Na⁺: 505,2197, gef.: 505,2229.

**¹H-NMR (200 MHz, CDCl₃):** δ 1,39 (t, *J* = 7,1 Hz, 3H), 1,88 (s, 3H), 2,00 (d, *J* = 7,3 Hz, 3H), 2,55-2,90 (m, 3H), 2,94-3,06 (m, 1H), 3,74 (dd, *J* = 8,7, 6,0 Hz, 1H), 3,87 (s, 3H), 3,87 (s, 3H), 4,06 (dd, *J* = 8,7, 6,0 Hz, 1H), 4,20-4,46 (m, 4H), 4,84 (d, *J* = 6,4 Hz, 1H), 6,11 (q, *J* = 7,3 Hz, 1H), 6,79-6,91 (m, 3H), 7,25 (d, *J* = 7,9 Hz, 2H), 7,98 (d, *J* = 8,1 Hz, 2H).

### Beispiel 12

### 10-11: (Z)-((2S,3R,4R)-2-(3,4-Dimethoxyphenyl)-4-(pyridin-2-ylmethyl)tetrahydrofuran-3-yl)methyl-2-methylbut-2-enoat (2823)

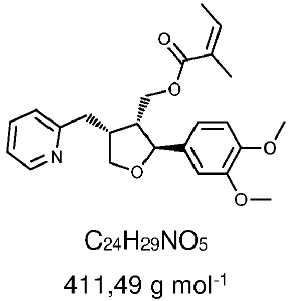

Herstellung analog zu jener von **10-1,** unter Verwendung von Ausgangsmaterial **9-13** (21,9 mg, 0,066 mmol, 1,00 Äquiv.).

Aufarbeitung und Reinigung: Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, Gradient 20 auf 50 % EtOAc binnen 40 min) und dann mittels präparativer HPLC (Phenomenex^{®} Luna 10u C18(2) 100 A, 250 x 21,20 mm, Durchflussgeschwindigkeit 20,0 ml/min, isokratisch bei 60 % MeOH in Wasser) gereinigt, um ein farbloses Öl zu erhalten (14,2 mg, 52 %).
**[α]_{D}²³:** +33,0 (c 0,80, MeOH)

**HR-MS (ESI)** ber. für M+H⁺: 412,2118, gef.: 412,2107.

**¹H-NMR (200 MHz, CDCl₃):** δ 1,82-1,89 (m, 3H), 1,98 (dd, *J* = 7,2, 1,5 Hz, 3H), 2,64 (quint, *J* = 6,7 Hz, 1H), 2,86 (dd, *J* = 15,2, 11,9 Hz, 1H), 2,98-3,19 (m, 2H), 3,77 (dd, *J* = 8,5, 6,9 Hz, 1H), 3,86 (s, 3H), 3,88 (s, 3H), 4,15 (dd, *J* = 8,5, 6,5 Hz, 1H), 4,27 (dd, *J* = 11,4, 7,0 Hz, 1H), 4,42 (dd, *J* = 11,4, 6,7 Hz, 1H), 4,85 (d, *J* = 6,2 Hz, 1H), 6,08 (qd, *J* = 7,2, 1,3 Hz, 1H), 6,78-6,90 (m, 3H), 7,12-7,21 (m, 2H), 7,64 (td, *J* = 7,7, 1,7 Hz, 1H), 8,56 (d, *J* = 4,5 Hz, 1H).

### Vergleichsbeispiel 24

### 10-13: (Z)-((2S,3R,4R)-2-(3,4-Dimethoxyphenyl)-4-(4-(trifluormethyl)benzyl)tetrahydrofuran-3-yl)methyl-2-methylbut-2-enoat (2827)

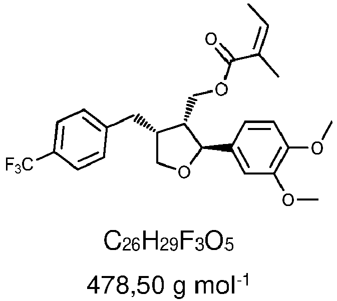

Herstellung analog zu jener von **10-1,** unter Verwendung von Ausgangsmaterial **9-17** (32,7 mg, 0,082 mmol, 1,00 Äquiv.).

Aufarbeitung und Reinigung: Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, Gradient 1 auf 40 % EtOAc in PE binnen 40 min) gereinigt, um ein farbloses Öl zu erhalten (34,3 mg, 87 %).
**[α]_{D}²⁰:** +28,5 (c 0,97, MeOH)

**HR-MS (ESI)** ber. für M+Na⁺: 501,1859, gef.: 501,1882

**¹H-NMR (200 MHz, CDCl₃):** δ 1,85-1,90 (m, 3H), 2,00 (dq, *J* = 7,2, 1,5 Hz, 3H), 2,55-2,89 (m, 3H), 2,95-3,07 (m, 1H), 3,74 (dd, *J* = 8,7, 5,9 Hz, 1H), 3,87 (s, 3H), 3,88 (s, 3H), 4,07 (dd, *J* = 8,8, 6,0 Hz, 1H), 4,28 (dd, *J* = 11,4, 6,6 Hz, 1H), 4,40 (dd, *J* = 11,4, 7,1 Hz, 1H), 4,84 (d, *J* = 6,4 Hz, 1H), 6,12 (qd, *J* = 7,2, 1,3 Hz, 1H), 6,79-6,92 (m, 3H), 7,30 (d, *J* = 8,1 Hz, 2H), 7,56 (d, *J* = 8,1 Hz, 2H).

### Beispiel 13

### 10-16: (Z)-((2S,3R,4R)-4-(4-Butylbenzyl)-2-(3,4-dimethoxyphenyl)tetrahydrofuran-3-yl)methyl-2-methylbut-2-enoat (3026)

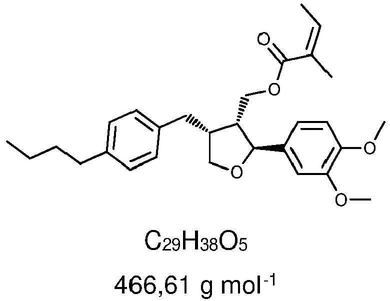

Herstellung analog zu jener von **10-1,** unter Verwendung von Ausgangsmaterial **9-20** (31,8 mg, 0,083 mmol, 1,00 Äquiv.) und 44- statt 22-stündigem Rühren.

Aufarbeitung und Reinigung: Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, Gradient 1 auf 40 % EtOAc in PE binnen 45 min) gereinigt, um ein farbloses Öl zu erhalten (29,8 mg, 77 %).
**[α]_{D}²⁵:** +17,1 (c 1,54, iPrOH)

**¹H-NMR (200 MHz, CDCl₃):** δ 0,92 (t, *J* = 7,2 Hz, 3H), 1,35 (sext, *J* = 7,2 Hz, 2H), 1,49-1,67 (m, 2H), 1,84-1,91 (m, 3H), 2,00 (dd, *J* = 7,3, 1,5 Hz, 3H), 2,50-2,86 (m, 5H), 2,91 (dd, *J* = 12,5, 4,2 Hz, 1H), 3,77 (dd, *J* = 8,5, 6,3 Hz, 1H), 3,86 (s, 3H), 3,87 (s, 3H), 4,08 (dd, *J* = 8,6, 6,3 Hz, 1H), 4,28 (dd, *J* = 11,3, 7,0 Hz, 1H), 4,41 (dd, *J* = 11,3, 7,0 Hz, 1H), 4,84 (d, *J* = 6,2 Hz, 1H), 6,09 (qd, *J* = 7,2, 1,4 Hz, 1H), 6,78-6,92 (m, 3H), 7,03-7,15 (m, 4H).

**¹³C-NMR (50 MHz, CDCl₃):** δ 14,1, 15,9, 20,7, 22,5, 33,3, 33,8, 35,3, 42,6, 49,3, 56,0, 56,1, 62,4, 72,9, 83,0, 109,0, 111,2, 118,2, 127,5, 128,6, 128,7, 135,2, 137,3, 139,0, 141,0, 148,6, 149,2, 167,8.

### Beispiel 14

### 10-18: (Z)-((2S,3R,4R)-4-(4-(1,1-Difluorethyl)benzyl)-2-(3,4-dimethoxyphenyl)tetrahydrofuran-3-yl)methyl-2-methylbut-2-enoat (3029)

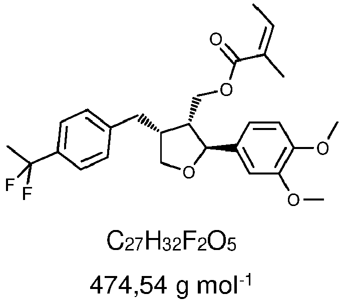

Herstellung analog zu jener von **10-1,** unter Verwendung von Ausgangsmaterial **9-22** (23,2 mg, 0,059 mmol, 1,00 Äquiv.) und 44- statt 22-stündigem Rühren.

Aufarbeitung und Reinigung: Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, Gradient 1 auf 40 % EtOAc in PE binnen 40 min) gereinigt, um ein farbloses Öl zu erhalten (21,8 mg, 78 %).
**[α]_{D}²⁵:** +19,9 (c 1,22, iPrOH)

**¹H-NMR (200 MHz, CDCl₃):** δ 1,85-1,90 (m, 3H), 1,91 (t, *J* = 18,2 Hz, 3H), 2,00 (dd, *J* = 7,2, 1,5 Hz, 3H), 2,55-2,88 (m, 3H), 2,91-3,03 (m, 1H), 3,75 (dd, *J* = 8,7, 6,0 Hz, 1H), 3,87 (s, 3H), 3,88 (s, 3H), 4,07 (dd, *J* = 8,7, 6,1 Hz, 1H), 4,28 (dd, *J* = 11,3, 6,8 Hz, 1H), 4,41 (dd, *J* = 11,4, 7,1 Hz, 1H), 4,84 (d, *J* = 6,3 Hz, 1H), 6,11 (qd, *J* = 7,2, 1,4 Hz, 1H), 6,80-6,91 (m, 3H), 7,23 (d, *J* = 8,1 Hz, 2H), 7,44 (d, *J* = 8,1 Hz, 2H).

**¹³C-NMR (50 MHz, CDCl₃):** δ 16,0, 20,7, 26,0 (t, *J* = 29,9 Hz), 33,5, 42,4, 49,3, 56,0, 56,1, 62,2, 72,7, 83,0, 109,0, 111,2, 118,2, 121,9, 125,1 (t, *J* = 5,9 Hz), 127,5, 128,8, 134,9, 139,2, 142,0, 148,7, 149,2, 167,8. Ein C_{q} nicht sichtbar.

### Beispiel 15

### 10-19: (Z)-((2S,3R,4R)-2-(4-Fluorphenyl)-4-(4-(trifluormethyl)benzyl)tetrahydrofuran-3-yl)methyl-2-methylbut-2-enoat (3016)

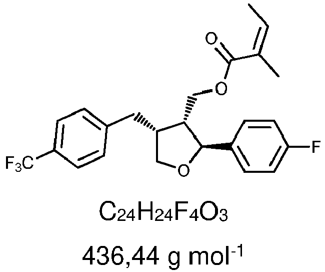

Herstellung analog zu jener von **10-1,** unter Verwendung von Ausgangsmaterial **9-43** (13,3 mg, 0,038 mmol, 1,0 Äquiv.) und 18,5- statt 22-stündigem Rühren.

Aufarbeitung und Reinigung: Das Lösungsmittel wurde abgedampft, und die Zielverbindung mittels Flash-Säulenchromatographie (9 g Kieselgel, Durchflussgeschwindigkeit 20 ml/min, Gradient 2 auf 10 % EtOAc in PE binnen 30 min) gereinigt, um ein farbloses Öl zu erhalten (10,1 mg, 61 %).
**[α]_{D}²⁰:** +13,8 (c 1,02, iPrOH)

**HR-MS (APCI-PI)** ber. für M+H⁺: 437,1734, gef.: 437,1756.

**¹H-NMR (200 MHz, CDCl₃):** δ 1,83-1,90 (m, 3H), 2,00 (dd, *J* = 7,2, 1,5 Hz, 3H), 2,50-2,96 (m, 3H), 3,00 (d, *J* = 8,7 Hz, 1H), 3,75 (dd, *J* = 8,7, 6,0 Hz, 1H), 4,07 (dd, *J* = 8,7, 6,0 Hz, 1H), 4,27 (dd, *J* = 11,4, 6,9 Hz, 1H), 4,41 (dd, *J* = 11,4, 6,9 Hz, 1H), 4,89 (d, *J* = 6,2 Hz, 1H), 6,12 (qd, *J* = 7,2, 1,4 Hz, 1H), 6,95-7,11 (m, 2H), 7,22-7,36 (m, 4H), 7,56 (d, *J* = 8,2 Hz, 2H).

### Vergleichsbeispiel 25

### 10-20: (Z)-((2S,3R,4R)-4-(3,4-Dimethoxybenzyl)-2-phenyltetrahydrofuran-3-yl)methyl-2-methylbut-2-enoat

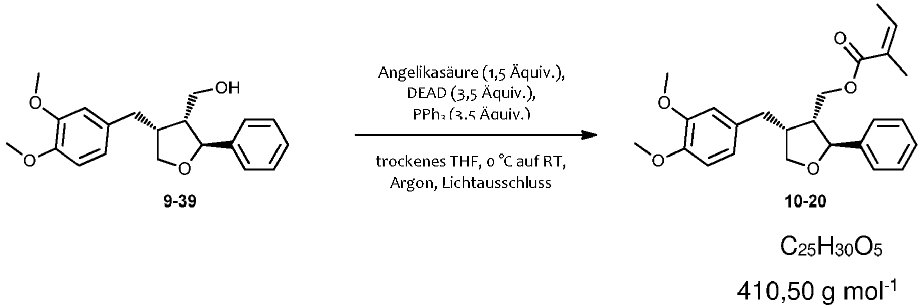

Herstellung: Ein Reaktionsgefäß wurde mit einem Rührstäbchen, Ausgangsmaterial **9-39** (40,4 mg, 0,123 mmol, 1,0 Äquiv.), Angelikasäure (18,2 mg, 0,182 mmol, 1,5 Äquiv.) und PPh₃ (117,5 mg, 0,431 mmol, 3,5 Äquiv.) beladen und dann evakuiert und mit Argon unter Anwendung eines Standard-Schlenk-Verfahrens (3x) wiederbelüftet. Trockenes THF (1,5 ml) wurde dann zugesetzt, und die Lösung wurde in einem Eisbad auf 0 °C gekühlt. Dann wurde zu dem gerührten Gemisch DEAD (73 mg, 0,421 mmol, 3,5 Äquiv.) mittels Spritze zugetropft, und die Reaktion wurde 18 h lang gerührt und dabei unter Lichtausschluss langsam auf Raumtemperatur erwärmen gelassen.

Aufarbeitung und Reinigung: Das Lösungsmittel wurde abgedampft, worauf Zusatz von CHCl₃ (1,0 ml), PE (10 ml) und Wasser (10 ml) folgte. Die Phasen wurden getrennt, und die wässrige Phase wurde mit PE neuerlich extrahiert. Die Lösungsmittel wurden aus den vereinigten organischen Phasen abgedampft, und die Zielverbindung wurde zuerst mittels Flash-Säulenchromatographie (90 g Kieselgel, Durchflussgeschwindigkeit 30 ml/min, isokratisch bei 5 % EtOAc in PE 5 min lang, dann Gradient 5 auf 10 % binnen 20 min, dann 10 auf 30 % binnen 20 min, schließlich 30 auf 100 % binnen 10 min), anschließend mittels präparativer HPLC (Phenomenex^{®} Luna 10u C18(2) 100 A, 250 x 21,20 mm, Durchflussgeschwindigkeit 20,0 ml/min, isokratisch bei 73 % MeOH in Wasser 25 min lang, dann Gradient 73 auf 77 % binnen 15 min) gereinigt, um ein farbloses Öl zu erhalten (24,0 mg, 48 %).
**[α]_{D}²⁰:** +17,2 (c 1,13, MeOH)

**HR-MS (ESI)** ber. für M+Na⁺: 433,1985, gef.: 433,1993.

**¹H-NMR (200 MHz, CDCl₃):** δ 1,84-1,91 (m, 3H), 2,00 (dq, *J* = 7,2, 1,5 Hz, 3H), 2,50-2,85 (m, 3H), 2,89 (dd, *J* = 12,3, 4,2 Hz, 1H), 3,81 (dd, *J* = 8,6, 6,2 Hz, 1H), 3,86 (s, 6H), 4,10 (dd, *J* = 8,6, 6,2 Hz, 1H), 4,28 (dd, *J* = 11,3, 7,1 Hz, 1H), 4,44 (dd, *J* = 11,3, 6,8 Hz, 1H), 4,93 (d, *J* = 5,8 Hz, 1H), 6,10 (qq, *J* = 7,2, 1,4 Hz, 1H), 6,66-6,75 (m, 2H), 6,80 (d, *J* = 7,9 Hz, 1H), 7,21-7,39 (m, 5H).

**¹³C-NMR (50 MHz, CDCl₃):** δ 16,0, 20,7, 33,2, 42,7, 49,5, 56,0, 56,0, 62,3, 72,9, 83,1, 111,4, 112,0, 120,6, 125,8, 127,5, 127,6, 128,6, 132,7, 139,0, 142,8, 147,6, 149,1, 167,8.

### Vergleichsbeispiel 26

### 10-21: (Z)-((2S,3R,4R)-4-(3,4-Dimethoxybenzyl)-2-(4-fluorphenyl)tetrahydrofuran-3-yl)methyl-2-methylbut-2-enoat (2548)

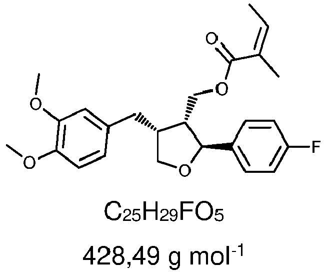

Herstellung analog zu jener von **10-20,** unter Verwendung von Ausgangsmaterial **9-24** (36,4 mg, 0,105 mmol, 1,0 Äquiv.).

Aufarbeitung und Reinigung: Das Lösungsmittel wurde abgedampft, worauf Zusatz von CHCl₃ (1,0 ml), PE (10 ml) und Wasser (10 ml) folgte. Die Phasen wurden getrennt, und die wässrige Phase wurde mit PE neuerlich extrahiert. Die Lösungsmittel wurde aus den vereinigten organischen Phasen abgedampft, und die Zielverbindung wurde zuerst mittels Flash-Säulenchromatographie (45 g Kieselgel, Durchflussgeschwindigkeit 30 ml/min, isokratisch bei 3 % EtOAc in PE 3 min lang, dann Gradient 3 auf 10 % binnen 40 min), anschließend mittels präparativer HPLC (Phenomenex^{®} Luna 10u C18(2) 100 A, 250 x 21,20 mm, Durchflussgeschwindigkeit 20,0 ml/min, isokratisch bei 73 % MeOH in Wasser 25 min lang, dann Gradient 73 auf 77 % binnen 15 min) gereinigt, um ein farbloses Öl zu erhalten (24,7 mg, 55 %).
**[α]_{D}²³:** +15,9 (c 0,90, MeOH)

**HR-MS (ESI)** ber. für M+Na⁺: 451,1891, gef.: 451,1892.

**¹H-NMR (200 MHz, CDCl₃):** δ 1,83-1,89 (m, 3H), 2,00 (dq, *J* = 7,2, 1,5 Hz, 3H), 2,49-2,84 (m, 3H), 2,89 (dd, *J* = 12,5, 4,2 Hz, 1H), 3,79 (dd, *J* = 8,6, 6,2 Hz, 1H), 3,86 (s, 3H), 3,86 (s, 3H), 4,08 (dd, *J* = 8,6, 6,2 Hz, 1H), 4,27 (dd, *J* = 11,3, 7,3 Hz, 1H), 4,43 (dd, *J* = 11,3, 6,8 Hz, 1H), 4,89 (d, *J* = 6,1 Hz, 1H), 6,10 (qd, *J* = 7,2, 1,4 Hz, 1H), 6,65-6,84 (m, 3H), 6,95-7,08 (m, 2H), 7,23-7,34 (m, 2H).

### Vergleichsbeispiel 27

### 10-22: (Z)-((2S,3R,4R)-4-(3,4-Dimethoxybenzyl)-2-(3,4-dimethoxyphenyl)tetrahydrofuran-3-yl)methyl-2-methylbut-2-enoat (Leoligin) (2418)

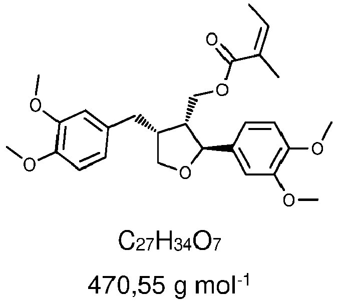

Herstellung analog zu jener von **10-20,** unter Verwendung von Ausgangsmaterial **9-1** (989 mg, 2,55 mmol, 1,0 Äquiv.), THF (20 ml) für die Reaktion und 12-stündigem Rühren.

Aufarbeitung und Reinigung: Das Lösungsmittel wurde abgedampft, worauf Zusatz von CHCl₃ (1,0 ml), PE (10 ml) und Wasser (10 ml) folgte. Die Phasen wurden getrennt, und die wässrige Phase wurde mit PE neuerlich extrahiert. Die Lösungsmittel wurde aus den vereinigten organischen Phasen abgedampft, und die Zielverbindung wurde mittels Flash-Säulenchromatographie (180 g Kieselgel, Durchflussgeschwindigkeit 40 ml/min, Gradient 25 auf 50 % EtOAc in PE binnen 60 min) gereinigt, um ein nahezu farbloses Öl zu erhalten (1,124 g, 94 %).
**[α]_{D}²⁵:** +23,4 (c 3,69, MeOH)

**HR-MS (ESI)** ber. für M+Na⁺: 493,2197, gef.: 493,2201.

**¹H-NMR (200 MHz, CDCl₃):** δ 1,85-1. 90 (m, 3H), 2,00 (dq, *J* = 7,2, 1,5 Hz, 3H), 2,49-2,82 (m, 3H), 2,90 (dd, *J* = 12,4, 4,2 Hz, 1H), 3,78 (dd, *J* = 8,6, 6,0 Hz, 1H), 3,86 (s, 3H), 3,87 (s, 6H), 3,88 (s, 3H), 4,08 (dd, *J* = 8,6, 6,2 Hz, 1H), 4,28 (dd, *J* = 11,3, 7,0 Hz, 1H), 4,42 (dd, *J* = 11,3, 7,0 Hz, 1H), 4,84 (d, *J* = 6,3 Hz, 1H), 6,10 (qd, *J* = 7,2, 1,3 Hz, 1H), 6,67-6,75 (m, 2H), 6,77-6,90 (m, 4H).

**¹³C-NMR (50 MHz, CDCl₃):** δ 15,9, 20,7, 33,4, 42,8, 49,4, 56,0, 56,0, 56,1, 56,1, 62,3, 72,9, 83,0, 109,2, 111,3, 111,6, 112,2, 118,2, 120,6, 127,6, 132,8, 135,2, 138,9, 147,8, 148,7, 149,2, 149,3, 167,8.

### Vergleichsbeispiel 28

### 10-23: (Z)-((2S,3R,4R)-4-(3,4-Dimethoxybenzyl)-2-(4-methoxyphenyl)tetrahydrofuran-3-yl)methyl-2-methylbut-2-enoat (3030)

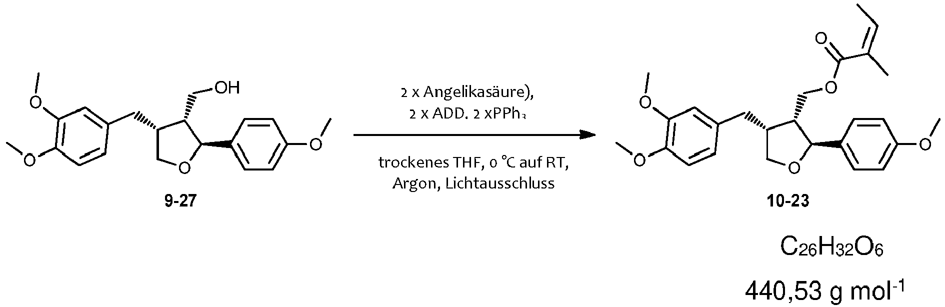

Herstellung: Ein Reaktionsgefäß wurde mit einem Rührstäbchen, Ausgangsmaterial **9-27** (20,6 mg, 0,057 mmol, 1,0 Äquiv.), Angelikasäure (8,6 mg, 0,086 mmol, 1,5 Äquiv.) und PPh₃ (52,7 mg, 0,201 mmol, 3,5 Äquiv.) beladen und dann evakuiert und mit Argon unter Anwendung eines Standard-Schlenk-Verfahrens (3x) wiederbelüftet. Trockenes THF (0,75 ml) wurde dann zugesetzt, und die Lösung wurde in einem Eisbad auf 0 °C gekühlt. Zu dem gerührten Gemisch wurde dann eine Lösung von ADD (50,7 mg, 0,201 mmol, 3,5 Äquiv.) in trockenem THF (1,0 ml) mittels Spritze binnen etwa 1 min zugesetzt, und die Reaktion wurde 46 h lang gerührt und dabei unter Lichtausschluss langsam auf Raumtemperatur erwärmen gelassen (erster Arm). Dann wurde die Reaktion neuerlich in einem Eisbad gekühlt, und es wurde mehr Angelikasäure (4,3 mg, 0,043 mmol, 0,8 Äquiv.) und PPh₃ (26,3 mg, 0,100 mmol, 1,8 Äquiv.) in trockenem THF (0,75 ml) mittels Spritze zugesetzt, gefolgt vom Zusatz von mehr ADD (25,3 mg, 0,100 mmol, 1,8 Äquiv.) in trockenem THF (1,0 ml) mittels Spritze binnen etwa 1 min, und die Reaktion wurde 24 h lang gerührt und dabei wieder unter Lichtausschluss auf Raumtemperatur erwärmen gelassen (zweiter Arm).

Aufarbeitung und Reinigung: Et₂O (5 ml) Et₂O (5 ml) wurde zu dem Reaktionsinhalt zugesetzt, der dann filtriert und mit weiterem Et₂O (15 ml) abgespült wurde. Die Lösungsmittel wurden abgedampft, und die Zielverbindung wurde mittels Flash-Säulenchromatographie (9 g Kieselgel, Durchflussgeschwindigkeit 20 ml/min, Gradient 1 auf 10 % EtOAc in Heptan binnen 10 min, dann 10 auf 70 % binnen 50 min) gereinigt, um ein farbloses Öl zu erhalten (22,6 mg, 89 %).
**[α]_{D}²⁵:** +13,5 (c 1,22, iPrOH)

**¹H-NMR (200 MHz, CDCl₃):** δ 1,84-1,90 (m, 3H), 2,00 (dd, *J* = 7,2, 1,5 Hz, 3H), 2,48-2,84 (m, 3H), 2,89 (dd, *J* = 12,6, 4,1 Hz, 1H), 3,72-3,82 (m, 1H), 3,80 (s, 3H), 3,86 (s, 3H), 3,86 (s, 3H), 4,07 (dd, *J* = 8,6, 6,1 Hz, 1H), 4,26 (dd, *J* = 11,3, 6,9 Hz, 1H), 4,41 (dd, *J* = 11,3, 7,1 Hz, 1H), 4,85 (d, *J* = 6,3 Hz, 1H), 6,08 (qd, *J* = 7,2, 1,4 Hz, 1H), 6,65-6,92 (m, 5H), 7,24 (d, *J* = 7,9 Hz, 2H).

**¹³C-NMR (50 MHz, CDCl₃):** δ 16,0, 20,7, 33,3, 42,8, 49,4, 55,4, 56,0, 56,0, 62,3, 72,8, 82,8, 111,5, 112,0, 114,0, 120,6, 127,1, 127,6, 132,8, 134,7, 138,9, 147,6, 149,1, 159,2, 167,8.

### Vergleichsbeispiel 29

### 10-24: (Z)-((2S,3R,4R)-4-(4-Methoxybenzyl)-2-phenyltetrahydrofuran-3-yl)methyl-2-methylbut-2-enoat (2859)

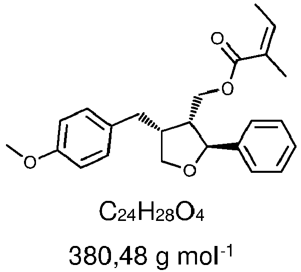

Herstellung analog zu jener von **10-23,** unter Verwendung von Ausgangsmaterial **9-40** (10,1 mg, 0,034 mmol, 1,0 Äquiv.). Erster Arm: Angelikasäure (5,1 mg, 0,051 mmol, 1,5 Äquiv.), ADD (30,0 mg, 0,119 mmol, 3,5 Äquiv.), PPh₃ (31,1 mg, 0,119 mmol, 3,5 Äquiv.), 16-stündiges Rühren. Zweiter Arm: Angelikasäure (5,1 mg, 0,051 mmol, 1,5 Äquiv.), ADD (30,0 mg, 0,119 mmol, 3,5 Äquiv.), PPh₃ (31,1 mg, 0,119 mmol, 3,5 Äquiv.), 16-stündiges Rühren.

Aufarbeitung und Reinigung: Das Lösungsmittel wurde abgedampft, und die Zielverbindung wurde mittels Flash-Säulenchromatographie (18 g Kieselgel, Durchflussgeschwindigkeit 20 ml/min, Gradient 1 auf 35 % EtOAc in PE binnen 35 min) gereinigt, um ein farbloses Öl zu erhalten (3,7 mg, 29 %).

**¹H-NMR (200 MHz, CDCl₃):** δ 1,84-1,91 (m, 3H), 2,00 (dd, *J* = 7,3, 1,5 Hz, 3H), 2,50-2,81 (m, 3H), 2,88 (dd, *J* = 12,3, 4,1 Hz, 1H), 3,74-3,83 (m, 4H), 4,09 (dd, *J* = 8,5, 6,2 Hz, 1H), 4,28 (dd, *J* = 11,3, 7,2 Hz, 1H), 4,43 (dd, *J* = 11,3, 6,8 Hz, 1H), 4,92 (d, *J* = 5,8 Hz, 1H), 6,10 (qd, *J* = 7,2, 1,4 Hz, 1H), 6,79-6,88 (m, 2H), 7,04-7,13 (m, 2H), 7,22-7,37 (m, 5H).

### Beispiel 16

### 10-25: (Z)-((2S,3R,4R)-2-Phenyl-4-(4-(trifluormethyl)benzyl)tetrahydrofuran-3-yl)-methyl-2-methylbut-2-enoat (3015)

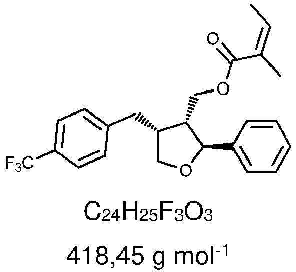

Herstellung analog zu jener von **10-23,** unter Verwendung von Ausgangsmaterial **9-41** (15,3 mg, 0,045 mmol, 1,0 Äquiv.). Erster Arm: Angelikasäure (6,8 mg, 0,068 mmol, 1,5 Äquiv.), ADD (40,1 mg, 0,159 mmol, 3,5 Äquiv.), PPh₃ (41,7 mg, 0,159 mmol, 3,5 Äquiv.), 16-stündiges Rühren. Zweiter Arm: Angelikasäure (6,8 mg, 0,068 mmol, 1,5 Äquiv.), ADD (40,1 mg, 0,159 mmol, 3,5 Äquiv.), PPh₃ (41,7 mg, 0,159 mmol, 3,5 Äquiv.), 16-stündiges Rühren.

Aufarbeitung und Reinigung: Das Lösungsmittel wurde abgedampft, und die Zielverbindung wurde mittels Flash-Säulenchromatographie (18 g Kieselgel, Durchflussgeschwindigkeit 20 ml/min, Gradient 2 auf 10 % EtOAc in PE binnen 30 min) gereinigt, um ein farbloses Öl zu erhalten (13,6 mg, 72 %).
**[α]_{D}²⁰:** +16,3 (c 0,74, iPrOH)

**HR-MS (APCI-PI)** ber. für M+H⁺: 419,1829, gef.: 419,1840.

**¹H-NMR (200 MHz, CDCl₃):** δ 1,85-1,91 (m, 3H), 2,00 (dq, *J* = 7,2, 1,5 Hz, 3H), 2,56-2,91 (m, 3H), 3,00 (d, *J* = 8,6 Hz, 1H), 3,77 (dd, *J* = 8,7, 6,1 Hz, 1H), 4,09 (dd, *J* = 8,7, 6,0 Hz, 1H), 4,29 (dd, *J* = 11,3, 6,8 Hz, 1H), 4,42 (dd, *J* = 11,4, 7,0 Hz, 1H), 4,93 (d, *J* = 5,9 Hz, 1H), 6,11 (qd, *J* = 7,2, 1,3 Hz, 1H), 7,22-7,40 (m, 7H), 7,55 (d, *J* = 8,2 Hz, 2H).

### Vergleichsbeispiel 30

### 10-26: (Z)-((2S,3R,4R)-2-(4-Fluorphenyl)-4-(4-methoxybenzyl)tetrahydrofuran-3-yl)-methyl-2-methylbut-2-enoat (3011)

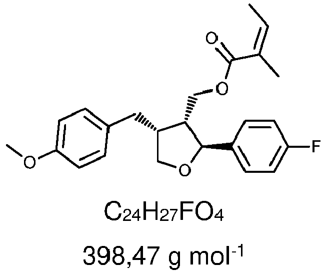

Herstellung analog zu jener von **10-23,** unter Verwendung von Ausgangsmaterial **9-25** (27,9 mg, 0,088 mmol, 1,0 Äquiv.). Erster Arm: Angelikasäure (13,2 mg, 0,132 mmol, 1,5 Äquiv.), ADD (77,9 mg, 0,309 mmol, 3,5 Äquiv.), PPh₃ (81,0 mg, 0,309 mmol, 3,5 Äquiv.), 16-stündiges Rühren. Zweiter Arm: Angelikasäure (13,2 mg, 0,132 mmol, 1,5 Äquiv.), ADD (77,9 mg, 0,309 mmol, 3,5 Äquiv.), PPh₃ (81,0 mg, 0,309 mmol, 3,5 Äquiv.), 21-stündiges Rühren.

Aufarbeitung und Reinigung: Das Lösungsmittel wurde abgedampft, und die Zielverbindung wurde mittels zweier aufeinanderfolgender Durchgänge Flash-Säulenchromatographie (erster Durchgang: 18 g Kieselgel, Durchflussgeschwindigkeit 20 ml/min, Gradient 1 bis 35 % EtOAc in PE binnen 35 min; zweiter Durchgang: 18 g Kieselgel, Durchflussgeschwindigkeit 20 ml/min, Gradient 1 auf 20 % EtOAc in PE binnen 35 min) gereinigt, um ein farbloses Öl zu erhalten (16,7 mg, 48 %).
**[α]_{D}²⁰:** +9,2 (c 0,75, iPrOH)

**HR-MS (APCI-PI)** ber. für M+H⁺: 399,1966, gef.: 399,1972.

**¹H-NMR (200 MHz, CDCl₃):** δ 1,83-1,90 (m, 3H), 1,96-2,03 (m, 3H), 2,49-2,93 (m, 4H), 3,72-3,83 (m, 4H), 4,07 (dd, *J* = 8,6, 6,2 Hz, 1H), 4,26 (dd, *J* = 11,3, 7,3 Hz, 1H), 4,41 (dd, *J* = 11,3, 6,7 Hz, 1H), 4,88 (d, *J* = 6,0 Hz, 1H), 6,10 (qd, *J* = 7,2, 1,4 Hz, 1H), 6,79-6,88 (m, 2H), 6,95-7,13 (m, 4H), 7,22-7,34 (m, 2H).

### Beispiel 17

### 10-27: (Z)-((2S,3R,4R)-4-(4-Fluorbenzyl)-2-(4-fluorphenyl)tetrahydrofuran-3-yl)-methyl-2-methylbut-2-enoat (2864)

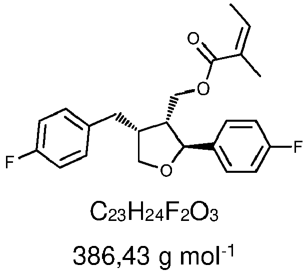

Herstellung analog zu jener von **10-23,** unter Verwendung von Ausgangsmaterial **9-26** (20,9 mg, 0,069 mmol, 1,0 Äquiv.). Erster Arm: Angelikasäure (10,3 mg, 0,103 mmol, 1,5 Äquiv.), ADD (60,7 mg, 0,241 mmol, 3,5 Äquiv.), PPh₃ (63,1 mg, 0,241 mmol, 3,5 Äquiv.), 16-stündiges Rühren. Zweiter Arm: Angelikasäure (10,3 mg, 0,103 mmol, 1,5 Äquiv.), ADD (60,7 mg, 0,241 mmol, 3,5 Äquiv.), PPh₃ (63,1 mg, 0,241 mmol, 3,5 Äquiv.), 21-stündiges Rühren.

Aufarbeitung und Reinigung: Das Lösungsmittel wurde abgedampft, und die Zielverbindung wurde mittels Flash-Säulenchromatographie (18 g Kieselgel, Durchflussgeschwindigkeit 20 ml/min, Gradient 0 auf 10 % EtOAc in PE binnen 50 min) gereinigt, um ein farbloses Öl zu erhalten (17,1 mg, 64 %).
**[α]_{D}²⁵:** +15,2 (c 1,13, iPrOH)

**HR-MS (APCI-PI)** ber. für M+H⁺: 387,1766; gef.: 387,1773.

**¹H-NMR (200 MHz, CDCl₃):** δ 1,83-1,90 (m, 3H), 1,99 (dd, *J* = 7,3, 1,5 Hz, 3H), 2,49-2,83 (m, 3H), 2,91 (dd, *J* = 12,2, 3,7 Hz, 1H), 3,75 (dd, *J* = 8,7, 6,2 Hz, 1H), 4,06 (dd, *J* = 8,7, 6,1 Hz, 1H), 4,26 (dd, *J* = 11,4, 7,1 Hz, 1H), 4,40 (dd, *J* = 11,4, 6,8 Hz, 1H), 4,88 (d, *J* = 6,1 Hz, 1H), 6,11 (qq, *J* = 7,3, 1,4 Hz, 1H), 6,92-7,18 (m, 6H), 7,23-7,34 (m, 2H).

### Beispiel 18

### 10-28: (Z)-((2S,3R,4R)-4-(4-(tert-Butyl)benzyl)-2-(4-fluorphenyl)tetrahydrofuran-3-yl)-methyl-2-methylbut-2-enoat (3010)

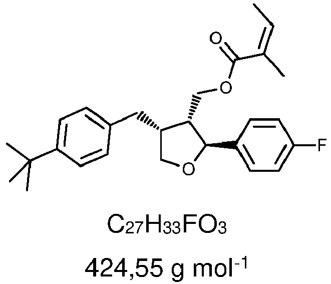

Herstellung analog zu jener von **10-23,** unter Verwendung von Ausgangsmaterial **9-42** (10,5 mg, 0,031 mmol, 1,0 Äquiv.). Erster Arm: Angelikasäure (4,6 mg, 0,046 mmol, 1,5 Äquiv.), ADD (27,1 mg, 0,108 mmol, 3,5 Äquiv.), PPh₃ (28,2 mg, 0,108 mmol, 3,5 Äquiv.), 18,5-stündiges Rühren. Zweiter Arm: Angelikasäure (4,6 mg, 0,046 mmol, 1,5 Äquiv.), ADD (27,1 mg, 0,108 mmol, 3,5 Äquiv.), PPh₃ (28,2 mg, 0,108 mmol, 3,5 Äquiv.), 47-stündiges Rühren.

Aufarbeitung und Reinigung: Das Lösungsmittel wurde abgedampft, und die Zielverbindung wurde mittels Flash-Säulenchromatographie (9 g Kieselgel, Durchflussgeschwindigkeit 20 ml/min, Gradient 2 bis 10 % EtOAc in PE binnen 40 min) gereinigt, um ein farbloses Öl zu erhalten (4,9 mg, 37 %).
**[α]_{D}²⁰:** +9,2 (c 0,69, iPrOH)

**HR-MS (APCI-PI)** ber. für M+H⁺: 425,2486; gef.: 425,2503.

**¹H-NMR (200 MHz, CDCl₃):** δ 1,83-1,90 (m, 3H), 1,31 (s, 9H), 1,99 (dd, *J* = 7,2, 1,5 Hz, 3H), 2,49-2,85 (m, 3H) , 2,90 (dd, *J* = 12,5, 4,2 Hz, 1H) , 3,78 (dd, *J* = 8,6, 6,4 Hz, 1H) , 4,09 (dd, *J* = 8,6, 6,3 Hz, 1H), 4,28 (dd, *J* = 11,3, 7,3 Hz, 1H), 4,42 (dd, *J* = 11,3, 6,7 Hz, 1H), 4,89 (d, *J* = 5,9 Hz, 1H), 5,98-6,21 (m, 1H), 6,95-7,15 (m, 4H), 7,22-7,37 (m, 4H).

### Beispiel 19

### 10-29: ((2S,3R,4R)-4-(3,4-Dimethoxybenzyl)-2-(3,4,5-trimethoxyphenyl)tetrahydrofuran-3-yl)methyl-3-methylbut-2-enoat (2755)

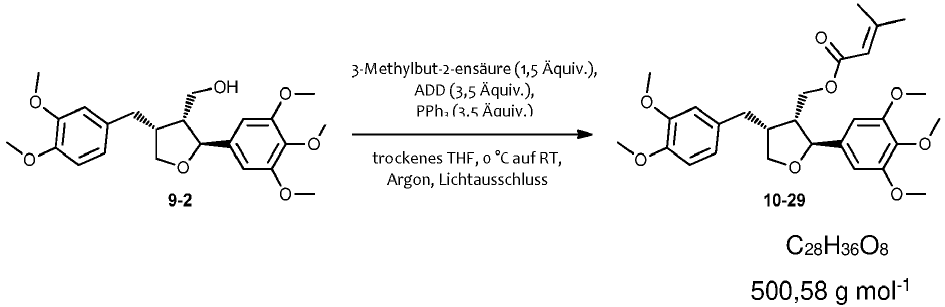

Herstellung: Ein Reaktionsgefäß wurde mit einem Rührstäbchen, Ausgangsmaterial **9-2** (21,2 mg, 0,051 mmol, 1,0 Äquiv.), 3-Methylbut-2-ensäure (7,7 mg, 0,077 mmol, 1,5 Äquiv.) und PPh₃ (46,8 mg, 0,179 mmol, 3,5 Äquiv.) beladen und dann evakuiert und mit Argon unter Anwendung eines Standard-Schlenk-Verfahrens (3x) wiederbelüftet. Trockenes THF wurde dann zugesetzt, um eine 0,13 M Lösung zu erhalten, die in einem Eisbad auf 0 °C gekühlt wurde. Zu dem gerührten Gemisch wurde dann eine Lösung von ADD (45,2 mg, 0,179 mmol, 3,5 Äquiv.) in trockenem THF mittels Spritze binnen etwa 1 min zugesetzt, und die Reaktion wurde 18,5 h lang gerührt und dabei unter Lichtausschluss langsam auf Raumtemperatur erwärmen gelassen.

Aufarbeitung und Reinigung: Zu dem Reaktionsgemisch wurde gesättigte Kochsalzlösung zugesetzt, die Phasen wurden getrennt, und die wässrige Phase wurde mit Et₂O (3x) extrahiert. Die vereinigten organischen Phasen wurden über Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 10 auf 22 % EtOAc in PE binnen 10 min, dann isokratisch bei 22 % 6 min lang, dann 22 auf 65 % binnen 30 min) gereinigt, um ein gelbliches, viskoses Öl zu erhalten (16,6 mg, 66 %).
**[α]_{D}²⁰:** +29,9 (c 1,19, MeOH)

**HR-MS (ESI)** ber. für M+Na⁺: 523,2302, gef.: 523,2310.

**¹H-NMR (200 MHz, CDCl₃):** δ 1,89 (d, *J* = 1,0 Hz, 3H), 2,18 (d, *J* = 1,2 Hz, 3H), 2,46-2,94

(m, 3H), 3,87 (dd, *J* = 12,6, 4,2 Hz, 1H), 3,74 (dd, *J* = 8,6, 6,7 Hz, 1H), 3,81 (s, 3H), 3,84 (s, 9H), 3,86 (s, 3H), 4,06 (dd, *J* = 8,6, 6,3 Hz, 1H), 4,22 (dd, *J* = 11,3, 7,2 Hz, 1H), 4,39 (dd, *J* = 11,3, 6,7 Hz, 1H), 4,79 (d, *J* = 5,9 Hz, 1H), 5,65 (m, 1H), 6,54 (s, 2H), 6,65-6,84 (m, 3H).

**¹³C-NMR (50 MHz, CDCl₃):** δ 20,3, 27,5, 33,1, 42,5, 49,1, 55,9, 56,1, 60,8, 61,7, 72,8, 83,2, 102,5, 111,3, 111,9, 115,5, 120,4, 132,6, 138,3, 147,4, 148,9, 153,2, 157,7, 166,4. Ein C_{q} nicht sichtbar.

### Vergleichsbeispiel 31

### 10-30: (Z)-((2S,3R,4R)-4-Benzyl-2-(3,4,5-trimethoxyphenyl)tetrahydrofuran-3-yl)-methyl-2-methylbut-2-enoat (2764)

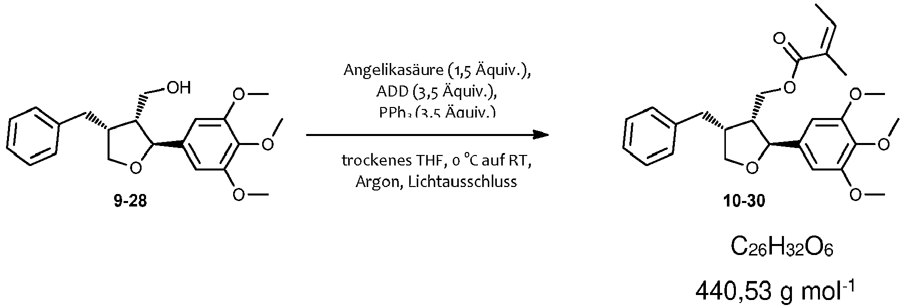

Herstellung: Ein Reaktionsgefäß wurde mit einem Rührstäbchen, Ausgangsmaterial **9-28** (16,1 mg, 0,045 mmol, 1,0 Äquiv.), Angelikasäure (6,8 mg, 0,068 mmol, 1,5 Äquiv.) und PPh₃ (41,3 mg, 0,158 mmol, 3,5 Äquiv.) beladen und dann evakuiert und mit Argon unter Anwendung eines Standard-Schlenk-Verfahrens (3x) wiederbelüftet. Trockenes THF wurde dann zugesetzt, um eine 0,13 M Lösung zu erhalten, die in einem Eisbad 0 °C gekühlt wurde. Zu dem gerührten Gemisch wurde dann eine Lösung von ADD (39,9 mg, 0,158 mmol, 3,5 Äquiv.) in trockenem THF mittels Spritze binnen etwa 1 min zugesetzt, und die Reaktion wurde 18,5 h lang gerührt und dabei unter Lichtausschluss langsam auf Raumtemperatur erwärmen gelassen. Aufarbeitung und Reinigung: Zum Reaktionsgemisch wurde gesättigte Kochsalzlösung zugesetzt, die Phasen wurden getrennt, und die wässrige Phase wurde mit Et₂O (3x) extrahiert. Die vereinigten organischen Phasen wurden über Na₂SO₄ getrocknet, filtriert und die Lösungsmittel abgedampft. Die Zielverbindung wurde mittels Flash-Säulenchromatographie (9 g Kieselgel, Durchflussgeschwindigkeit 20 ml/min, Gradient 10 auf 22 % EtOAc in PE binnen 10 min, dann isokratisch bei 22 % 6 min lang, dann 22 auf 65 % binnen 30 min) gereinigt, um ein gelbliches, viskoses Öl zu erhalten (12,1 mg, 61 %).
**[α]_{D}²⁰:** +28,1 (c 0,70, MeOH)

**¹H-NMR (400 MHz, CDCl₃):** δ 1,85-1,89 (m, 3H), 1,97-2,02 (m, 3H), 2,58-2,67 (m, 2H), 2,72-2,83 (m, 1H), 2,93 (dd, *J* = 13,3, 4,8 Hz, 1H), 3,77 (dd, *J* = 8,5, 7,0 Hz, 1H), 3,82 (s, 3H), 3,84 (s, 6H), 4,07 (dd, *J* = 8,5, 6,7 Hz, 1H), 4,30 (dd, *J* = 11,4, 7,3, 1H), 4,42 (dd, *J* = 11,4, 7,0 Hz, 1H), 4,83 (d, *J* = 6,1, 1H), 6,06-6,14 (m, 1H), 6,53 (s, 2H), 7,15-7,32 (m, 5H).

**¹³C-NMR (100 MHz, CDCl₃):** δ 15,8, 20,6, 33,5, 42,3, 49,2, 56,1, 60,8, 62,2, 72,8, 83,2, 102,5, 126,3, 127,3, 128,6, 128,6, 137,2, 138,2, 139,1, 134,0, 153,3, 167,7.

### Beispiel 20

### 10-31: (Z)-((2S,3R,4R)-4-(4-(tert-Butyl)benzyl)-2-(3,4,5-trimethoxyphenyl)tetrahydrofuran-3-yl)methyl-2-methylbut-2-enoat (2765)

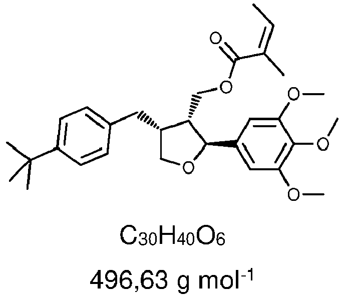

Herstellung analog zu, Aufarbeitung und Reinigung identisch mit jener von **10-30,** unter Verwendung von Ausgangsmaterial **9-29** (23,7 mg, 0,057 mmol, 1,0 Äquiv.), um ein gelbliches, viskoses Öl zu erhalten (19,1 mg, 76 %).
**[α]_{D}²⁰:** +22,4 (c 0,1,49, MeOH)

**HR-MS (ESI)** ber. für M+Na⁺: 519,2717, gef.: 519,2717.

**¹H-NMR (200 MHz, CDCl₃):** δ 1,30 (s, 9H), 1,82-1,92 (m, 3H), 1,94-2,05 (m, 3H), 2,51-2,97 (m, 4H), 3,81 (s, 4H), 3,83 (s, 6H), 4,09 (dd, *J* = 8,6, 6,3 Hz, 1H), 4,29 (dd, *J* = 11,4, 7,2 Hz, 1H), 4,43 (dd, *J* = 11,4, 6,8 Hz, 1H), 4,83 (d, *J* = 5,9 Hz, 1H), 6,02-6,17 (m, 1H), 6,54 (s, 2H), 7,05-7,14 (m, 2H), 7,27-7,36 (m, 2H).

### Vergleichsbeispiel 32

### 10-33: (Z)-((2S,3R,4R)-4-(4-(Trifluormethyl)benzyl)-2-(3,4,5-trimethoxyphenyl)tetrahydrofuran-3-yl)methyl-2-methylbut-2-enoat (2769)

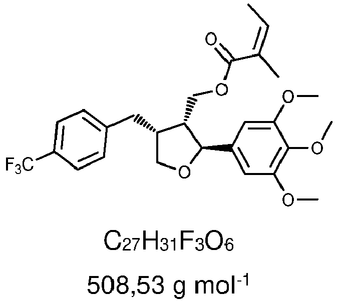

Herstellung analog zu, Aufarbeitung und Reinigung identisch mit jener von **10-30,** unter Verwendung von Ausgangsmaterial **9-32** (35,2 mg, 0,083 mmol, 1,0 Äquiv.), um ein gelbliches, viskoses Öl zu erhalten (31,2 mg, 74 %).
**[α]_{D}²⁰:** +26,2 (c 1,30, MeOH)

**HR-MS (ESI)** ber. für M+Na⁺: 531,1965, gef.: 531,1973.

**¹H-NMR (200 MHz, CDCl₃):** δ 1,85-1,88 (m, 3H), 1,97-2,02 (m, 3H), 2,59-2,83 (m, 3H), 2,99 (dd, *J* = 12,9, 4,1 Hz, 1H), 3,74 (dd, *J* = 8,6, 6,9 Hz, 1H), 3,82 (s, 3H), 3,84 (s, 6H), 4,06 (dd, *J* = 8,6, 6,39 Hz, 1H), 4,30 (dd, *J* = 11,4, 7,0 Hz, 1H), 4,41 (dd, *J* = 11,4, 7,0 Hz, 1H), 4,83 (d, *J* = 6,1 Hz, 1H), 6,07-6,15 (m, 1H), 6,53 (s, 2H), 7,29 (d, *J* = 8,1 Hz, 2H), 7,55 (d, *J* = 8,1 Hz, 2H).

### Vergleichsbeispiel 33

### 10-34: (Z)-((2S,3R,4R)-4-(4-Methoxybenzyl)-2-(3,4,5-trimethoxyphenyl)tetrahydrofuran-3-yl)methyl-2-methylbut-2-enoat (2770)

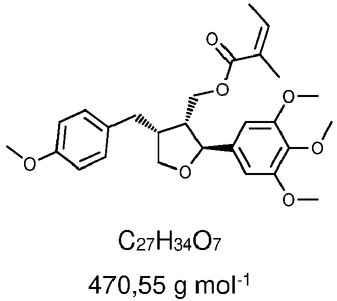

Herstellung analog zu, Aufarbeitung und Reinigung identisch mit jener von **10-30,** unter Verwendung von Ausgangsmaterial **9-30** (14,3 mg, 0,037 mmol, 1,0 Äquiv.), um ein gelbliches, viskoses Öl zu erhalten (10,1 mg, 58 %).
**[α]_{D}²⁰:** +20,1 (c 1,00, MeOH)

**¹H-NMR (200 MHz, CDCl₃):** δ 1,82-1,91 (m, 3H), 1,94-2,04 (m, 3H), 2,48-2,80 (m, 3H), 2,87 (dd, *J* = 12,4, 4,2 Hz, 1H), 3,78 (s, 4H), 3,81 (s, 3H), 3,84 (s, 6H), 4,07 (dd, *J* = 8,6, 6,3 Hz, 1H), 4,28 (dd, *J* = 11,4, 7,2 Hz, 1H), 4,42 (dd, *J* = 11,4, 6,7, 1H), 4,83 (d, *J* = 5,7, 1H), 6,10 (m, 1H), 6,53 (s, 2H), 6,8-6,9 (m, 2H), 7,03-7,13 (m, 2H).

**¹³C-NMR (50 MHz, CDCl₃):** δ 15,8, 20,6, 32,6, 42,5, 49,1, 55,2, 56,1, 60,8, 62,2, 72,8, 83,2, 102,5, 114,0, 127,3, 129,5, 131,9, 138,3, 139,1, 153,3, 158,1, 167,7. Ein C_{q} nicht sichtbar.

### Beispiel 21

### 10-35: (Z)-((2S,3R,4R)-4-(4-Acetylbenzyl)-2-(3,4,5-trimethoxyphenyl)tetrahydrofuran-3-yl)methyl-2-methylbut-2-enoat (2771)

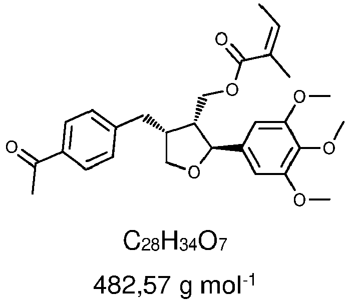

Herstellung analog zu, Aufarbeitung und Reinigung identisch mit jener von **10-30,** unter Verwendung von Ausgangsmaterial **9-36** (14,3 mg, 0,036 mmol, 1,0 Äquiv.), um ein gelbliches, viskoses Öl zu erhalten (13,1 mg, 76 %).
**[α]_{D}²⁰:** +19,7 (c 1,28, MeOH)

**¹H-NMR (200 MHz, CDCl₃):** δ 1,82-1,92 (m, 3H), 1,94-2,05 (m, 3H), 2,58 (s, 3H), 2,61-2,91 (m, 3H), 2,92-3,05 (m, 1H), 3,74 (dd, *J* = 8,8, 6,2 Hz, 1H), 3,81 (s, 3H), 3,84 (s, 6H), 4,06 (dd, *J* = 8,8, 6,1 Hz, 1H), 4,29 (dd, *J* = 11,4 & 7,0, 1H), 4,41 (dd, *J* = 11,4, 6,9 Hz, 1H), 4,83 (d, *J* = 6,0, 1H), 6,03-6,19 (m, 1H), 6,52 (s, 2H), 7,27 (d, *J* = 8,2 Hz, 2H), 7,89 (d, *J* = 8,2 Hz, 2H).

**¹³C-NMR (50 MHz, CDCl₃):** δ 15,8, 20,6, 26,5, 33,6, 42,8, 49,2, 56,1, 60,8, 62,0, 72,6, 83,1, 102,5, 128,8, 128,8, 127,2, 135,5, 136,9, 137,3, 137,9, 139,3, 153,3, 167,6, 197,6.

### Beispiel 22

### 10-38: Ethyl-4-(((3R,4R,5S)-4-((((Z)-2-methylbut-2-enoyl)oxy)methyl)-5-(3,4,5-trimethoxyphenyl)tetrahydrofuran-3-yl)methyl)benzoat (2774)

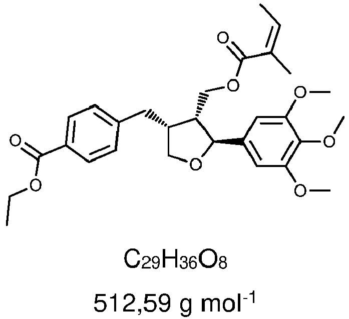

Herstellung analog zu, Aufarbeitung und Reinigung identisch mit jener von **10-30,** unter Verwendung von Ausgangsmaterial **9-37** (34,0 mg, 0,079 mmol, 1,0 Äquiv.), um ein gelbliches, viskoses Öl zu erhalten (29,8 mg, 74 %).
**[α]_{D}²⁰:** +17,7 (c 1,23, MeOH)

**¹H-NMR (400 MHz, CDCl₃):** δ 1,39 (t, *J* = 7,16 Hz, 3H), 1,85-1,91 (m, 3H), 1,98-2,04 (m, 3H), 2,59-2,85 (m, 3H), 3,00 (dd, *J* = 13,0 & 4,5 Hz, 1H), 3,75 (dd, *J* = 8,5, 6,7 Hz, 1H), 3,83 (s, 3H), 3,85 (s, 6H), 4,06 (dd, *J* = 8,5 & 6,6 Hz, 1H), 4,27-4,46 (m, 4H), 4,84 (d, *J* = 6,14, 1H), 6,08-6,16 (m, 1H), 6,54 (s, 2H), 7,25 (d, *J* = 8,5 Hz, 2H), 7,99 (d, *J* = 8,5 Hz, 2H).

**¹³C-NMR (50 MHz, CDCl₃):** δ 14,3, 15,8, 20,5, 33,6, 42,1, 49,2, 56,0, 60,8, 60,9, 62,0, 72,6, 83,0, 102,5, 127,2, 128,5, 128,7, 129,9, 137,2, 138,0, 139,2, 142,3, 153,3, 166,4, 167,6.

### Vergleichsbeispiel 34

### 10-39: (Z)-((2S,3R,4R)-4-(4-Methylbenzyl)-2-(3,4,5-trimethoxyphenyl)tetrahydrofuran-3-yl)methyl-2-methylbut-2-enoat (2775)

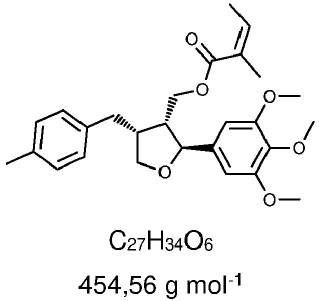

Herstellung analog zu, Aufarbeitung und Reinigung identisch mit jener von **10-30,** unter Verwendung von Ausgangsmaterial **9-33** (23,7 mg, 0,064 mmol, 1,0 Äquiv.), um ein gelbliches, viskoses Öl zu erhalten (18,6 mg, 64 %).
**[α]_{D}²⁰:** +25,3 (c 1,40, MeOH)

**¹H-NMR (200 MHz, CDCl₃):** δ 1,84-1,92 (m, 3H), 1,96-2,05 (m, 3H), 2,32 (s, 3H), 2,51-2,83 (m, 3H), 2,90 (dd, *J* = 12,3, 4,1 Hz, 1H), 3,77 (dd, *J* = 8,6, 6,5 Hz, 1H), 3,82 (s, 3H), 3,85 (s, 6H), 4,08 (dd, *J* = 8,6, 6,4 Hz, 1H), 4,30 (dd, *J* = 11,4, 7,2, 1H), 4,43 (dd, *J* = 11,4, 6,7 Hz, 1H), 4,84 (d, *J* = 5,9, 1H), 6,03-6,19 (m, 1H), 6,54 (s, 2H), 7,00-7,18 (m, 4H).

**¹³C-NMR (50 MHz, CDCl₃):** δ 15,8, 20,6, 20,9, 33,1, 42,4, 49,2, 56,1, 60,8, 62,3, 72,8, 83,18, 102,5, 127,3, 128,5, 129,3, 135,9, 136,9, 138,3, 139,0, 153,3, 167,7. Ein C_{q} nicht sichtbar.

### Beispiele 23 bis 41 und Vergleichsbeispiele 35 bis 46

In dieser Beispielsgruppe werden freie Alkohole der Formel (9) durch Steglich-Veresterung in Verbindungen der Formel (10) übergeführt.

### Vergleichsbeispiel 35

### 10-41: (E)-((2S,3R,4R)-4-(3,4-Dimethoxybenzyl)-2-(3,4-dimethoxyphenyl)tetrahydrofuran-3-yl)methyl-2-methylbut-2-enoat (2539)

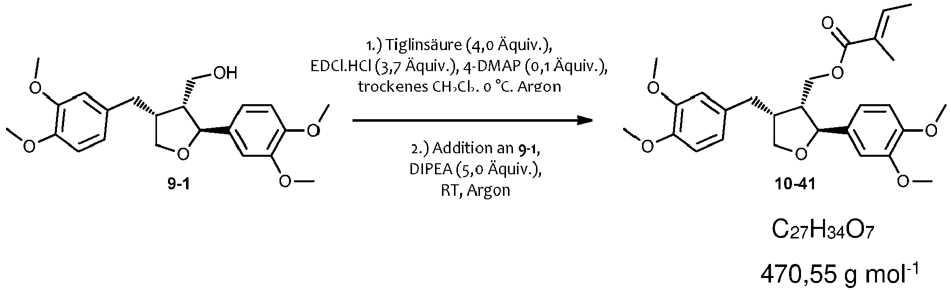

Herstellung: Ein Reaktionsgefäß wurde mit einem Rührstäbchen, Tiglinsäure (36,0 mg, 0,360 mmol, 4,0 Äquiv.) und 4-DMAP (1,1 mg, 9,0 µmol, 0,1 Äquiv.) beladen und dann evakuiert und mit Argon unter Anwendung eines Standard-Schlenk-Verfahrens (1x) wiederbelüftet. Dann wurde trockenes CH₂Cl₂ (1,0 ml) mittels Spritze zugesetzt, und die Lösung wurde in einem Eisbad auf 0 °C gekühlt. Das Gefäß wurde kurz geöffnet, EDCI.HCI (63,8 mg, 0,333 mmol, 3,7 Äquiv.) wurde auf einmal zugesetzt, und das Gemisch wurde 3 h lang bei 0 °C gerührt. Unterdessen wurde ein zweites Gefäß mit einem Rührstäbchen und **9-1** (35,0 mg, 0,090 mmol, 1,00 Äquiv.) beladen, evakuiert und mit Argon (3x) wiederbelüftet, und DIPEA (78 µl, 0,45 mmol, 5,0 Äquiv.) wurde mittels Spritze zugesetzt. Nach 3 h wurde die die aktivierte Carbonsäure enthaltende Lösung mittels Spritze in das zweite Gefäß übergeführt und 16 h lang bei Raumtemperatur gerührt.

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 50 % EtOAc in PE binnen 30 min) verwendet, um ein nahezu farbloses Öl zu erhalten (40,3 mg, 95 %).
**[α]_{D}²⁰:** +17,5 (c 2,48, MeOH)

**HR-MS (ESI)** ber. für M+Na⁺: 493,2197, gef.: 493,2204.

**¹H-NMR (200 MHz, CDCl₃):** δ 1,73-1,84 (m, 6H), 2,48-2,97 (m, 4H), 3,77 (dd, *J* = 8,6, 6,2 Hz, 1H), 3,86 (s, 3H), 3,87 (s, 6H), 3,88 (s, 3H), 4,09 (dd, *J* = 8,6, 6,2 Hz, 1H), 4,26 (dd, *J* = 11,3, 7,0 Hz, 1H), 4,43 (dd, *J* = 11,3, 6,6 Hz, 1H), 4,83 (d, *J* = 6,3 Hz, 1H), 6,67-6,88 (m, 7H).

**¹³C-NMR (50 MHz, CDCl₃):** 12,1, 14,5, 33,4, 42,8, 49,3, 56,0, 56,0, 56,0, 56,1, 62,8, 72,9, 83,3, 109,1, 111,1, 111,4, 112,0, 118,3, 120,6, 128,4, 132,8, 135,1, 137,9, 147,6, 148,6, 149,1, 149,2, 168,0.

### Vergleichsbeispiel 36

### 10-45: ((2S,3R,4R)-4-(3,4-Dimethoxybenzyl)-2-(3,4-dimethoxyphenyl)tetrahydrofuran-3-yl)methylcyclopentancarboxylat (2544)

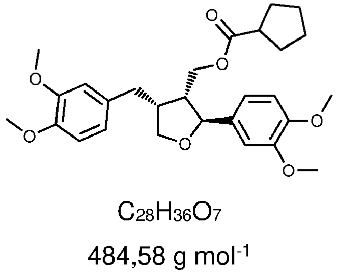

Herstellung analog zu jener von **10-41**, unter Verwendung von Ausgangsmaterial **9-1** (35,0 mg, 0,090 mmol, 1,00 Äquiv.), Cyclopentancarbonsäure (23,6 mg, 0,207 mmol, 2,3 Äquiv.), EDCI.HCI (34,5 mg, 0,180 mmol, 2,0 Äquiv.), 4-DMAP (1,1 mg, 9,0 µmol, 0,1 Äquiv.) und DIPEA (39 µl, 0,23 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 22 % EtOAc in PE binnen 9 min, dann isokratisch bei 22 % 6 min lang, dann 22 auf 62 % binnen 30 min) verwendet, um ein farbloses Öl zu erhalten (38,7 mg, 89 %).
**[α]_{D}²⁰:** +17,4 (c 2,47, MeOH)

**HR-MS (ESI)** ber. für M+Na⁺: 507,2353, gef.: 507,2358.

**¹H-NMR (200 MHz, CDCl₃):** δ 1,48-1,97 (m, 8H), 2,47-2,93 (m, 5H), 3,76 (dd, *J* = 8,5, 6,3 Hz, 1H), 3,87 (s, 3H), 3,87 (s, 6H), 3,89 (s, 3H), 4,07 (dd, *J* = 8,6, 6,2 Hz, 1H), 4,18 (dd, *J* = 11,3, 7,0 Hz, 1H), 4,37 (dd, *J* = 11,2, 6,9 Hz, 1H), 4,80 (d, *J* = 6,3 Hz, 1H), 6,66-6,91 (m, 6H).

### Vergleichsbeispiel 37

### 10-46: ((2S,3R,4R)-4-(3,4-Dimethoxybenzyl)-2-(3,4-dimethoxyphenyl)tetrahydrofuran-3-yl)methylcyclohexancarboxylat (2545)

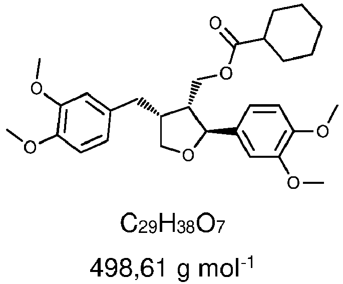

Herstellung analog zu jener von **10-41**, unter Verwendung von Ausgangsmaterial **9-1** (35,0 mg, 0,090 mmol, 1,00 Äquiv.), Cyclohexancarbonsäure (26,5 mg, 0,207 mmol, 2,3 Äquiv.), EDCI.HCI (34,5 mg, 0,180 mmol, 2,0 Äquiv.), 4-DMAP (1,1 mg, 9,0 µmol, 0,1 Äquiv.) und DIPEA (39 µl, 0,23 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 22 % EtOAc in PE binnen 9 min, dann isokratisch bei 22 % 6 min lang, dann 22 auf 62 % binnen 30 min) verwendet, um ein farbloses Öl zu erhalten (33,3 mg, 74 %).
**[α]_{D}²⁰:** +16,8 (c 1,62, MeOH)

**HR-MS (ESI)** ber. für M+Na⁺: 521,2510, gef.: 521,2516.

**¹H-NMR (200 MHz, CDCl₃):** δ 1,12-1,96 (m, 10H), 2,18-2,35 (m, 1H), 2,46-2,83 (m, 3H), 2,86 (dd, *J* = 12,4, 4,3 Hz, 1H), 3,76 (dd, *J* = 8,6, 6,2 Hz, 1H), 3,87 (s, 3H), 3,87 (s, 6H), 3,89 (s, 3H), 4,07 (dd, *J* = 8,6, 6,3 Hz, 1H), 4,17 (dd, *J=* 11,3, 6,9 Hz, 1H), 4,37 (dd, *J=* 11,3, 6,9 Hz, 1H), 4,80 (d, *J* = 6,4 Hz, 1H), 6,66-6,91 (m, 6H).

### Beispiel 23

### 10-47: (E)-((2S,3R,4R)-4-(3,4-Dimethoxybenzyl)-2-(3,4-dimethoxyphenyl)tetrahydrofuran-3-yl)methylbut-2-enoat (2549)

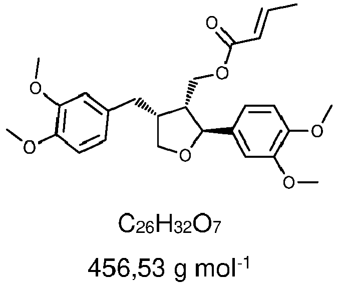

Herstellung analog zu jener von **10-41,** unter Verwendung von Ausgangsmaterial **9-1** (35,0 mg, 0,090 mmol, 1,00 Äquiv.) und Crotonsäure (31,0 mg, 0,360 mmol, 4,0 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 50 % EtOAc in PE binnen 30 min) verwendet, um ein nahezu farbloses Öl zu erhalten (32,0 mg, 78 %).
**[a]_{D}²⁰:** +24,6 (c 0,63, MeOH)

**HR-MS (ESI)** ber. für M+Na⁺: 479,2040, gef.: 479,2046.

**¹H-NMR (200 MHz, CDCl₃):** δ 1,88 (dd, *J*= 6,9, 1,5 Hz, 3H), 2,48-2,94 (m, 4H), 3,75 (dd, *J*= 8,6, 6,3 Hz, 1H), 3,86 (s, 3H), 3,87 (s, 6H), 3,88 (s, 3H), 4,08 (dd, *J*= 8,6, 6,3 Hz, 1H), 4,25 (dd, *J=* 11,3, 7,1 Hz, 1H), 4,43 (dd, *J=* 11,3, 6,9 Hz, 1H), 4,81 (d, *J* = 6,3 Hz, 1H), 5,82 (dd, *J*= 15,6, 1,6 Hz, 1H), 6,66-7,01 (m, 7H).

### Beispiel 24

### 10-52: ((2S,3R,4R)-4-(3,4-Dimethoxybenzyl)-2-(3,4-dimethoxyphenyl)tetrahydrofuran-3-yl)methylcinnamat (2739)

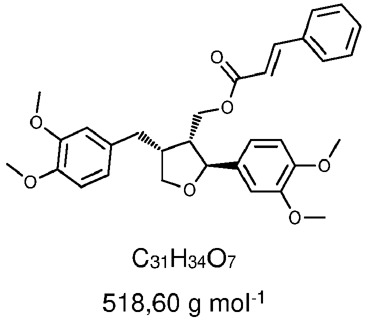

Herstellung analog zu jener von **10-41,** unter Verwendung von Ausgangsmaterial **9-1** (35,0 mg, 0,090 mmol, 1,00 Äquiv.) und Zimtsäure (53,3 mg, 0,360 mmol, 4,0 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 22 % EtOAc in PE binnen 9 min, dann isokratisch bei 22 % 6 min lang, dann 22 auf 62 % binnen 30 min) verwendet, um ein farbloses Öl zu erhalten (45,1 mg, 97 %).
**[α]_{D}²⁵:** +35,4 (c 4,24, MeOH)

**HR-MS (ESI)** ber. für M+Na⁺: 541,2197, gef.: 541,2205.

**¹H-NMR (200 MHz, CDCl₃):** δ 2,50-3,00 (m, 4H), 3,78 (dd, *J* = 8,6, 5,8 Hz, 1H), 3,83 (s, 3H), 3,85 (s, 3H), 3,86 (s, 3H), 3,88 (s, 3H), 4,11 (dd, *J* = 8,6, 6,1 Hz, 1H), 4,34 (dd, *J* = 11,3, 7,2 Hz, 1H), 4,52 (dd, *J* = 11,3, 6,6 Hz, 1H), 4,85 (d, *J* = 6,4 Hz, 1H), 6,38 (d, *J* = 16,1 Hz, 1H), 6,68-6,95 (m, 6H), 7,35-7,44 (m, 3H), 7,44-7,54 (m, 2H), 7,57 (d, *J* = 16,1 Hz, 1H).

### Beispiel 25

### 10-55: ((2S,3R,4R)-4-(3,4-Dimethoxybenzyl)-2-(3,4-dimethoxyphenyl)tetrahydrofuran-3-yl)methyl-3-methylbut-2-enoat (2738)

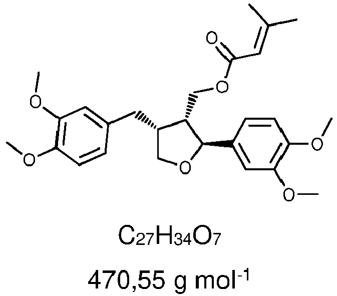

Herstellung analog zu jener von **10-41,** unter Verwendung von Ausgangsmaterial **9-1** (35,0 mg, 0,090 mmol) und 3-Methylbut-2-ensäure (36,0 mg, 0,360 mmol).

Aufarbeitung, Nachbehandlung und Reinigung: Die Reaktionslösung wurde direkt für die Flash-Säulenchromatographie (9 g Kieselgel, Durchflussgeschwindigkeit 20 ml/ min, Gradient 10 auf 22 % EtOAc in PE binnen 9 min, dann isokratisch bei 22 % 6 min lang, dann 22 auf 62 % binnen 30 min) verwendet, um ein Gemisch aus Ziel- und isomerisierter Verbindung (34,4 mg, das entsprechende 3-Methylbut-3-enoat) zu erhalten. Somit wurde ein neues Reaktionsgefäß mit einem Rührstäbchen und einem Teil des derart erhaltenen Materials (24,7 mg, 0,052 mmol) beladen, evakuiert und mit Argon wiederbelüftet. Hierzu wurde dann *tert*-BuOK (2,9 mg, 0,026 mmol) in trockenem THF (1,0 ml) mittels Spritze zugesetzt, und die Lösung wurde bei Raumtemperatur 18 h lang gerührt. Dann wurde das Gefäß geöffnet, THF (1,0 ml), gefolgt von Et₂O (15 ml) und einer Lösung von KHSO₄ (0,029 mmol, 3,9 mg) in gesättigter Kochsalzlösung (2 ml), wurde zugesetzt. Um die Salze zu lösen, wurde Wasser (1,5 ml) zugesetzt, die Phasen wurden getrennt, die wässrige Phase mit Et₂O (2 × 10 ml) neuerlich extrahiert, die vereinigten organischen Phasen wurden über Na₂SO₄ getrocknet und die Lösungsmittel abgedampft. Schließlich wurde die Zielverbindung mittels Flash-Säulenchromatographie (9 g Kieselgel, Durchflussgeschwindigkeit 20 ml/min, Gradient 10 auf 50 % EtOAc in PE binnen 30 min) gewonnen, um ein farbloses Öl zu erhalten (17,0 mg, 40 % bezogen auf das Ausgangsmaterial **9-1, 56** % bezogen auf das in der Nachbehandlung eingesetzte Gemisch).
**[a]_{D}²⁰:** +29,2 (c 1,63, MeOH)

**HR-MS (ESI)** ber. für M+Na⁺: 493,2197, gef.: 493,2201.

**¹H-NMR (200 MHz, CDCl₃):** δ 1,90 (d, *J* = 1,0 Hz, 3H), 2,17 (d, *J* = 1,1 Hz, 3H), 2,47-2,84 (m, 3H), 2,89 (dd, *J* = 12,7, 4,3 Hz, 1H), 3,75 (dd, *J*= 8,6, 6,3 Hz, 1H), 3,86 (s, 3H), 3,87 (s, 6H), 3,88 (s, 3H), 4,07 (dd, *J* = 8,4, 6,1 Hz, 1H), 4,21 (dd, *J =* 11,4, 7,0 Hz, 1H), 4,37 (dd, *J* = 11,3, 7,0 Hz, 1H), 4,81 (d, *J* = 6,3 Hz, 1H), 5,63-5,68 (m, 1H), 6,66-6,81 (m, 3H), 6,81-6,92 (m, 3H).

### Beispiel 26

### 10-56: ((2S,3R,4R)-4-(3,4-Dimethoxybenzyl)-2-phenyltetrahydrofuran-3-yl)methyl-cyclopentancarboxylat (2742)

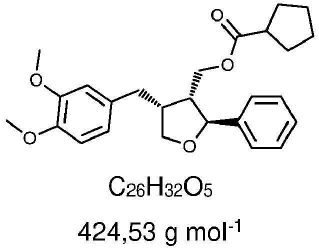

Herstellung analog zu jener von **10-41,** unter Verwendung von Ausgangsmaterial **9-39** (20,0 mg, 0,061 mmol, 1,00 Äquiv.), Cyclopentancarbonsäure (27,8 mg, 0,244 mmol, 4,0 Äquiv.), EDCl.HCl (23,3 mg, 0,122 mmol, 2,0 Äquiv.), 4-DMAP (0,7 mg, 6,1 µmol, 0,1 Äquiv.) und DIPEA (25 µl, 0,15 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 15 % EtOAc in PE binnen 10 min, dann 15 auf 22 % binnen 5 min, dann 22 auf 60 % binnen 15 min) verwendet, um ein farbloses Öl zu erhalten (22,3 mg, 86 %).
**[α]_{D}²⁵:** +16,7 (c 2,20, MeOH)

**HR-MS (ESI)** ber. für M+Na⁺: 447,2142, gef.: 447,2148.

**¹H-NMR (200 MHz, CDCl₃):** δ 1,47-1,99 (m, 8H), 2,48-2,91 (m, 5H), 3,78 (dd, *J*= 8,5, 6,4 Hz, 1H), 3,86 (s, 3H), 3,87 (s, 3H), 4,09 (dd, *J* = 8,6, 6,3 Hz, 1H), 4,19 (dd, *J =* 11,2, 7,2 Hz, 1H), 4,39 (dd, *J* = 11,2, 6,7 Hz, 1H), 4,89 (d, *J* = 5,9 Hz, 1H), 6,65-6,75 (m, 2H), 6,80 (d, *J* = 7,9 Hz, 1H), 7,40-7,21 (m, 5H).

### Beispiel 27

### 10-59: ((2S,3R,4R)-4-(3,4-Dimethoxybenzyl)-2-(3,4-dimethoxyphenyl)tetrahydrofuran-3-yl)methyl-2-(adamantan-1-yl)acetat (2745)

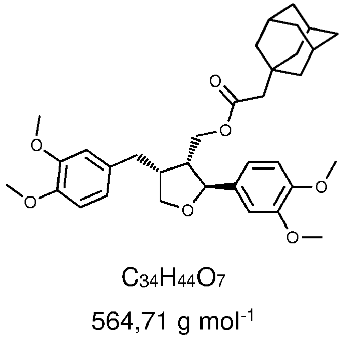

Herstellung analog zu jener von **10-41,** unter Verwendung von Ausgangsmaterial **9-1** (35,0 mg, 0,090 mmol, 1,00 Äquiv.), 2-(Adamantan-1-yl)essigsäure (40,2 mg, 0,207 mmol, 2,3 Äquiv.), EDCI.HCI (34,5 mg, 0,180 mmol, 2,0 Äquiv.), 4-DMAP (1,1 mg, 9,0 µmol, 0,1 Äquiv.) und DIPEA (39 µl, 0,23 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 15 % EtOAc in PE binnen 10 min, dann 15 auf 25 % binnen 5 min, dann 25 auf 48 % binnen 7 min) verwendet, um ein farbloses Öl zu erhalten (40,5 mg, 80 %).
**[α]_{D}²⁵:** +18,3 (c 4,05, MeOH)

**HR-MS (ESI)** ber. für M+Na⁺: 587,2979, gef.: 587,3000.

**¹H-NMR (200 MHz, CDCl₃):** δ 1,52-1,77 (m, 12H), 1,89-2,03 (m, 3H), 2,07 (s, 2H), 2,46-2,64 (m, 2H), 2,64-2,81 (m, 1H), 2,89 (dd, *J* = 12,7, 4,2 Hz, 1H), 3,76 (dd, *J =* 8,6, 6,2 Hz, 1H), 3,86 (s, 3H), 3,87 (s, 6H), 3,89 (s, 3H), 4,06 (dd, *J*= 8,6, 6,3 Hz, 1H), 4,16 (dd, *J =* 11,3, 7,0 Hz, 1H), 4,36 (dd, *J* = 11,2, 7,1 Hz, 1H), 4,81 (d, *J* = 6,3 Hz, 1H), 6,65-6,91 (m, 6H).

### Beispiel 28

### 10-60: ((2S,3R,4R)-4-(3,4-Dimethoxybenzyl)-2-phenyltetrahydrofuran-3-yl)methyl-cyclohexancarboxylat (2746)

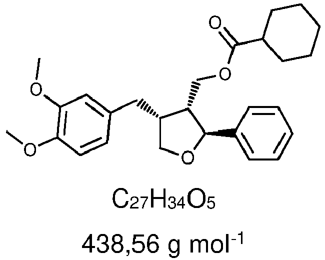

Herstellung analog zu jener von **10-41,** unter Verwendung von Ausgangsmaterial **9-39** (19,4 mg, 0,059 mmol, 1,00 Äquiv.), Cyclohexancarbonsäure (30,3 mg, 0,236 mmol, 4,0 Äquiv.), EDCl.HCl (22,7 mg, 0,118 mmol, 2,0 Äquiv.), 4-DMAP (0,7 mg, 5,9 µmol, 0,1 Äquiv.) und DIPEA (25 µl, 0,15 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 12 % EtOAc in PE binnen 10 min, dann 12 auf 19 % binnen 5 min, dann 19 auf 57 % binnen 15 min) verwendet, um ein farbloses Öl zu erhalten (21,7 mg, 84 %).
**[α]_{D}²⁵:** +17,9 (c 2,10, MeOH)

**HR-MS (ESI)** ber. für M+Na⁺: 461,2298, gef.: 461,2302.

**¹H-NMR (200 MHz, CDCl₃):** δ 1,15-1,96 (m, 10H), 2,18-2,35 (m, 1H), 2,47-2,91 (m, 4H), 3,78 (dd, *J*= 8,5, 6,3 Hz, 1H), 3,86 (s, 6H), 4,09 (dd, *J*= 8,5, 6,2 Hz, 1H), 4,18 (dd, *J* = 11,2, 7,2 Hz, 1H), 4,39 (dd, *J* = 11,2, 6,8 Hz, 1H), 4,88 (d, *J* = 6,0 Hz, 1H), 6,65-6,75 (m, 2H), 6,80 (d, *J* = 7,9 Hz, 1H), 7,22-7,40 (m, 5H).

### Beispiel 29

### 10-62: ((2S,3R,4R)-4-(3,4-Dimethoxybenzyl)-2-(4-fluorphenyl)tetrahydrofuran-3-yl)-methylcyclopentancarboxylat (2781)

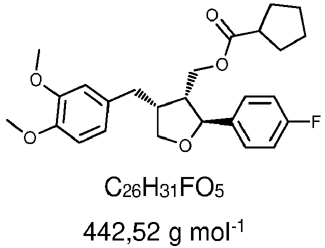

Herstellung analog zu jener von **10-41,** unter Verwendung von Ausgangsmaterial **9-24** (31,2 mg, 0,090 mmol, 1,00 Äquiv.), Cyclopentancarbonsäure (23,6 mg, 0,207 mmol, 2,3 Äquiv.), EDCl.HCl (34,5 mg, 0,180 mmol, 2,0 Äquiv.), 4-DMAP (1,1 mg, 9,0 µmol, 0,1 Äquiv.) und DIPEA (39 µl, 0,23 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 0 auf 15 % EtOAc in PE binnen 16 min, dann 15 auf 22 % binnen 4 min), um ein farbloses Öl zu erhalten (38,2 mg, 96 %).
**[α]_{D}²⁰:** +13,8 (c 2,46, MeOH)

**HR-MS (ESI)** ber. für M+Na⁺: 465,2048, gef.: 465,2024.

**¹H-NMR (200 MHz, CDCl₃):** δ 1,47-1,98 (m, 8H), 2,44-2,79 (m, 4H), 2,85 (dd, *J* = 12,4, 4,3 Hz, 1H), 3,76 (dd, *J*= 8,5, 6,4 Hz, 1H), 3,86 (s, 3H), 3,87 (s, 3H), 4,07 (dd, *J* = 8,6, 6,2 Hz, 1H), 4,18 (dd, *J* = 11,2, 7,4 Hz, 1H), 4,38 (dd, *J* = 11,2, 6,6 Hz, 1H), 4,85 (d, *J* = 6,0 Hz, 1H), 6,65-6,75 (m, 2H), 6,80 (d, *J* = 7,9 Hz, 1H), 7,02 (t, *J=* 8,7 Hz, 2H), 7,29 (dd, *J*= 8,6, 5,4 Hz, 2H).

### Vergleichsbeispiel 38

### 10-63: ((2S,3R,4R)-4-(3,4-Dimethoxybenzyl)-2-(3,4-dimethoxyphenyl)tetrahydrofuran-3-yl)methyl-2-naphthoat (2784)

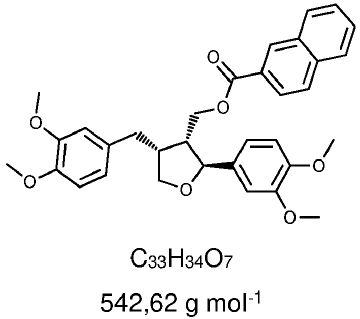

Herstellung analog zu jener von **10-41,** unter Verwendung von Ausgangsmaterial **9-1** (35,0 mg, 0,090 mmol, 1,00 Äquiv.) und 2-Naphthoesäure (62,0 mg, 0,360 mmol, 4,0 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 8 auf 50 % EtOAc in PE binnen 30 min) verwendet, um ein farbloses Öl zu erhalten (43,7 mg, 90 %).
**[α]_{D}²⁰:** +17,6 (c 2,38, MeOH)

**HR-MS (APCI-PI)** ber. für M+Na⁺: 565,2197, gef.: 565,2211.

**¹H-NMR (200 MHz, CDCl₃):** δ 2,58-3,04 (m, 4H), 3,81 (s, 3H), 3,81-3,90 (m, 1H), 3,83 (s, 3H), 3,84 (s, 3H), 3,84 (s, 3H), 4,15 (dd, *J*= 8,5, 6,0 Hz, 1H), 4,52 (dd, *J =* 11,2, 7,3 Hz, 1H), 4,71 (dd, *J* = 11,2, 6,0 Hz, 1H), 4,94 (d, *J* = 6,2 Hz, 1H), 6,77 (m, 4H), 6,87-6,98 (m, 2H), 7,49-7,65 (m, 2H), 7,80-7,98 (m, 4H), 8,40 (s, 1H).

### Vergleichsbeispiel 39

### 10-66: ((2S,3R,4R)-4-(4-Methylbenzyl)-2-(3,4,5-trimethoxyphenyl)tetrahydrofuran-3-yl)methylcyclopentancarboxylat (2866)

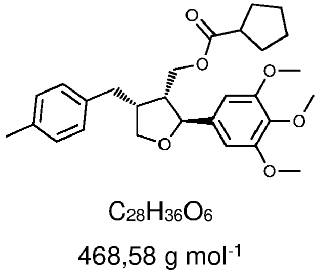

Herstellung analog zu jener von **10-41,** unter Verwendung von Ausgangsmaterial **9-33** (10,4 mg, 0,028 mmol, 1,00 Äquiv.), Cyclopentancarbonsäure (7,3 mg, 0,064 mmol, 2,3 Äquiv.), EDCl.HCl (10,7 mg, 0,056 mmol, 2,0 Äquiv.), 4-DMAP (0,3 mg, 2,8 µmol, 0,1 Äquiv.) und DIPEA (12 µl, 0,07 mmol, 2,5 Äquiv.), und 24- statt 16-stündigem Rühren der Reaktion.

Aufarbeitung und Reinigung: Die Reaktionslösung wurde direkt für die Flash-Säulenchromatographie (9 g Kieselgel, Durchflussgeschwindigkeit 20 ml/min, isokratisch bei 10 % EtOAc in PE, dann Gradient 10 auf 15 % binnen 10 min, dann 15 auf 25 % binnen 5 min, dann 25 auf 50 % binnen 7 min) verwendet, um ein farbloses Öl zu erhalten (7,2 mg, 55 %).

**¹H-NMR (200 MHz, CDCl₃):** δ 1,48-1,98 (m, 8H), 2,33 (s, 3H), 2,48-2,94 (m, 5H), 3,75 (dd, *J*= 8,5, 6,6 Hz, 1H), 3,83 (s, 3H), 3,86 (s, 6H), 4,07 (dd, *J*= 8,6, 6,3 Hz, 1H), 4,20 (dd, *J* = 11,2, 7,0 Hz, 1H), 4,38 (dd, *J* = 11,3, 6,7 Hz, 1H), 4,79 (d, *J* = 5,9 Hz, 1H), 6,54 (s, 2H), 7,03-7,16 (m, 4H).

**¹³C-NMR (50 MHz, CDCl₃):** δ 21,1, 25,9, 30,1, 33,2, 42,5, 44,0, 49,3, 56,3, 61,0, 62,7, 73,0, 83,3, 102,7, 128,6, 129,4, 136,0, 137,1, 137,4, 138,4, 153,4, 176,7.

### Beispiel 30

### 10-67: ((2S,3R,4R)-4-(4-(tert-Butyl)benzyl)-2-(3,4-dimethoxyphenyl)tetrahydrofuran-3-yl)methylcyclopentancarboxylat (2867)

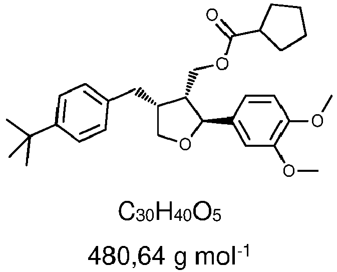

Herstellung analog zu jener von **10-41,** unter Verwendung von Ausgangsmaterial **9-5** (16,7 mg, 0,043 mmol, 1,00 Äquiv.), Cyclopentancarbonsäure (11,4 mg, 0,100 mmol, 2,3 Äquiv.), EDCl.HCl (16,6 mg, 0,087 mmol, 2,0 Äquiv.), 4-DMAP (0,5 mg, 4,3 µmol, 0,1 Äquiv.) und DIPEA (19 µl, 0,11 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, isokratisch 3 % EtOAc in PE 3 min lang, dann Gradient 3 auf 30 % binnen 30 min) verwendet, um ein farbloses Öl zu erhalten (16,6 mg, 79 %).
**[α]_{D}²⁵:** +14,2 (c 1,07, *i*PrOH)

**HR-MS (APCI-PI)** ber. für M+Na⁺: 503,2768, gef.: 503,2751.

**¹H-NMR (200 MHz, CDCl₃):** δ 1,31 (s, 9H), 1,48-1,96 (m, 8H), 2,48-2,96 (m, 5H), 3,75 (dd, *J*= 8,5, 6,5 Hz, 1H), 3,87 (s, 3H), 3,89 (s, 3H), 4,08 (dd, *J*= 8,6, 6,4 Hz, 1H), 4,19 (dd, *J* = 11,3, 6,9 Hz, 1H), 4,36 (dd, *J* = 11,2, 6,9 Hz, 1H), 4,80 (d, *J* = 6,3 Hz, 1H), 6,78-6,92 (m, 3H), 7,10 (d, *J* = 8,3 Hz, 2H), 7,32 (d, *J* = 8,3 Hz, 2H).

### Vergleichsbeispiel 40

### 10-69: ((2S,3R,4R)-2-(3,4-Dimethoxyphenyl)-4-(4-(trifluormethyl)benzyl)tetrahydrofuran-3-yl)methyl-2-methylbenzoat (3027)

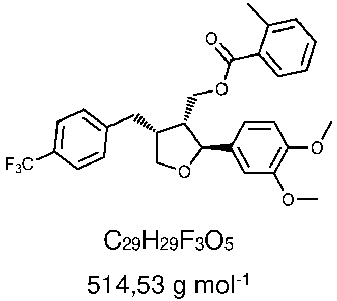

Herstellung analog zu jener von **10-41,** unter Verwendung von Ausgangsmaterial **9-17** (33,6 mg, 0,085 mmol, 1,00 Äquiv.) und 2-Methylbenzoesäure (46,1 mg, 0,339 mmol, 4,0 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 5 auf 40 % EtOAc in Heptan binnen 40 min) verwendet, um ein farbloses Öl zu erhalten (27,6 mg, 63 %).
**[α]_{D}²⁵:** +17,0 (c 0,83, iPrOH)

**¹H-NMR (200 MHz, CDCl₃):** δ 2,59 (s, 3H), 2,65-2,96 (m, 3H), 3,05 (dd, *J*= 12,5, 3,6 Hz, 1H), 3,77 (dd, *J* = 8,8, 5,8 Hz, 1H), 3,83 (s, 3H), 3,86 (s, 3H), 4,11 (dd, *J*= 8,8, 6,0 Hz, 1H), 4,43 (dd, *J=* 11,3, 6,9 Hz, 1H), 4,58 (dd, *J=* 11,3, 6,8 Hz, 1H), 4,91 (d, *J* = 6,3 Hz, 1H), 6,78-6,93 (m, 3H), 7,15-7,35 (m, 4H), 7,41 (td, *J*= 7,4, 1,5 Hz, 1H), 7,55 (d, *J* = 8,1 Hz, 2H), 7,76 (dd, *J*= 7,9, 1,4 Hz, 1H).

**¹³C-NMR (50 MHz, CDCl₃):** δ 21,9, 33,7, 42,4, 49,4, 56,0, 56,1, 62,9, 72,7, 83,3, 109,1, 111,2, 118,3, 125,7 (q, J = 3,7 Hz), 125,9, 128,9 (q, J = 32,5 Hz), 129,1, 130,6, 132,0, 132,4, 134,7, 140,6, 144,3, 148,7, 149,3, 167,3. Zwei C_{q} nicht sichtbar.

### Vergleichsbeispiel 41

### 10-70: ((2S,3R,4R)-4-(3,4-Dimethoxybenzyl)-2-(3,4-dimethoxyphenyl)tetrahydrofuran-3-yl)methyl-3-methylbutanoat (2854)

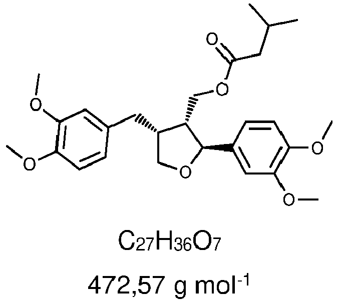

Herstellung analog zu jener von **10-41,** unter Verwendung von Ausgangsmaterial **9-1** (22,8 mg, 0,059 mmol, 1,00 Äquiv.) und 3-Methylbutansäure (13,8 mg, 0,135 mmol, 2,3 Äquiv.), EDCI.HCI (22,5 mg, 0,117 mmol, 2,0 Äquiv.), 4-DMAP (0,7 mg, 5,9 µmol, 0,1 Äquiv.) und DIPEA (26 µl, 0,15 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 30 % EtOAc in PE binnen 30 min) verwendet, um ein farbloses Öl zu erhalten (22,0 mg, 96 %).
**[α]_{D}²³:** +22,9 (c 0,90, MeOH)

**HR-MS (APCI-PI)** ber. für M+Na⁺: 495,2353, gef.: 495,2371.

**¹H-NMR (200 MHz, CDCl₃):** δ 0,96 (d, *J* = 6,4 Hz, 6H), 1,97-2,22 (m, 3H), 2,47-2,64 (m, 2H), 2,64-2,81 (m, 1H), 2,87 (dd, *J*= 12,5, 4,3 Hz, 1H), 3,75 (dd, *J*= 8,6, 6,2 Hz, 1H), 3,86 (s, 3H), 3,87 (s, 6H), 3,89 (s, 3H), 4,07 (dd, *J*= 8,6, 6,3 Hz, 1H), 4,18 (dd, *J* = 11,3, 7,1 Hz, 1H), 4,38 (dd, *J* = 11,2, 7,0 Hz, 1H), 4,79 (d, *J* = 6,4 Hz, 1H), 6,66-6,90 (m, 6H).

### Vergleichsbeispiel 42

### 10-71: ((2S,3R,4R)-4-(3,4-Dimethoxybenzyl)-2-(3,4-dimethoxyphenyl)tetrahydrofuran-3-yl)methyl-3,3-dimethylbutanoat (2855)

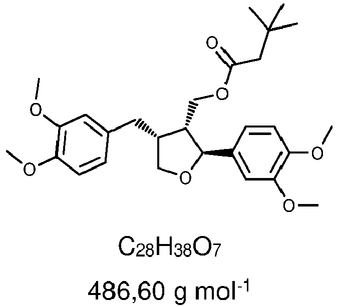

Herstellung analog zu jener von **10-41,** unter Verwendung von Ausgangsmaterial **9-1** (30,0 mg, 0,077 mmol, 1,00 Äquiv.) und 3,3-Dimethylbutansäure (20,6 mg, 0,178 mmol, 2,3 Äquiv.), EDCl.HCl (29,6 mg, 0,154 mmol, 2,0 Äquiv.), 4-DMAP (0,9 mg, 7,7 µmol, 0,1 Äquiv.) und DIPEA (34 µl, 0,19 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 30 % EtOAc in PE binnen 30 min) verwendet, um ein farbloses Öl zu erhalten (19,5 mg, 51 %).
**[α]_{D}²³:** +24,7 (c 1,01, MeOH)

**HR-MS (APCI-PI)** ber. für M+Na⁺: 509,2510, gef.: 509,2512.

**¹H-NMR (200 MHz, CDCl₃):** δ 1,03 (d, *J* = 5,5 Hz, 9H), 2,20 (s, 2H), 2,47-2,63 (m, 2H), 2,64-2,82 (m, 1H), 2,88 (dd, *J* = 12,5, 4,2 Hz, 1H), 3,76 (dd, *J* = 8,6, 6,1 Hz, 1H), 3,86 (s, 3H), 3,87 (s, 3H), 3,87 (s, 3H), 3,89 (s, 3H), 4,06 (dd, *J* = 8,6, 6,2 Hz, 1H), 4,16 (dd, *J* = 11,3, 6,9 Hz, 1H), 4,36 (dd, *J* = 11,3, 7,2 Hz, 1H), 4,80 (d, *J* = 6,4 Hz, 1H), 6,66-6,90 (m, 6H).

### Beispiel 31

### 10-72: ((2S,3R,4R)-4-(3,4-Dimethoxybenzyl)-2-(4-fluorphenyl)tetrahydrofuran-3-yl)-methyl-2-ethylbutanoat (2856)

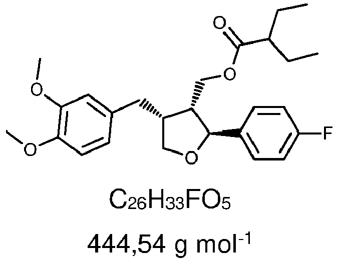

Herstellung analog zu jener von **10-41,** unter Verwendung von Ausgangsmaterial **9-24** (15,2 mg, 0,044 mmol, 1,00 Äquiv.) und 2-Ethylbutansäure (11,7 mg, 0,101 mmol, 2,3 Äquiv.), EDCl.HCl (16,8 mg, 0,088 mmol, 2,0 Äquiv.), 4-DMAP (0,5 mg, 4,4 µmol, 0,1 Äquiv.) und DIPEA (19 µl, 0,110 mmol, 2,5 Äquiv.) und 13- statt 16-stündigem Rühren.

Aufarbeitung und Reinigung: Die Reaktionslösung wurde direkt für die Flash-Säulenchromatographie (9 g Kieselgel, Durchflussgeschwindigkeit 20 ml/min, Gradient 0 auf 10 % EtOAc in PE binnen 60 min) verwendet, um ein farbloses Öl zu erhalten (16,2 mg, 83 %).
**[α]_{D}²³:** +14,2 (c 0,65, MeOH)

**HR-MS (APCI-PI)** ber. für M+Na⁺: 467,2204, gef.: 467,2197.

**¹H-NMR (200 MHz, CDCl₃):** δ 0,89 (t, *J* = 7,4 Hz, 3H), 0,90 (t, *J* = 7,4 Hz, 3H), 1,44-1,70 (m, 4H), 2,14-2,29 (m, 1H), 2,45-2,61 (m, 2H), 2,62-2,81 (m, 1H), 2,87 (dd, *J* = 12,5, 4,1 Hz, 1H), 3,77 (dd, *J*= 8,6, 6,3 Hz, 1H), 3,86 (s, 6H), 4,06 (dd, *J*= 8,6, 6,2 Hz, 1H), 4,17 (dd, *J =* 11,2, 7,2 Hz, 1H), 4,41 (dd, *J=* 11,3, 6,7 Hz, 1H), 4,85 (d, *J* = 6,1 Hz, 1H), 6,65-6,84 (m, 3H), 6,97-7,09 (m, 2H), 7,24-7,34 (m, 2H).

### Vergleichsbeispiel 43

### 10-75: ((2S,3R,4R)-4-(3,4-Dimethoxybenzyl)-2-(4-fluorphenyl)tetrahydrofuran-3-yl)-methyl-3,3-dimethylbutanoat (2861)

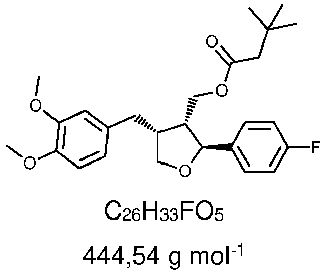

Herstellung analog zu jener von **10-41,** unter Verwendung von Ausgangsmaterial **9-24** (22,0 mg, 0,064 mmol, 1,00 Äquiv.) und 3,3-Dimethylbutansäure (16,9 mg, 0,146 mmol, 2,3 Äquiv.), EDCl.HCl (24,3 mg, 0,127 mmol, 2,0 Äquiv.), 4-DMAP (0,8 mg, 6,4 µmol, 0,1 Äquiv.) und DIPEA (28 µl, 0,16 mmol, 2,5 Äquiv.), und 13,5- statt 16-stündigem Rühren.

Aufarbeitung und Reinigung: Die Reaktionslösung wurde direkt für die Flash-Säulenchromatographie (9 g Kieselgel, Durchflussgeschwindigkeit 20 ml/min, Gradient 9 auf 25 % EtOAc in PE binnen 60 min) verwendet, um ein farbloses Öl zu erhalten (25,8 mg, 92%).
**[α]_{D}²³:** +15,7 (c 2,07, MeOH)

**HR-MS (APCI-PI)** ber. für M+H⁺: 445,2385, gef.: 445,2398.

**¹H-NMR (200 MHz, CDCl₃):** δ 1,03 (s, 9H), 2,19 (s, 2H), 2,43-2,62 (m, 2H), 2,62-2,82 (m, 1H), 2,86 (dd, *J*= 12,5, 4,2 Hz, 1H), 3,77 (dd, *J*= 8,6, 6,3 Hz, 1H), 3,86 (s, 3H), 3,86 (s, 3H), 4,06 (dd, *J* = 8,6, 6,2 Hz, 1H), 4,16 (dd, *J* = 11,1, 7,2 Hz, 1H), 4,37 (dd, *J* = 11,3, 6,9 Hz, 1H), 4,85 (d, *J* = 6,1 Hz, 1H), 6,64-6,84 (m, 3H), 6,96-7,09 (m, 2H), 7,23-7,34 (m, 2H).

### Vergleichsbeispiel 44

### 10-76: ((2S,3R,4R)-4-(3,4-Dimethoxybenzyl)-2-(3,4-dimethoxyphenyl)tetrahydrofuran-3-yl)methyl-2-ethylbutanoat (2860)

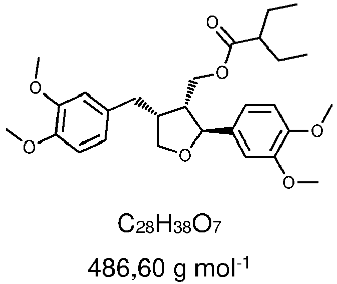

Herstellung analog zu jener von **10-41,** unter Verwendung von Ausgangsmaterial **9-1** (20,3 mg, 0,052 mmol, 1,00 Äquiv.) und 2-Ethylbutansäure (14,0 mg, 0,120 mmol, 2,3 Äquiv.), EDCI.HCI (20,0 mg, 0,105 mmol, 2,0 Äquiv.), 4-DMAP (0,6 mg, 5,2 µmol, 0,1 Äquiv.) und DIPEA (23 µl, 0,13 mmol, 2,5 Äquiv.).

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 30 % EtOAc in PE binnen 30 min) verwendet, um ein farbloses Öl zu erhalten (22,2 mg, 87 %).
**[α]_{D}²³:** +23,3 (c 0,88, MeOH)

**HR-MS (APCI-PI)** ber. für M+Na⁺: 509,2510, gef.: 509,2533.

**¹H-NMR (200 MHz, CDCl₃):** δ 0,90 (t, *J* = 7,4 Hz, 6H), 1,42-1,75 (m, 4H), 2,15-2,30 (m, 1H), 2,47-2,82 (m, 3H), 2,88 (dd, *J*= 12,6, 4,0 Hz, 1H), 3,76 (dd, *J*= 8,6, 6,1 Hz, 1H), 3,87 (s, 6H), 3,87 (s, 3H), 3,89 (s, 3H), 4,06 (dd, *J*= 8,6, 6,2 Hz, 1H), 4,18 (dd, *J* = 11,3, 6,8 Hz, 1H), 4,41 (dd, *J=* 11,3, 7,1 Hz, 1H), 4,81 (d, *J* = 6,4 Hz, 1H), 6,64-6,91 (m, 6H).

### Beispiel 32

### 10-77: ((2S,3R,4R)-4-(3,4-Dimethoxybenzyl)-2-(4-fluorphenyl)tetrahydrofuran-3-yl)-methyl-3-methylbutanoat (2862)

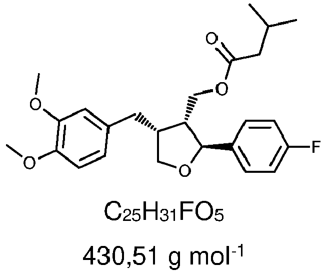

Herstellung analog zu jener von **10-41,** unter Verwendung von Ausgangsmaterial **9-24** (23,7 mg, 0,068 mmol, 1,00 Äquiv.) und 3-Methylbutansäure (16,1 mg, 0,157 mmol, 2,3 Äquiv.), EDCl.HCl (26,2 mg, 0,137 mmol, 2,0 Äquiv.), 4-DMAP (0,8 mg, 6,8 µmol, 0,1 Äquiv.) und DIPEA (30 µl, 0,17 mmol, 2,5 Äquiv.), und 24- statt 16-stündigem Rühren.

Aufarbeitung und Reinigung: Die Reaktionslösung wurde direkt für die Flash-Säulenchromatographie (9 g Kieselgel, Durchflussgeschwindigkeit 20 ml/min, Gradient 9 auf 25 % EtOAc in PE binnen 60 min), um ein farbloses Öl zu erhalten (18,6 mg, 63 %).
**[α]_{D}²³:** +15,6 (c 0,48, MeOH)

**HR-MS (APCI-PI)** ber. für M+H⁺: 431,2228, gef.: 431,2247.

**¹H-NMR (200 MHz, CDCl₃):** δ 0,95 (d, *J* = 6,3 Hz, 6H), 1,96-2,21 (m, 3H), 2,43-2,62 (m, 2H), 2,63-2,80 (m, 1H), 2,85 (dd, *J*= 12,4, 4,3 Hz, 1H), 3,76 (dd, *J*= 8,6, 6,4 Hz, 1H), 3,86 (s, 3H), 3,87 (s, 3H), 4,07 (dd, *J* = 8,6, 6,2 Hz, 1H), 4,18 (dd, *J* = 11,2, 7,5 Hz, 1H), 4,39 (dd, *J* = 11,2, 6,7 Hz, 1H), 4,84 (d, *J* = 6,0 Hz, 1H), 6,65-6,84 (m, 3H), 6,96-7,09 (m, 2H), 7,22-7,34 (m, 2H).

### Beispiel 33

### 10-78: ((2S,3R,4R)-4-(3,4-Dimethoxybenzyl)-2-(4-fluorphenyl)tetrahydrofuran-3-yl)-methylcyclobutancarboxylat (2863)

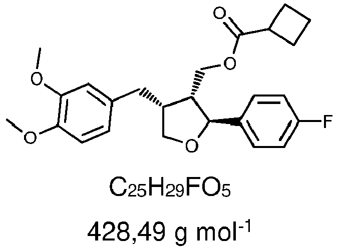

Herstellung analog zu jener von **10-41,** unter Verwendung von Ausgangsmaterial **9-24** (17,0 mg, 0,049 mmol, 1,00 Äquiv.) und Cyclobutancarbonsäure (11,3 mg, 0,113 mmol, 2,3 Äquiv.), EDCl.HCl (18,8 mg, 0,098 mmol, 2,0 Äquiv.), 4-DMAP (0,6 mg, 4,9 µmol, 0,1 Äquiv.) und DIPEA (21 µl, 0,12 mmol, 2,5 Äquiv.), und 13,5- statt 16-stündigem Rühren.

Aufarbeitung und Reinigung: Die Reaktionslösung wurde direkt für die Flash-Säulenchromatographie (9 g Kieselgel, Durchflussgeschwindigkeit 20 ml/min, isokratisch bei 9 % EtOAc in PE 20 min lang, dann Gradient 9 auf 25 % binnen 60 min) verwendet, um ein farbloses Öl zu erhalten (15,8 mg, 75 %).
**[α]_{D}²³:** +14,2 (c 1,25, MeOH)

**HR-MS (APCI-PI)** ber. für M+H⁺: 429,2072, gef.: 429,2085.

**¹H-NMR (200 MHz, CDCl₃):** δ 1,78-2,07 (m, 2H), 2,07-2,36 (m, 4H), 2,44-2,91 (m, 4H), 3,10 (quint, *J* = 8,3 Hz, 1H), 3,75 (dd, *J* = 8,6, 6,4 Hz, 1H), 3,86 (s, 3H), 3,87 (s, 3H), 4,07 (dd, *J* = 8,6, 6,3 Hz, 1H), 4,19 (dd, *J* = 11,2, 7,5 Hz, 1H), 4,39 (dd, *J* = 11,2, 6,6 Hz, 1H), 4,84 (d, *J* = 6,0 Hz, 1H), 6,65-6,85 (m, 3H), 6,95-7,09 (m, 2H), 7,21-7,35 (m, 2H).

### Vergleichsbeispiel 45

### 10-79: ((2S,3R,4R)-4-(3,4-Dimethoxybenzyl)-2-(3,4-dimethoxyphenyl)tetrahydrofuran-3-yl)methylpivalat (2734)

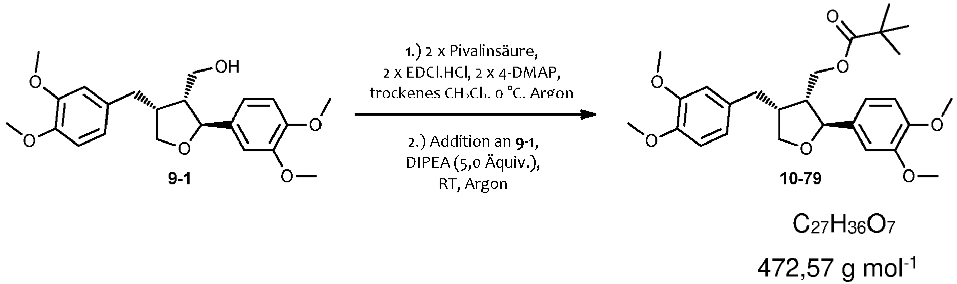

Herstellung: Ein Reaktionsgefäß wurde mit einem Rührstäbchen, Pivalinsäure (21,1 mg, 0,207 mmol, 2,3 Äquiv.) und 4-DMAP (1,1 mg, 9,0 µmol, 0,1 Äquiv.) beladen und dann evakuiert und mit Argon unter Anwendung eines Standard-Schlenk-Verfahrens (1x) wieder wiederbelüftet. Dann wurde trockenes CH₂CI₂ (1,0 ml) mittels Spritze zugesetzt, und die Lösung wurde in einem Eisbad auf 0 °C gekühlt. Das Gefäß wurde kurz geöffnet, EDCl.HCl (63,8 mg, 0,333 mmol, 3,7 Äquiv.) wurde auf einmal zugesetzt, und das Gemisch wurde 3 h lang bei 0 °C gerührt. Unterdessen wurde ein zweites Gefäß mit einem Rührstäbchen und **9-1** (35,0 mg, 0,090 mmol, 1,00 Äquiv.) beladen, evakuiert und mit Argon (3x) wiederbelüftet, und DIPEA (39 µl, 0,23 mmol, 2,5 Äquiv.) wurde mittels Spritze zugesetzt. Nach 3 h wurde die die aktivierte Carbonsäure enthaltende Lösung mittels Spritze in das zweite Gefäß übergeführt und 70 h lang bei Raumtemperatur gerührt. Um die Reaktion zu vervollständigen, wurde mehr von der aktivierten Carbonsäure in einem eigenen Gefäß wie oben (unter Verwendung von Pivalinsäure (18,3 mg, 0,180 mmol, 2,0 Äquiv.), 4-DMAP (1,1 mg, 9,0 µmol, 0,1 Äquiv.) und EDCl.HCl (30,0 mg, 0,157 mmol, 1,7 Äquiv.)) hergestellt, dann, nach 3 h bei 0 °C, in das Reaktionsgefäß zugesetzt, gefolgt von mehr DIPEA (39 µl, 0,23 mmol, 2,5 Äquiv.) mittels Spritze, und das Gemisch wurde weitere 96 h lang bei Raumtemperatur gerührt.

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 22 % EtOAc in PE binnen 9 min, dann isokratisch bei 22 % 6 min lang, dann 22 auf 38 % binnen 12 min, dann 38 auf 100 % binnen 10 min) verwendet, um ein farbloses Öl zu erhalten (32,2 mg, 76 %).
**[α]_{D}²³:** +22,5 (c 2,72, MeOH)

**HR-MS (ESI)** ber. für M+Na⁺: 495,2353, gef.: 495,2351.

**¹H-NMR (200 MHz, CDCl₃):** δ 1,21 (s, 9H), 2,45-2,81 (m, 3H), 2,87 (dd, *J*= 12,4, 4,1 Hz, 1H), 3,77 (dd, *J* = 8,6, 6,2 Hz, 1H), 3,88 (s, 9H), 3,89 (s, 3H), 4,07 (dd, *J* = 8,6, 6,3 Hz, 1H), 4,17 (dd, *J* = 11,3, 6,8 Hz, 1H), 4,36 (dd, *J* = 11,3, 6,9 Hz, 1H), 4,82 (d, *J* = 6,3 Hz, 1H), 6,66-6,90 (m, 6H).

**¹³C-NMR (50 MHz, CDCl₃):** δ 27,3, 33,3, 38,9, 42,8, 49,4, 56,0, 56,0, 56,0, 56,1, 62,7, 72,8, 82,9, 108,9, 111,2, 111,4, 112,0, 118,1, 120,6, 132,7, 135,1, 147,6, 148,6, 149,1, 149,2, 178,5.

### Beispiel 34

### 10-80: (E)-((2S,3R,4R)-4-(3,4-Dimethoxybenzyl)-2-(3,4,5-trimethoxyphenyl)tetrahydrofuran-3-yl)methyl-2-methylbut-2-enoat (2628)

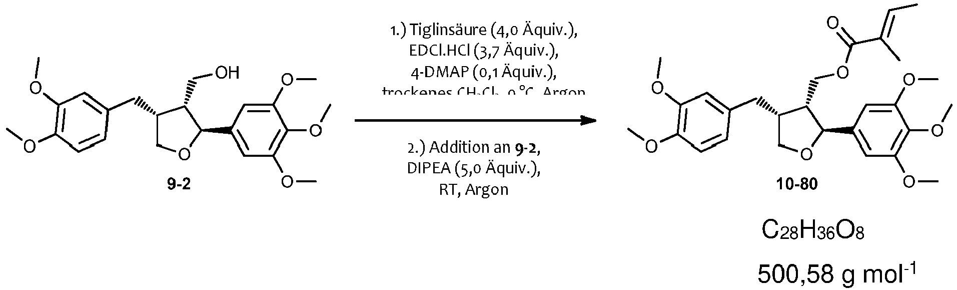

Herstellung: Ein Reaktionsgefäß wurde mit einem Rührstäbchen, Tiglinsäure (29,6 mg, 0,296 mmol, 4,0 Äquiv.) und 4-DMAP (0,9 mg, 7,4 µmol, 0,1 Äquiv.) beladen und dann evakuiert und mit Argon unter Anwendung eines Standard-Schlenk-Verfahrens (1x) wieder wiederbelüftet. Dann wurde trockenes CH₂CI₂ (1,0 ml) mittels Spritze zugesetzt, um eine 0,3 M Lösung (bezogen auf die Carbonsäure) zu erhalten, und die Lösung wurde in einem Eisbad auf 0 °C gekühlt. Das Gefäß wurde kurz geöffnet, EDCI.HCI (52,5 mg, 0,274 mmol, 3,7 Äquiv.) wurde auf einmal zugesetzt, und das Gemisch wurde 3 h lang bei 0 °C gerührt. Unterdessen wurde ein zweites Gefäß mit einem Rührstäbchen und **9-2** (30,0 mg, 0,074 mmol, 1,00 Äquiv.) beladen, evakuiert und mit Argon (3x) wiederbelüftet, und DIPEA (64 µl, 0,37 mmol, 5,0 Äquiv.) wurde mittels Spritze zugesetzt. Nach 3 h wurde die die aktivierte Carbonsäure enthaltende Lösung mittels Spritze in das zweite Gefäß übergeführt und 20 h lang bei Raumtemperatur gerührt.

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 22 % EtOAc in PE binnen 10 min, dann isokratisch bei 22 % 6 min lang, dann 22 auf 65 % binnen 30 min) verwendet, um ein gelbliches, viskoses Öl zu erhalten (25,4 mg, 70 %).
**[α]_{D}²⁰:** +19,2 (c 1,9, MeOH)

**HR-MS (ESI)** ber. für M+Na⁺: 523,2302, gef.: 523,2311.

**¹H-NMR (200 MHz, CDCl₃):** δ 1,69-1,85 (m, 6H), 2,46-2,95 (m, 4H), 3,81 (s, 3H), 3,83 (s, 7H), 3,84 (s, 3H), 3,85 (s, 3H), 4,07 (dd, *J* = 8,5, 6,2 Hz, 1H), 4,27 (dd, *J =* 11,3, 7,3 Hz, 1H), 4,43 (dd, *J* = 11,3, 6,4 Hz, 1H), 4,81 (d, *J* = 6,1 Hz, 1H), 6,53 (s, 2H), 6,64-6,84 (m, 4H).

**¹³C-NMR (50 MHz, CDCl₃):** δ 12,0, 14,4, 33,1, 42,6, 49,1, 55,8, 56,0, 60,8, 62,7, 72,9, 83,4, 102,6, 111,3, 111,8, 120,4, 128,2, 132,5, 137,2, 137,8, 138,2, 147,5, 148,9, 153,2, 167,8.

### Vergleichsbeispiel 46

### 10-82: ((2S,3R,4R)-4-(3,4-Dimethoxybenzyl)-2-(3,4,5-trimethoxyphenyl)tetrahydrofuran-3-yl)methylcyclohex-1-encarboxylat (2634)

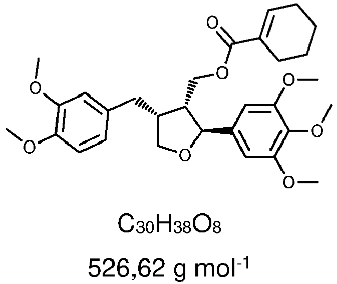

Herstellung, Aufarbeitung und Reinigung identisch mit jener von **10-80,** mit der Ausnahme, dass Cyclohex-1-encarbonsäure verwendet wurde, um ein gelbliches, viskoses Öl zu erhalten (21,3 mg, 56 %).
**[α]_{D}²⁰:** +17,9 (c 1,16, MeOH)

**¹H-NMR (200 MHz, CDCl₃):** δ 1,49-1,70 (m, 4H), 2,07-2,29 (m, 4H), 2,48-2,93 (m, 4H), 3,69-3,80 (m, 1H), 3,83 (s, 3H), 3,85 (s, 6H), 3,86 (s, 3H), 3,87 (s, 3H), 4,02-4,14 (m, 1H), 4,21-4,34 (m, 1H), 4,37-4,50 (m, 1H), 4,82 (d, *J* = 6,1 Hz, 1H), 6,55 (s, 2H), 6,64-6,83 (m, 3H), 6,90 (m, 1H).

**¹³C-NMR (50 MHz, CDCl₃):** δ 21,3, 22,0, 24,1, 25,8, 33,1, 42,6, 49,1, 55,8, 55,9, 56,0, 60,8, 62,5, 72,9, 83,5, 102,6, 111,3, 111,8, 120,4, 129,5, 130,3, 132,5, 138,3, 140,4, 147,5, 148,9, 153,2, 167,3.

### Beispiel 35

### 10-83: ((2S,3R,4R)-4-(3,4-Dimethoxybenzyl)-2-(3,4,5-trimethoxyphenyl)tetrahydrofuran-3-yl)methylcyclopent-1-encarboxylat (2636)

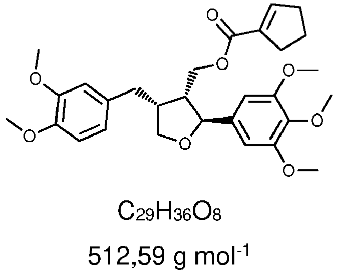

Herstellung, Aufarbeitung und Reinigung identisch mit jener von **10-80,** mit der Ausnahme, dass Cyclopent-1-encarbonsäure verwendet wurde, um ein gelbliches, viskoses Öl zu erhalten (25,7 mg, 69 %).
**[a]_{D}²⁰:** +21,4 (c 1,71, MeOH)

**¹H-NMR (200 MHz, CDCl₃):** δ 1,85-2,04 (m, 2H), 2,39-2,95 (m, 8H), 3,76 (dd, *J*= 8,6, 6,8 Hz, 1H), 3,81 (s, 3H), 3,84 (s, 6H), 3,85 (s, 3H), 3,86 (s, 3H), 4,08 (dd, *J* = 8,6, 6,5 Hz, 1H), 4,28 (dd, *J* = 11,2, 7,2 Hz, 1H), 4,44 (dd, *J* = 11,2, 6,4 Hz, 1H), 4,81 (d, *J* = 5,9 Hz, 1H), 6,53 (s, 2H), 6,64-6,84 (m, 4H).

**¹³C-NMR (50 MHz, CDCl₃):** δ 23,0, 31,3, 33,1, 33,4, 42,6, 49,0, 55,8, 55,9, 56,0, 60,8, 62,4, 72,9, 83,4, 102,8, 111,3, 111,8, 120,4, 132,5, 136,2, 137,2, 138,2, 144,5, 147,5, 148,9, 153,2, 165,1.

### Beispiel 36

### 10-84: ((2S,3R,4R)-4-(3,4-Dimethoxybenzyl)-2-(3,4,5-trimethoxyphenyl)tetrahydrofuran-3-yl)methylbenzoat (2638)

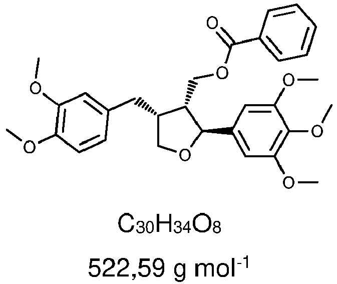

Herstellung, Aufarbeitung und Reinigung identisch mit jener von **10-80,** mit der Ausnahme, dass Benzoesäure verwendet wurde, um ein gelbliches, viskoses Öl zu erhalten (34,5 mg, 93 %).
**[a]_{D}²⁰:** +23,3 (c 2,17, MeOH)

**¹H-NMR (200 MHz, CDCl₃):** δ 2,47-2,54 (m, 4H), 3,80 (s, 6H), 3,81 (s, 3H), 3,85 (s, 6H), 4,14 (dd, *J* = 8,0, 6,1 Hz, 1H), 4,48 (dd, *J* = 11,4, 7,3 Hz, 1H), 4,66 (dd, *J* = 11,4, 6,2 Hz, 1H), 4,91 (d, *J* = 5,9 Hz, 1H), 6,57 (s, 2H), 6,64-6,84 (m, 3H), 7,37-7,49 (m, 2H), 7,58 (tt, *J* = 7,3, 1,4 Hz, 1H), 7,90-8,00 (m, 2H).

**¹³C-NMR (50 MHz, CDCl₃):** δ 33,2, 42,6, 49,1, 55,8, 56,0, 60,8, 63,3, 72,9, 83,6, 102,6, 111,3, 111,8, 120,4, 128,4, 129,5, 129,7, 132,4, 133,3, 137,2, 138,1, 148,9, 147,5, 153,3, 166,3.

### Beispiel 37

### 10-85: ((2S,3R,4R)-4-(3,4-Dimethoxybenzyl)-2-(3,4,5-trimethoxyphenyl)tetrahydrofuran-3-yl)methylbutyrat (2629)

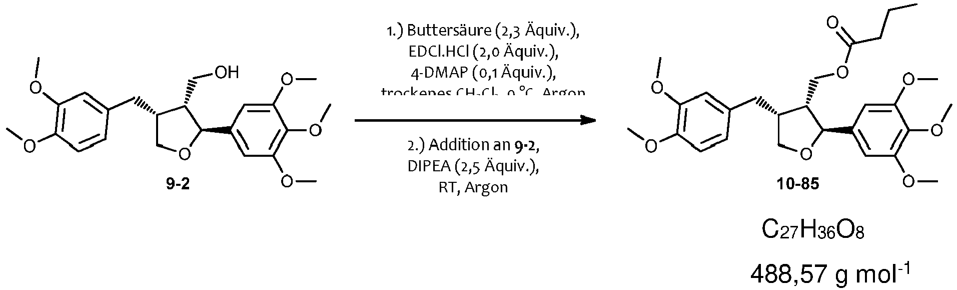

Herstellung: Ein Reaktionsgefäß wurde mit einem Rührstäbchen, Buttersäure (15,0 mg, 0,170 mmol, 2,3 Äquiv.) und 4-DMAP (0,9 mg, 7,4 µmol, 0,1 Äquiv.) beladen und dann evakuiert und mit Argon unter Anwendung eines Standard-Schlenk-Verfahrens (1x) wieder wiederbelüftet. Dann wurde trockenes CH₂CI₂ (1,0 ml) mittels Spritze zugesetzt, um eine 0,1 M Lösung (bezogen auf die Carbonsäure) zu erhalten, und die Lösung wurde in einem Eisbad auf 0 °C gekühlt. Das Gefäß wurde kurz geöffnet, EDCl.HCl (28,4 mg, 0,148 mmol, 2,0 Äquiv.) wurde auf einmal zugesetzt, und das Gemisch wurde 3 h lang bei 0 °C gerührt. Unterdessen wurde ein zweites Gefäß mit einem Rührstäbchen und **9-2** (30,0 mg, 0,074 mmol, 1,00 Äquiv.) beladen, evakuiert und mit Argon (3x) wiederbelüftet, und DIPEA (32 µl, 0,19 mmol, 2,5 Äquiv.) wurde mittels Spritze zugesetzt. Nach 3 h wurde die die aktivierte Carbonsäure enthaltende Lösung mittels Spritze in das zweite Gefäß übergeführt und 20 h lang bei Raumtemperatur gerührt.

Aufarbeitung und Reinigung: Die Reaktionslösung wurde direkt für die Flash-Säulenchromatographie (9 g Kieselgel, Durchflussgeschwindigkeit 20 ml/min, Gradient 10 auf 22 % EtOAc in PE binnen 10 min, dann isokratisch bei 22 % 6 min lang, dann 22 auf 65 % binnen 30 min) verwendet, um ein gelbliches, viskoses Öl zu erhalten (25,1 mg, 69 %).
**[α]_{D}²⁰:** +19,2 (c 1,95, MeOH)

**HR-MS (ESI)** ber. für M+Na⁺: 511,2302, gef.: 511,2282.

**¹H-NMR (200 MHz, CDCl₃):** δ 0,94 (t, *J* = 7,4, 3H), 1,64 (qt, *J*= 7,4, 7,4, 2H), 2,26 (t, *J* = 7,4, 2H), 2,46-2,92 (m, 4H), 3,74 (dd, *J* = 8,6, 6,5 Hz, 1H), 3,82 (s, 3H), 3,85 (s, 9H), 3,88 (s, 3H), 4,06 (dd, *J* = 8,6, 6,3 Hz, 1H), 4,20 (dd, *J* = 11,2, 7,2 Hz, 1H), 4,38 (dd, *J* = 11,2, 6,8 Hz, 1H), 4,77 (d, *J* = 5,9 Hz, 1H), 6,53 (s, 2H), 6,64-6,83 (m, 3H).

**¹³C-NMR (50 MHz, CDCl₃):** δ 13,7, 18,4, 33,1, 36,2, 42,4, 49,0, 55,9, 56,1, 60,8, 62,5, 72,8, 83,2, 102,6, 111,3, 111,8, 120,4, 132,5, 138,2, 147,5, 148,9, 153,3, 173,5. Ein C_{q} nicht sichtbar.

### Beispiel 38

### 10-86: ((2S,3R,4R)-4-(3,4-Dimethoxybenzyl)-2-(3,4,5-trimethoxyphenyl)tetrahydrofuran-3-yl)methylisobutyrat (2632)

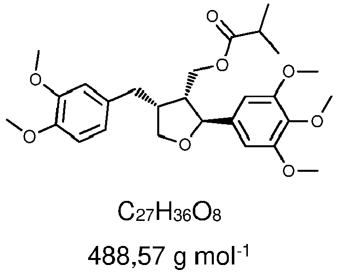

Herstellung, Aufarbeitung und Reinigung identisch mit jener von **10-85,** mit der Ausnahme, dass Isobuttersäure verwendet wurde, um ein gelbliches, viskoses Öl zu erhalten (29,1 mg, 83 %).
**[α]_{D}²⁰:** +19,9 (c 2,19, MeOH)

**¹H-NMR (200 MHz, CDCl₃):** δ 1,16 (d, *J* = 6,9 Hz, 6H), 2,45-2,91 (m, 5H), 3,75 (dd, *J* = 8,6, 6,5 Hz, 1H), 3,81 (s, 3H), 3,85 (s, 12H), 4,06 (dd, *J* = 8,6, 6,3 Hz, 1H), 4,19 (dd, *J* = 11,4, 7,1 Hz, 1H), 4,38 (dd, *J* = 11,4, 6,7 Hz, 1H), 4,79 (d, *J* = 5,9 Hz, 1H), 6,53 (s, 2H), 6,63-6,84 (m, 3H).

**¹³C-NMR (50 MHz, CDCl₃):** δ 18,9, 33,1, 34,0, 42,5, 49,1, 55,8, 56,1, 60,8, 62,5, 72,8, 83,1, 102,5, 111,3, 111,8, 120,4, 132,5, 138,2, 147,5, 148,9, 153,3, 176,8. Ein C_{q} nicht sichtbar.

### Beispiel 39

### 10-87: ((2S,3R,4R)-4-(3,4-Dimethoxybenzyl)-2-(3,4,5-trimethoxyphenyl)tetrahydrofuran-3-yl)methylcyclohexancarboxylat (2633)

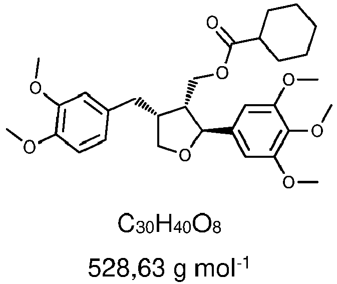

Herstellung, Aufarbeitung und Reinigung identisch mit jener von **10-85,** mit der Ausnahme, dass Cyclohexancarbonsäure verwendet wurde, um ein gelbliches, viskoses Öl zu erhalten (16,5 mg, 42 %).
**[a]_{D}²⁰:** +18,9 (c 2,10, MeOH)

**¹H-NMR (200 MHz, CDCl₃):** δ 1,13-1,97 (m, 10H), 2,18-2,35 (m, 1H), 2,44-2,93 (m, 4H), 3,75 (dd, *J* = 8,6, 6,7 Hz, 1H), 3,81 (s, 3H), 3,85 (s, 12H), 4,05 (dd, *J* = 8,6, 6,2 Hz, 1H), 4,18 (dd, *J=* 11,2, 7,1 Hz, 1H), 4,38 (dd, *J=* 11,2, 6,7 Hz, 1H), 4,78 (d, *J* = 6,1, 1H), 6,53 (s, 2H), 6,63-6,84 (m, 3H).

**¹³C-NMR (50 MHz, CDCl₃):** δ 25,4, 25,7, 29,0, 33,1, 42,5, 43,2, 49,1, 55,8, 55,9, 56,1, 60,8, 62,4, 72,8, 83,1, 102,5, 111,3, 111,8, 120,4, 132,5, 137,2, 138,2, 147,5, 148,9, 153,3, 175,8.

### Beispiel 40

### 10-88: ((2S,3R,4R)-4-(3,4-Dimethoxybenzyl)-2-(3,4,5-trimethoxyphenyl)tetrahydrofuran-3-yl)methylcyclopentancarboxylat (2635)

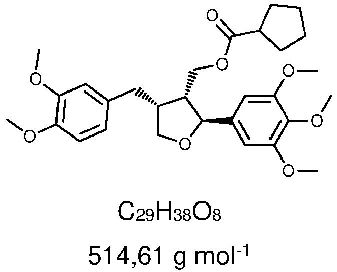

Herstellung, Aufarbeitung und Reinigung identisch mit jener von **10-85,** mit der Ausnahme, dass Cyclopentancarbonsäure verwendet wurde, um ein gelbliches, viskoses Öl zu erhalten (31,4 mg, 85 %).
**[a]_{D}²⁰:** +20,5 (c 2,05; MeOH)

**¹H-NMR (200 MHz, CDCl₃):** δ 1,45-1,96 (m, 8H), 2,45-2,91 (m, 5H), 3,75 (dd, *J*= 8,6, 6,5 Hz, 1H), 3,82 (s, 3H), 3,85 (s, 9H), 3,86 (s, 3H), 4,06 (dd, *J* = 8,6, 6,5 Hz, 1H), 4,19 (dd, *J* = 11,4, 7,0 Hz, 1H), 4,38 (dd, *J* = 11,4, 6,7 Hz, 1H), 4,78 (d, *J* = 6,1, 1H), 6,53 (s, 2H), 6,64-6,84 (m, 3H).

**¹³C-NMR (50 MHz, CDCl₃):** δ 25,6, 29,9, 33,1, 42,5, 43,8, 49,1, 50,1, 55,8, 55,9, 60,8, 62,5, 72,8, 83,1, 102,5, 111,3, 111,8, 120,4, 128,6, 132,5, 138,2, 147,5, 148,9, 153,3, 176,5.

### Beispiel 41

### 10-89: ((2S,3R,4R)-4-(3,4-Dimethoxybenzyl)-2-(3,4,5-trimethoxyphenyl)tetrahydrofuran-3-yl)methylcyclopropancarboxylat (2637)

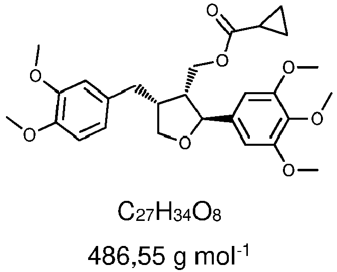

Herstellung, Aufarbeitung und Reinigung identisch mit jener von **10-85,** mit der Ausnahme, dass Cyclopropancarbonsäure verwendet wurde, um ein gelbliches, viskoses Öl zu erhalten (26,2 mg, 75 %).
**[a]_{D}²⁰:** +20,4 (MeOH; c 1,785)

**¹H-NMR (200 MHz, CDCl₃):** δ 0,78-1,03 (m, 4H), 1,49-1,65 (m, 1H), 2,47-2,93 (m, 4H), 3,74 (dd, *J*= 8,6, 6,5 Hz, 1H), 3,82 (s, 3H), 3,85 (s, 9H), 3,86 (s, 3H), 4,07 (dd, *J* = 8,6, 6,2 Hz, 1H), 4,20 (dd, *J* = 11,2, 7,2 Hz, 1H), 4,38 (dd, *J* = 11,2, 6,8 Hz, 1H), 4,79 (d, *J* = 6,1 Hz, 1H), 6,54 (s, 2H), 6,64-6,84 (m, 3H).

**¹³C-NMR (50 MHz, CDCl₃):** δ 8,5, 12,8, 33,1, 42,4, 49,0, 55,8, 56,1, 60,8, 62,7, 72,8, 83,2, 102,5, 111,3, 111,8, 120,4, 132,5, 138,2, 147,5, 148,9, 153,2, 174,7. Ein C_{q} nicht sichtbar.

### Beispiele 42 und 43

In dieser abschließenden Beispielsgruppe werden freie Alkohole der Formel (9) durch Williamson-Veretherung in Verbindungen der Formel (10) übergeführt.

### Beispiel 42

### 10-90: (2S,3R,4R)-3-((Allyloxy)methyl)-4-(3,4-dimethoxybenzyl)-2-(3,4-dimethoxyphenyl)tetrahydrofuran (2540)

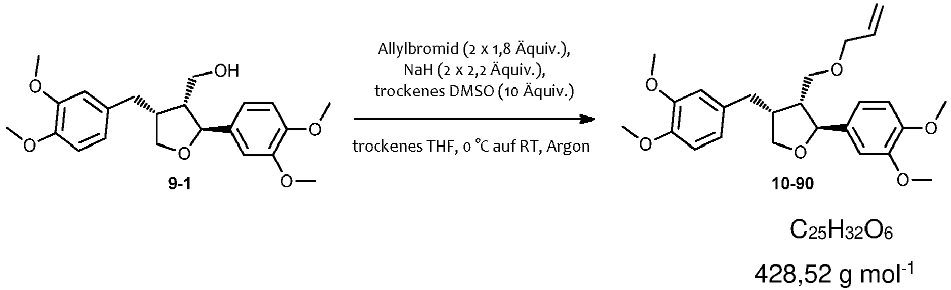

Herstellung: Ein Reaktionsgefäß wurde mit einem Rührstäbchen, einer Mineralöldispersion von NaH (etwa 60%ig, 8,8 mg, 0,22 mmol, 2,2 Äquiv.) beladen und dann evakuiert und mit Argon unter Anwendung eines Standard-Schlenk-Verfahrens (3x) wiederbelüftet. Dann wurde trockenes THF (0,5 ml) zugesetzt, gefolgt von trockenem DMSO (71 µl, 1,0 mmol, 10 Äquiv.), beide mittels Spritze, und die gerührte Suspension wurde in einem Eisbad auf 0 °C gekühlt. Darauf folgte das Zutropfen von **9-1** (38,8 mg, 0,100 mmol, 1,0 Äquiv.) in trockenem THF (1,0 ml), anschließend 15-minütiges Rühren der Reaktion bei 0 °C und schließlich Allylbromid (16 µl, 0,18 mmol, 1,8 Äquiv.), beide mittels Spritze. Das Eisbad wurde entfernt, und das Gemisch wurde bei Raumtemperatur 18 h lang gerührt, bevor das Gefäß kurz geöffnet wurde, um mehr NaH-Dispersion (8,8 mg, 0,22 mmol, 2,2 Äquiv.) zuzusetzen. 1 h später wurde mehr Allylbromid (16 µl, 0,18 mmol, 1,8 Äquiv.) mittels Spritze zugesetzt, und das Gemisch wurde 25 h lang bei Raumtemperatur gerührt.

Aufarbeitung und Reinigung: Die Reaktion wurde durch den Zusatz von gesättigtem wässrigem NH₄CI (1,0 ml) gequencht und mit Et₂O (2 x 10 ml) extrahiert. Die vereinigten organischen Phasen wurden mit Na₂SO₄ getrocknet, die Lösungsmittel wurden abgedampft, und die Zielverbindung wurde mittels Flash-Säulenchromatographie (18 g Kieselgel, Durchflussgeschwindigkeit 20 ml/min, Gradient 10 auf 90 % EtOAc in PE binnen 60 min) gereinigt, um ein nahezu farbloses Öl zu erhalten (31,8 mg, 74 %).
**[α]_{D}²⁰:** +19,6 (c 1,63, MeOH)

**HR-MS (ESI)** ber. für M+Na⁺: 451,2091, gef.: 451,2096.

**¹H-NMR (200 MHz, CDCl₃):** δ 2,41-2,60 (m, 2H), 2,63-2,82 (m, 1H), 2,93 (dd, *J* = 13,0, 4,2 Hz, 1H), 3,52 (dd, *J* = 9,3, 6,3 Hz, 1H), 3,60-3,81 (m, 2H), 3,87 (s, 3H), 3,87 (s, 6H), 3,88 (s, 3H), 3,95-4,09 (m, 3H), 4,80 (d, *J* = 6,6 Hz, 1H), 5,20 (ddt, *J* = 10,3, 1,7, 1,3 Hz, 1H), 5,30 (ddt, *J* = 17,3, 1,7, 1,6 Hz, 1H), 5,93 (dddd, *J* = 17,3, 10,3, 5,9, 5,0 Hz, 1H), 6,67-6,92 (m, 6H).

**¹³C-NMR (50 MHz, CDCl₃):** δ 33,2, 42,7, 50,5, 56,0, 56,0, 56,0, 56,0, 68,2, 72,3, 72,9, 82,8, 109,1, 111,0, 111,3, 112,0, 117,1, 118,1, 120,6, 133,3, 134,8, 135,7, 147,5, 148,4, 149,0, 149,1.

### Beispiel 43

### 10-91: (2S,3R,4R)-4-(3,4-Dimethoxybenzyl)-2-(3,4-dimethoxyphenyl)-3-((prop-2-in-1-yloxy)methyl)tetrahydrofuran (2541)

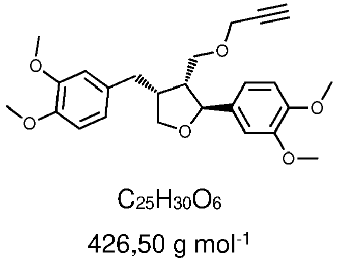

Herstellung identisch mit jener von **10-90,** mit der Ausnahme, dass Propargylbromid (2 × 20 µl, 2 × 0,18 mmol, 2 × 1,8 Äquiv.) anstelle von Allylbromid verwendet wurde. Aufarbeitung und Reinigung: Die Reaktion wurde durch den Zusatz von gesättigtem wässrigem NH₄CI (1,0 ml) gequencht und mit Et₂O (2 × 10 ml) extrahiert. Die vereinigten organischen Phasen wurden mit Na₂SO₄ getrocknet, die Lösungsmittel wurden abgedampft, und die Zielverbindung wurde mittels Flash-Säulenchromatographie (18 g Kieselgel, Durchflussgeschwindigkeit 20 ml/min, Gradient 10 auf 90 % EtOAc in PE binnen 60 min) gereinigt, um ein nahezu farbloses Öl zu erhalten (23,2 mg, 54 %).
**[α]_{D}²⁰:** +25,1 (c 1,49, MeOH)

**HR-MS (ESI)** ber. für M+Na⁺: 449,1935, gef.: 449,1940.

**¹H-NMR (200 MHz, CDCl₃):** δ 2,41-2,61 (m, 2H), 2,45 (t, *J =* 2,4 Hz, 1H), 2,62-2,83 (m, 1H), 2,95 (dd, *J* = 13,0, 4,2 Hz, 1H), 3,60 (dd, *J* = 9,1, 6,2 Hz, 1H), 3,81-3,69 (m, 2H), 3,86 (s, 3H), 3,87 (s, 6H), 3,89 (s, 3H), 4,03 (dd, *J* = 8,5, 6,3 Hz, 1H), 4,18 (t, *J* = 2,1 Hz, 2H), 4,80 (d, *J* = 6,7 Hz, 1H), 6,92-6,66 (m, 6H).

### Beispiel 44 - Aktivitätsmessungen

Zur Bestimmung der zellvermehrungshemmenden Aktivität der wie oben beschrieben hergestellten Testverbindungen wurden die folgenden Tests durchgeführt.

Zur Bestimmung der Gefäß-SMC-Proliferation wurden 0,5 × 10⁴ lebende Rattenaorta-SMC pro Well in SMC-Nährmedium (DMEM/F12-Medium mit 20 % Serum, 30 µg/ ml Gentamicin und 15 ng/ml Amphotericin) in 96-Well-Patten ausgesät. 24 h später wurde das Medium entfernt, die Zellen wurden einmal mit SMC-Aushungerungsmedium (DMEM/F12-Medium mit 0,1 % Serum, 0,2 % BSA, 30 µg/ml Gentamicin und 15 ng/ml Amphotericin) gewaschen und weitere 24 h lang in Aushungerungsmedium inkubiert. Die ruhenden Zellen wurden dann vorab für 30 min mit den Testverbindungen versetzt und mit 20 ng/ml Platelet-Derived Growth Factor (PDGF) zum Proliferieren gebracht. Nicht stimulierte Zellen wurden zur Normalisierung und Abschätzung des Ausgangsniveaus der Proliferation herangezogen. Die Endkonzentration des Lösungsmittelvehikels, Dimethylsulfoxid (DMSO), war in allen Wells gleich (0,1 %). 48 h später wurde die SMC-Proliferation mittels 2-stündiger Umsetzung mit Resazurinfarbstoff wie zuvor beschrieben (H. Joa et al., J. Nat. Prod. 74(6),1513-6 (2011)) quantifiziert.

Zur Bestimmung der Endothelzellenproliferation wurden 0,5 × 10⁴ mit HUVECtert immortalisierte menschliche Nabelschnurvenen-Endothelzellen (H.B. Schiller et al., Mol. Biol. Cell. 20(3), 745-756 (2009)) für 24 h in 96-Well-Platten in HUVEC-Complete-Medium (EBM^{™}-Nährmedium, ergänzt mit 10% fötalem Rinderserum, EBM^{™}-SingleQuots (Lonza, Basel, Schweiz), 100 U/ml Benzylpenicillin, 100 µg/ml Streptomycin und 1 % Amphotericin) ausgesät. Danach wurde das Medium gegen frisches HUVEC-Complete-Medium ausgetauscht, und die Zellen wurde mit den angegebenen Verbindungen 48 h lang inkubiert. Nach Ende der Inkubationsphase wurde das Medium entfernt, die Zellen wurden einmal mit 200 µl/Well PBS gewaschen und mit 150 µl HUVEC-Complete-Medium versetzt, das 10 µg/ml Resazurin enthielt. 2 h später wurde die Fluoreszenz bei einer Wellenlänge von 580 nm mit einer Anregungswellenlänge von 535 nm in einem 96-Well-Plattenlesegerät (Tecan GENios Pro; Tecan Group Ltd., Männedorf, Schweiz) gemessen.

Der Anteil an lebenden Zellen wurde in allen Fällen von 1 abgezogen, um ein Maß für die Wirkung der Testverbindungen auf den jeweiligen Zelltyp zu erhalten, und danach wurde der Quotient der Wirkungen auf SMC und auf EC gebildet und als "Aktivitätsverhältnis" und somit als Maß für die selektive Wirkung der jeweiligen Testverbindung auf SMC in Bezug auf EC herangezogen. So lieferte beispielsweise das in Vergleichsbeispiel 27 als Verbindung 10-22 hergestellte synthetische Leoligin mit der Bio-ID: 2418 die folgenden Ergebnisse:

| | |
|---|---|
| Anteil an überlebenden SMC: | 0,81 (81 %) |
| Anteil an überlebenden EC: | 0,51 (51 %) |
| Wirkung auf SMC: | 1-0,81 = 0,19 |
| Wirkung auf EC: | 1-0,51 = 0,49 |
| Aktivitätsverhältnis: | 0,19/0,49 = 0,39 |

Das Verhältnis < 1 zeigt, dass Leoligin in diesen Assays EC in Bezug auf SMC selektiv hemmte, nämlich etwa um den Faktor 2,5 (d.i. der Kehrwert von 0,39). Dieses Ergebnis ist, wie zuvor erwähnt, konsistent mit früheren Untersuchungen an der Universität Innsbruck, aus denen ein Verhältnis der IC₅₀-Werte von 0,33 ableitbar ist.

Die Erfinder haben hingegen überraschenderweise festgestellt, dass manche der neu synthetisierten Derivate von Leoligin genau die umgekehrte Selektivität aufweisen, d.h. SMC in Bezug auf EC selektiv hemmen, was z.B. speziell bei der Behandlung bzw. Prophylaxe von Restenose besonders vorteilhaft ist.

Aufgrund der üblichen Schwankungen bei der Bestimmung der überlebenden Zellen von rund ± 10 % und den daraus resultierenden relativ starken Schwankungen bei der Quotientenbildung - insbesondere bei sehr geringer Hemmwirkung der neuen Verbindungen auf EC - wurde der bereits erwähnte Schwellenwert von 1,50 für das Aktivitätsverhältnis eingezogen. Dies schließt aber nicht aus, dass sich Verbindungen, die bisher nicht die gewünschte Aktivität gezeigt haben, in Zukunft als doch für die Zwecke der Offenbarung geeignet herausstellen könnten.

Nachstehend folgt eine tabellarische Auflistung aller hierin in Beispielen und Vergleichsbeispielen in Bezug auf die Aktivität hergestellten Verbindungen, für die ein Aktivitätsverhältnis (A.V.) größer 1,0 festgestellt wurde. Aufgrund der erwähnten Schwankungen erfolgt die Angabe nicht anhand der exakten errechneten Verhältnisse, sondern lediglich in den folgenden vier buchstabenkodierten Gruppen:

| | |
|---|---|
| Vergleichsbeispiele - V: | 1,0 < A.V. < 1,50 |
| Erfindungsgemäße Beispiele -A: | A.V. ≥ 1,50 |
| Bevorzugte Beispiele - AA: | A.V. ≥ 2,0 |
| Besonders bevorzugte Beispiele - AAA: | A.V. ≥ 5,0 |

Die Reihenfolge in Tabelle 1 entspricht allerdings sehr wohl den errechneten Werten.

**Tabelle 1**

| **Beispiel** | **Bio-ID** | **A.V.-Code** |
|---|---|---|
| Beispiel 38 | 2632 | AAA |
| Beispiel 13 | 3026 | AAA |
| Beispiel 8 | 2760 | AAA |
| Beispiel 18 | 3010 | AAA |
| Beispiel 42 | 2540 | AAA |
| Beispiel 15 | 3016 | AAA |
| Beispiel 2 | 2754 | AAA |
| Beispiel 19 | 2755 | AAA |
| Beispiel 7 | 2756 | AAA |
| Beispiel 16 | 3015 | AAA |
| Beispiel 32 | 2862 | AAA |
| Beispiel 17 | 2864 | AAA |
| Beispiel 37 | 2629 | AAA |
| Beispiel 34 | 2628 | AAA |
| Beispiel 12 | 2823 | AAA |
| Beispiel 23 | 2549 | AA |
| Beispiel 9 | 2767 | AA |
| Beispiel 6 | 3013 | AA |
| Beispiel 33 | 2863 | AA |
| Beispiel 40 | 2635 | AA |
| Beispiel 41 | 2637 | AA |
| Beispiel 3 | 2821 | AA |
| Beispiel 5 | 3008 | AA |
| Beispiel 27 | 2745 | AA |
| Beispiel 10 | 2788 | AA |
| Beispiel 21 | 2771 | AA |
| Beispiel 20 | 2765 | AA |
| Beispiel 25 | 2738 | AA |
| Beispiel 22 | 2774 | AA |
| Beispiel 35 | 2636 | AA |
| Beispiel 24 | 2739 | AA |
| Beispiel 30 | 2867 | AA |
| Beispiel 28 | 2746 | A |
| Beispiel 43 | 2541 | A |
| Beispiel 4 | 3005 | A |
| Beispiel 1 | 2749 | A |
| Beispiel 26 | 2742 | A |
| Beispiel 14 | 3029 | A |
| Beispiel 29 | 2781 | A |
| Beispiel 11 | 2792 | A |
| Beispiel 36 | 2638 | A |
| Beispiel 31 | 2856 | A |
| Beispiel 39 | 2633 | A |
| Vergleichsbeispiel 26 | 2548 | V |
| Vergleichsbeispiel 34 | 2775 | V |
| Vergleichsbeispiel 35 | 2539 | V |
| Vergleichsbeispiel 13 | 2757 | V |
| Vergleichsbeispiel 40 | 3027 | V |
| Vergleichsbeispiel 43 | 2861 | V |
| Vergleichsbeispiel 45 | 2734 | V |
| Vergleichsbeispiel 33 | 2770 | V |
| Vergleichsbeispiel 41 | 2854 | V |
| Vergleichsbeispiel 24 | 2827 | V |
| Vergleichsbeispiel 38 | 2784 | V |
| Vergleichsbeispiel 29 | 2859 | V |
| Vergleichsbeispiel 36 | 2544 | V |
| Vergleichsbeispiel 42 | 2855 | V |
| Vergleichsbeispiel 44 | 2860 | V |
| Vergleichsbeispiel 46 | 2634 | V |
| Vergleichsbeispiel 28 | 3030 | V |
| Vergleichsbeispiel 31 | 2764 | V |
| Vergleichsbeispiel 39 | 2866 | V |
| Vergleichsbeispiel 30 | 3011 | V |
| Vergleichsbeispiel 32 | 2769 | V |
| Vergleichsbeispiel 37 | 2545 | V |

Die obigen Ausführungen belegen jedenfalls klar, dass ein Teil (nämlich 43 von insgesamt 130, also ziemlich genau ein Drittel) der von den Erfindern synthetisierten neuen Verbindungen die Proliferation von SMC in Bezug auf EC selektiv zu hemmen imstande sind. Dadurch eignen sich die Verbindungen nicht nur zur Behandlung von durch übermäßige SMC-Vermehrung ausgelösten Krankheiten und Leiden, sondern speziell zur Behandlung von oder Vorbeugung gegen Restenose nach Stentimplantation. Die Offenbarung umfasst daher auch entsprechende Arzneimittel bzw. pharmazeutische Zusammensetzungen sowie damit beschichtete Stents.

Die vorliegende Erfindung stellt dabei ein Verfahren zur Herstellung dieser Verbindungen bereit, durch das die Verbindungen in deutlich höheren Ausbeuten, kostengünstiger und rascher hergestellt werden können, als dies nach dem Stand der Technik möglich war.

## Patentansprüche

1. Verfahren zur Herstellung einer Verbindung der nachstehenden Formel (II): 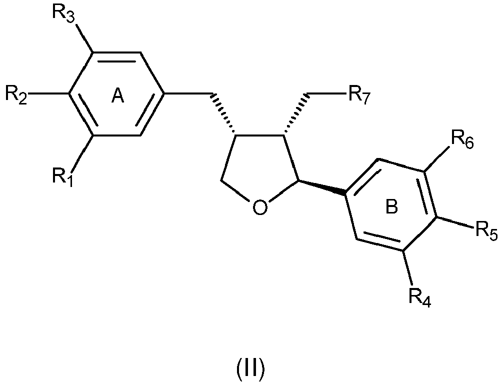 worin entweder
R₁ bis R₆ ausgewählt sind aus -H, -F, -CH₃, -CF₃, -CF₂CH₃, -OCH₃, -COCH₃, -C₄H₉, -COOC₂H₅ und -C₆H₅ oder zwei vicinale Reste aus R₁ bis R₆ so beschaffen und miteinander verbunden sind, dass sie zusammen mit den beiden Kohlenstoffatomen, an die sie gebunden sind, einen gesättigten oder ungesättigten carbocyclischen Ring bilden, und R₇ ausgewählt ist aus OH, Propargyloxy, Cyclopropylcarbonyloxy, Cyclobutylcarbonyloxy, Cyclopentylcarbonyloxy, Cyclopentenylcarbonyloxy, Cyclohexylcarbonyloxy, Cyclohexenylcarbonyloxy, Adamantylethanoyloxy, 3-Phenylpropenoyloxy (Cinnamyloyloxy), 2-Methylbenzoyloxy und Naphthoyloxy, wobei im Phenylring A gegebenenfalls das ortho-Kohlenstoffatom durch ein Stickstoffatom ersetzt ist; oder
R₁ bis R₆ ausgewählt sind aus -H, -OH, Halogen, Alkyl und Alkoxy und R₇ ausgewählt ist aus -OR₉, -N(R₉')R₉, -SR₉, -C(O)Rg, -OC(O)Rg, -C(O)OR₉, -N(Rg')C(O)Rg, -C(O)N(R₉')R₉ und -S(O)Rg, worin R₉ aus Alkyl und Alkenyl und R₉' aus -H sowie Alkyl und Alkenyl ausgewählt sind,
**gekennzeichnet durch** die folgenden Reaktionsschritte a) bis h):
a) die an sich bekannte Umsetzung eines mit den entsprechenden Resten R₄ bis R₆ substituierten Benzaldehyds der Formel (1) mit Vinylmagnesiumbromid zum entsprechenden racemischen α-Vinylbenzylalkohol *rac*-(2): 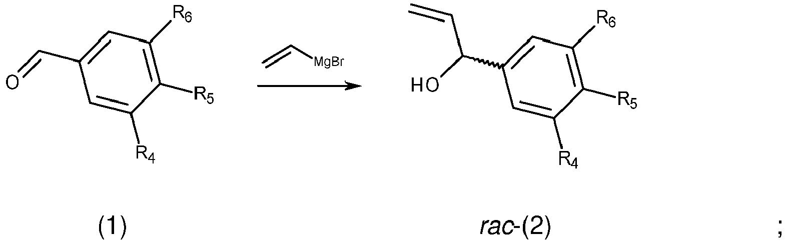
b) kinetische Racematspaltung von *rac*-(2) mittels enzymatischer Katalyse zum Erhalt des (*S*)-Enantiomers (*S*)-(2) unter gleichzeitiger Bildung von (R)-Rs-(2) als abzutrennendes Nebenprodukt, worin R₈ für einen Acylrest steht: 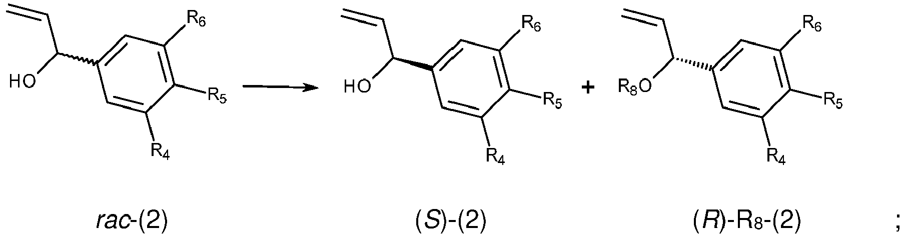
c) anschließende Veretherung und Oxidation von (*S*)-(2) in beliebiger Reihenfolge, d.h. entweder:
c1) Veretherung von (*S*)-(2) mit Propargylbromid zum Propargylether (3): 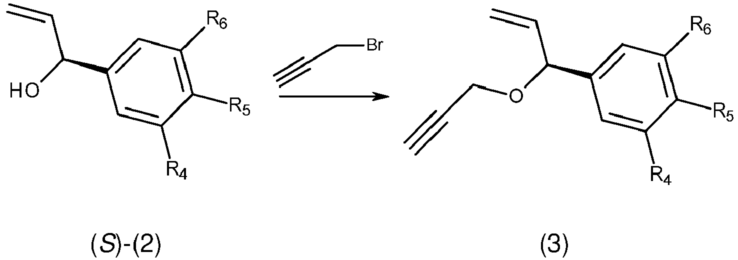
und darauf folgende Oxidation von (3) zum Propargylether-Epoxid (5): 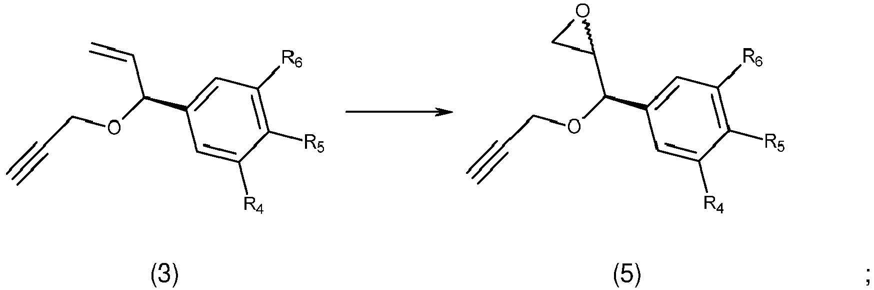 oder umgekehrt:
c2) Oxidation von (*S*)-(2) zum Epoxid (4): 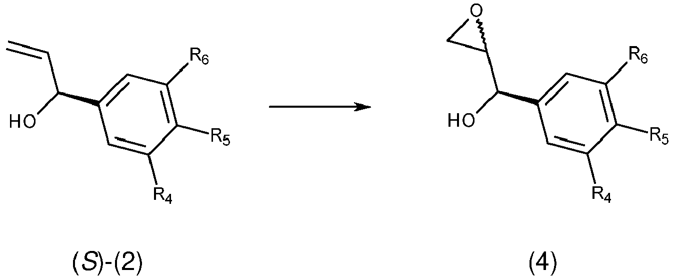 und darauf folgende Veretherung von (4) mit Propargylbromid zum Propargylether-Epoxid (5): 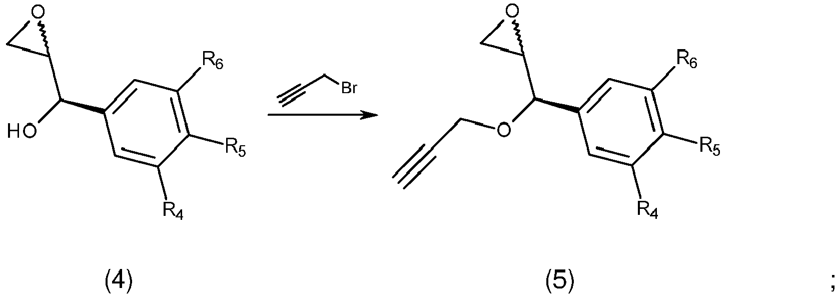
d) Zyklisierung von (5) mithilfe eines Zyklisierungsmittels zum Tetrahydrofuranylmethanol (6): 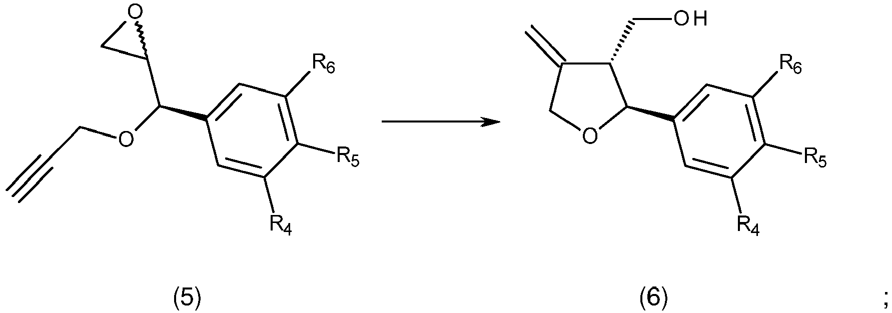
e) Schützen der Alkoholfunktionalität von (6) mit der tert-Butyldimethylsilyl- (TBDMS-) Gruppe zum geschützten Methanol (7): 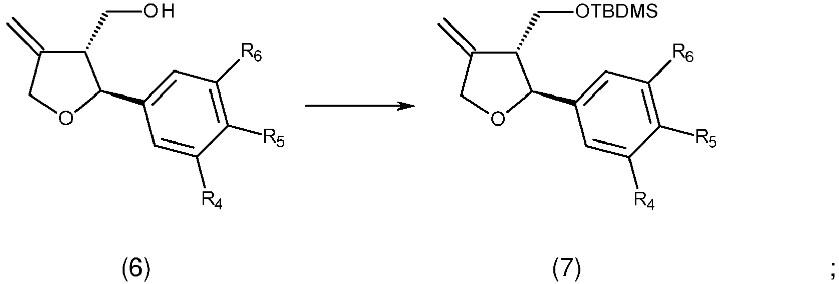
f) Einführung eines mit den entsprechenden Resten R₁ bis R₃ substituierten Phenyl- oder 2-Pyridylrests (7b) durch Reaktion mit der exozyklischen Methylengruppe von (7) zum geschützten Produkt (8): 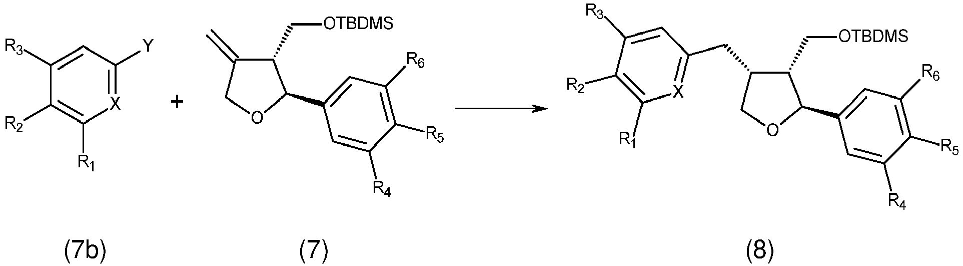 worin X jeweils für CH oder N steht und Y für eine Abgangsgruppe steht;
g) Abspaltung der Schutzgruppe von (8) zum freien Alkohol (9): 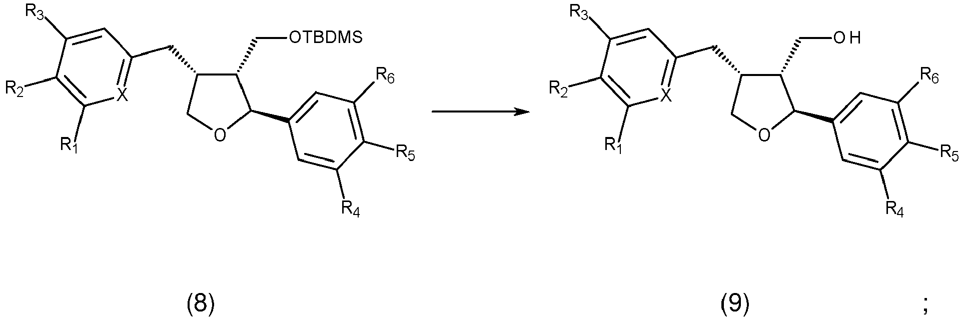
h) wenn R₇ ≠ OH ist, die gleichzeitige oder anschließende Veretherung oder Veresterung der freien OH-Gruppe, um Verbindung (10) mit dem entsprechenden Rest R₇ zu erhalten:

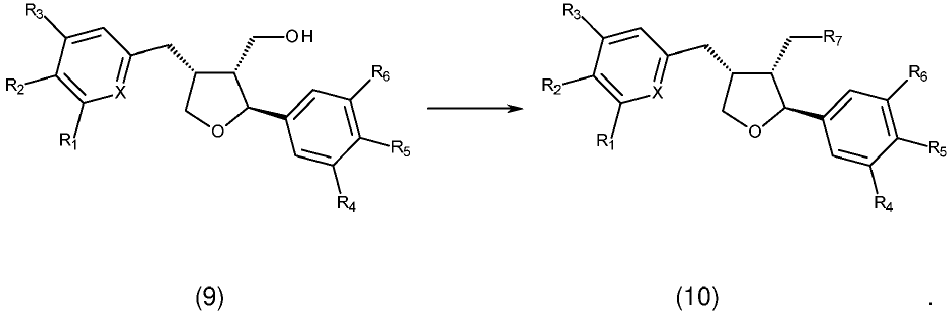

2. Verfahren zur Herstellung einer Verbindung der Formel (II) wie in Anspruch 1 definiert, **gekennzeichnet durch** die folgenden Reaktionsschritte a) bis f),
a) bis d) Durchführung derselben Reaktionsschritte a) bis d) wie in Anspruch 1 definiert, ausgehend von dem mit den entsprechenden Resten R₄ bis R₆ substituierten Benzaldehyd der Formel (1), um das entsprechende Tetrahydrofuranylmethanol (6) zu ergeben; gefolgt von
e) wenn R₇ ≠ OH ist, Verestern der OH-Gruppe von (6) mit jenem Rest, der zusammen mit dem Sauerstoffatom den jeweiligen Rest R₇ ergibt; und
f) Einführung eines mit den entsprechenden Resten R₁ bis R₃ substituierten Phenyl- oder 2-Pyridylrests (7b) durch Reaktion mit der exozyklischen Methylengruppe, um die gewünschte Verbindung der Formel (10) zu erhalten.

3. Verfahren nach Anspruch 1 oder 2, **dadurch gekennzeichnet, dass** in Schritt b) als Enzym Amano-Lipase PS und als deren Donor-Komponente Vinylacetat eingesetzt werden, so dass der Rest R₈ ein Acetylrest ist und als Nebenprodukt (R)-Acetyl-(2) anfällt.

4. Verfahren nach einem der Ansprüche 1 bis 3, **dadurch gekennzeichnet, dass**
in Schritt c1) die nach der Propargylierung erfolgende Oxidation mit m-Chlorperbenzoesäure als Oxidationsmittel durchgeführt wird; oder
in Schritt c2) die vor der Propargylierung erfolgende Oxidation als enantioselektive Sharpless-Epoxidierung mit (*S,S*)-Diethyltartrat, (-)-DET, als Co-Reagens, tert-Butylhydroperoxid, TBHP, als Oxidationsmittel und Titan(IV)-isopropanolat, Ti(O-i-Pr)₄, als Katalysator durchgeführt wird.

5. Verfahren nach einem der Ansprüche 1 bis 4, **dadurch gekennzeichnet, dass** in Schritt d) Titanocendichlorid (Dichlorobis(cyclopentadienyl)titan(IV), Cp₂TiCl₂) als Zyklisierungsmittel eingesetzt wird.

6. Verfahren nach einem der Ansprüche 1 und 3 bis 4, **dadurch gekennzeichnet, dass** in Schritt f) eine Suzuki-Kupplung durchgeführt wird, bei der die exozyklische Methylengruppe von (7) zunächst in einem Schritt f1) intermediär mit einem Organoboran stereoselektiv zum Zwischenprodukt (7a) hydroboriert wird, das unmittelbar darauf in einem Schritt f2) mit Verbindung (7b), worin Y für ein Halogenatom steht, umgesetzt wird, um den Borylrest durch den mit R₁ bis R₃ substituierten Phenyl- oder 2-Pyridylrest zu ersetzen.

7. Verfahren nach Anspruch 6, **dadurch gekennzeichnet, dass** in Schritt f1) die Verbindung der Formel (7) mit 9-Borabicyclo[3.3.1]nonan, 9-BBN, umgesetzt wird, um intermediär das Boran (7a) zu erhalten: 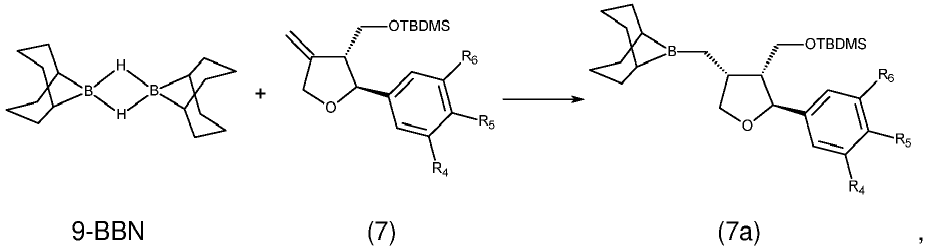 das im unmittelbar folgenden Schritt f2) mit dem entsprechend substituierten Phenyl oder 2-Pyridylbromid (7b) unter Verwendung von 1,1'-Bis(diphenylphosphino)ferrocendichloropalladium(II)-Dichlormethan-Komplex, Pd(dppf)Cl₂.CH₂Cl₂, als Katalysator zu Verbindung (8) umgesetzt wird.

8. Verfahren nach einem der Ansprüche 1 und 3 bis 7, **dadurch gekennzeichnet, dass** in Schritt g) die Abspaltung der Alkohol-Schutzgruppe mit Tetra-n-butylammoniumfluorid, TBAF, durchgeführt wird, wobei die Schritte f) und g) gegebenenfalls als Eintopfreaktion durchgeführt werden.

9. Verfahren nach einem der Ansprüche 1 bis 8, **dadurch gekennzeichnet, dass** zur Veresterung der OH-Gruppe in Schritt h) bzw. e)
eine Mitsunobu-Veresterung, die gegebenenfalls in Gegenwart von 1,1'-(Azodicarbonyl)dipiperidin, ADD, oder Diethylazodicarboxylat, DEAD, und Triphenylphosphin, PPh₃, erfolgt, oder
eine Steglich-Veresterung, die gegebenenfalls in Gegenwart von N-(3-Dimethylaminopropyl)-N'-ethylcarbodiimid, EDC, oder N,N'-Dicyclohexylcarbodiimid, DCC, und 4-Dimethylaminopyridin, DMAP, erfolgt,
mit einer freien Säure, die zur Bildung des jeweiligen Rests R₇ dient, durchgeführt wird.

10. Verfahren nach einem der Ansprüche 1 bis 9, **dadurch gekennzeichnet, dass** als Verbindung der Formel (II) eine der folgenden, durch entsprechende Kombination der Reste R₁ bis R₇ erhältlichen Verbindungen hergestellt wird:
((*2S,3R,4R*)-4-(4-(Trifluormethyl)benzyl)-2-(3,4,5-trimethoxyphenyl)tetrahydrofuran-3-yl)methanol (2760),
(*Z)*-2-Methyl-2-butensäure-((*2S,3R,4R*)-2-(4-fluorphenyl)-4-(4-(trifluormethyl)benzyl)-tetrahydrofuran-3-yl)methylester (3016),
((*2S,3R,4R*)-2-(3,4-Dimethoxyphenyl)-4-(4-methylbenzyl)tetrahydrofuran-3-yl)-methanol (2754),
((*2S,3R,4R*)-4-Benzyl-2-(3,4,5-trimethoxyphenyl)tetrahydrofuran-3-yl)methanol (2756),
(Z)-2-Methyl-2-butensäure-((*2S,3R,4R*)-2-phenyl-4-(4-(trifluormethyl)benzyl)-tetrahydrofuran-3-yl)methylester (3015),
(*Z*)-2-Methyl-2-butensäure-((*2S,3R,4R*)-2-(3,4-dimethoxyphenyl)-4-(pyridin-2-yl-methyl)tetrahydrofuran-3-yl)methylester (2823),
((*2S,3R,4R*)-4-(Biphenyl-4-ylmethyl)-2-(3,4,5-trimethoxyphenyl)tetrahydrofuran-3-yl)-methanol (2767),
((*2S,3R,4R*)-4-(4-(1,1-Difluorethyl)benzyl)-2-(3,4-dimethoxyphenyl)tetrahydrofuran-3-yl)methanol (3013),
Cyclobutancarbonsäure-((*2S,3R,4R*)-4-(3,4-dimethoxybenzyl)-2-(4-fluorphenyl)-tetrahydrofuran-3-yl)methylester (2863),
Cyclopentancarbonsäure-((*2S,3R,4R*)-4-(3,4-dimethoxybenzyl)-2-(3,4,5-trimethoxyphenyl)tetrahydrofuran-3-yl)methylester (2635),
Cyclopropancarbonsäure-((*2S,3R,4R*)-4-(3,4-dimethoxybenzyl)-2-(3,4,5-trimethoxyphenyl)tetrahydrofuran-3-yl)methylester (2637),
((*2S,3R,4R*)-2-(3,4-Dimethoxyphenyl)-4-(naphthalin-1-ylmethyl)tetrahydrofuran-3-yl)-methanol (2821),
((*2S,3R,4R*)-4-(4-Butylbenzyl)-2-(3,4-dimethoxyphenyl)tetrahydrofuran-3-yl)methanol (3008),
2-(Adamantan-1-yl)essigsäure-((*2S,3R,4R*)-4-(3,4-dimethoxybenzyl)-2-(3,4-dimethoxyphenyl)tetrahydrofuran-3-yl)methylester (2745),
(*Z*)-2-Methyl-2-butensäure-((*2S,3R,4R*)-4-(4-acetylbenzyl)-2-(3,4,5-trimethoxyphenyl)tetrahydrofuran-3-yl)methylester (2771),
4-(((*3R,4R,5S*)-4-((((*Z*)-2-Methyl-2-butenoyl)oxy)methyl)-5-(3,4,5-trimethoxyphenyl)-tetrahydrofuran-3-yl)methyl)benzoesäureethylester (2774),
Cyclopenten-1-carbonsäure-((*2S,3R,4R*)-4-(3,4-dimethoxybenzyl)-2-(3,4,5-trimethoxyphenyl)tetrahydrofuran-3-yl)methylester (2636),
Zimtsäure-((*2S,3R,4R*)-4-(3,4-dimethoxybenzyl)-2-(3,4-dimethoxyphenyl)tetrahydrofuran-3-yl)methylester (2739),
Cyclopentancarbonsäure-((*2S,3R,4R*)-4-(4-(*tert*-butyl)benzyl)-2-(3,4-dimethoxyphenyl)tetrahydrofuran-3-yl)methylester (2867),
Cyclohexancarbonsäure-((*2S,3R,4R*)-4-(3,4-dimethoxybenzyl)-2-phenyltetrahydro-furan-3-yl)methylester (2746),
(*2S,3R,4R*)-4-(3,4-Dimethoxybenzyl)-2-(3,4-dimethoxyphenyl)-3-(propargyloxy-methyl)tetrahydrofuran (2541),
((*2S,3R,4R*)-2-(3,4-Dimethoxyphenyl)-4-(4-(trifluormethyl)benzyl)tetrahydrofuran-3-yl)methanol (3005),
((*2S,3R,4R*)-4-(4-(*tert*-Butyl)benzyl)-2-(3,4-dimethoxyphenyl)tetrahydrofuran-3-yl)-methanol (2749),
Cyclopentancarbonsäure-((*2S,3R,4R*)-4-(3,4-dimethoxybenzyl)-2-phenyltetrahydro-furan-3-yl)methylester (2742),
(*Z*)-2-Methyl-2-butensäure-((*2S,3R,4R*)-4-(4-(1,1-difluorethyl)benzyl)-2-(3,4-dimethoxyphenyl)tetrahydrofuran-3-yl)methylester (3029),
Cyclopentancarbonsäure-((*2S,3R,4R*)-4-(3,4-dimethoxybenzyl)-2-(4-fluorphenyl)-tetrahydrofuran-3-yl)methylester (2781),
4-(((*3R,4R,5S*)-5-(3,4-Dimethoxyphenyl)-4-((((*Z*)-2-methyl-2-butenoyl)oxy)methyl)-tetrahydrofuran-3-yl)methyl)benzoesäureethylester (2792),
Benzoesäure-((*2S,3R,4R*)-4-(3,4-dimethoxybenzyl)-2-(3,4,5-trimethoxyphenyl)-tetrahydrofuran-3-yl)methylester (2638),
Cyclohexancarbonsäure-((*2S,3R,4R*)-4-(3,4-dimethoxybenzyl)-2-(3,4,5-trimethoxyphenyl)tetrahydrofuran-3-yl)methylester (2633),
Isobuttersäure-((*2S,3R,4R*)-4-(3,4-dimethoxybenzyl)-2-(3,4,5-trimethoxyphenyl)-tetrahydrofuran-3-yl)methylester (2632),
(Z)-2-Methyl-2-butensäure-((*2S,3R,4R*)-4-(4-butylbenzyl)-2-(3,4-dimethoxyphenyl)-tetrahydrofuran-3-yl)methylester (3026),
(*Z*-2-Methyl-2-butensäure-((2S,3R,4R)-4-(4-(tert-butyl)benzyl)-2-(4-fluorphenyl)-tetrahydrofuran-3-yl)methylester (3010),
(*2S,3R,4R*)-3-((Allyloxy)methyl)-4-(3,4-dimethoxybenzyl)-2-(3,4-dimethoxyphenyl)-tetrahydrofuran (2540),
3-Methyl-2-butensäure-((*2S,3R,4R*)-4-(3,4-dimethoxybenzyl)-2-(3,4,5-trimethoxyphenyl)tetrahydrofuran-3-yl)methylester (2755),
3-Methylbuttersäure-((*2S,3R,4R*)-4-(3,4-dimethoxybenzyl)-2-(4-fluorphenyl)-tetrahydrofuran-3-yl)methylester (2862),
(*Z*)-2-Methyl-2-butensäure-((*2S,3R,4R*)-4-(4-fluorbenzyl)-2-(4-fluorphenyl)tetrahydrofuran-3-yl)methylester (2864),
Buttersäure-((*2S,3R,4R*)-4-(3,4-dimethoxybenzyl)-2-(3,4,5-trimethoxyphenyl)-tetrahydrofuran-3-yl)methylester (2629),
(*E)*-2-Methyl-2-butensäure-((*2S,3R,4R*)-4-(3,4-dimethoxybenzyl)-2-(3,4,5-trimethoxyphenyl)tetrahydrofuran-3-yl)methylester (2628),
(*E*)-2-Butensäure-((*2S,3R,4R*)-4-(3,4-dimethoxybenzyl)-2-(3,4-dimethoxyphenyl)-tetrahydrofuran-3-yl)methylester (2549),
(*Z*)-2-Methyl-2-butensäure-((*2S,3R,4R*)-4-(4-(tert-butyl)benzyl)-2-(3,4-dimethoxyphenyl)tetrahydrofuran-3-yl)methylester (2788),
(*Z*)-2-Methyl-2-butensäure-((*2S,3R,4R*)-4-(4-(tert-butyl)benzyl)-2-(3,4,5-trimethoxyphenyl)tetrahydrofuran-3-yl)methylester (2765),
3-Methyl-2-butensäure-((*2S,3R,4R*)-4-(3,4-dimethoxybenzyl)-2-(3,4-dimethoxyphenyl)tetrahydrofuran-3-yl)methylester (2738),
2-Ethylbuttersäure-((*2S,3R,4R*)-4-(3,4-dimethoxybenzyl)-2-(4-fluorphenyl)tetrahydrofuran-3-yl)methylester (2856).
